# EUROPEAN PATENT APPLICATION

(11) **EP 4 218 411 A2**
(43) Date of publication of application: **02.08.2023**
(21) Application number: 23154833.0
(22) Date of filing: 18.03.2015
(51) Int. Cl.: A01N 1/02, C12Q 1/68

(54) **STABILIZATION AND ISOLATION OF EXTRACELLULAR NUCLEIC ACIDS**

(30) Priority: 18.03.2014 EP 14000990; 18.03.2014 US 201461955200 P
(62) Divisional of application: 15711721.9
(71) Applicant: QIAGEN GmbH, 40724 Hilden (DE)
(72) Inventor: GRÖLZ, Daniel, 40724 Hilden (DE)
(74) Representative: König Szynka Tilmann von Renesse Patentanwälte Partnerschaft mbB Düsseldorf

(57) **Abstract**

The present invention provides methods, compositions and devices for stabilizing the extracellular nucleic acid population in a cell-containing biological sample using a poly(oxyethylene) polymer or mono-ethylene glycol as stabilizing agent.

## Description

### FIELD OF THE INVENTION

The technology disclosed herein relates to methods and compositions suitable for stabilizing the extracellular nucleic acid population in a cell-containing sample, in particular a blood sample, and to a method for isolating extracellular nucleic acids from respectively stabilized biological samples.

### BACKGROUND

Extracellular nucleic acids have been identified in blood, plasma, serum and other body fluids. Extracellular nucleic acids that are found in respective samples are to a certain extent degradation resistant due to the fact that they are protected from nucleases (e.g. because they are secreted in form of a proteolipid complex, are associated with proteins or are contained in vesicles). The presence of elevated levels of extracellular nucleic acids such as DNA and/or RNA in many medical conditions, malignancies, and infectious processes is of interest *inter alia* for screening, diagnosis, prognosis, surveillance for disease progression, for identifying potential therapeutic targets, and for monitoring treatment response. Additionally, elevated fetal DNA/RNA in maternal blood is being used to determine e.g. gender identity, assess chromosomal abnormalities, and monitor pregnancy-associated complications. Thus, extracellular nucleic acids are in particular useful in non-invasive diagnosis and prognosis and can be used e.g. as diagnostic markers in many fields of application, such as non-invasive prenatal genetic testing, oncology, transplantation medicine or many other diseases and, hence, are of diagnostic relevance (e.g. fetal- or tumor-derived nucleic acids). However, extracellular nucleic acids are also found in healthy human beings. Common applications and analysis methods of extracellular nucleic acids are e.g. described in WO97/035589, WO97/34015, Swarup et al, FEBS Letters 581 (2007) 795-799, Fleischhacker Ann. N.Y. Acad. Sci. 1075: 40-49 (2006), Fleischhacker and Schmidt, Biochmica et Biophysica Acta 1775 (2007) 191-232, Hromadnikova et al (2006) DNA and Cell biology, Volume 25, Number 11 pp 635-640; Fan et al (2010) Clinical Chemistry 56:8.

Extracellular nucleic acids are usually only comprised in a low concentration in the samples. E.g. free circulating nucleic acids are present in plasma in a concentration of 1-100ng/ml plasma. Furthermore, extracellular nucleic acids often circulate as fragments of a size of 500nt, 300nt (when indicating the size and hence the chain length the term "nt" also includes "bp" in case of DNA) or even less (circulating nucleosomes). For ccfDNA in plasma, the average length is often only approx. 140-170bp. Additionally, the actual target extracellular nucleic acid that is supposed to be identified for diagnostic purposes usually also represents only a small fraction among the total extracellular nucleic acids. With respect to ccfDNA, usually only a few thousand amplifiable copies are present per ml of blood depending e.g. on the pregnancy state or tumor grade. Specifically tumor specific DNA fragments are very rare and often are comprised in a concentration that is 1000-fold less than the "normal" extracellular nucleic acid background. This low concentration poses challenges with respect to the stabilization of the sample and the subsequent isolation of the extracellular nucleic acids from the stabilized samples.

A major problem regarding the analysis of circulating, cell-free nucleic acids (cfNA) such as from tumors or of foetal origin is-besides the degradation that occurs in serum and probably also plasma-the possible dilution of extracellular DNA (and RNA) by genetic material from damaged or decaying cells after sample collection. After the sample was collected, cellular nucleic acid are released from the cells contained in the sample due to cell breakage during ex *vivo* incubation, typically within a relatively short period of time after sample collection. Once cell lysis begins, the lysed cells release large amounts of additional nucleic acids which become mixed with the extracellular nucleic acids and it becomes increasingly difficult to recover the extracellular nucleic acids for testing. With respect to blood samples, in particular the lysis of white blood cells is a problem as they release large amounts of genomic DNA in addition to RNA. Red blood cells do not contain genomic DNA. Therefore, stabilization of circulating nucleic acids in whole blood must include mechanism to stabilize blood cells in order to prevent during stabilization a contamination of the extracellular nucleic acid population by cellular genomic DNA and also RNA. In particular the dilution of the extracellular nucleic acids, in particular rare target extracellular nucleic acids, is an issue and must be prevented. These problems are discussed in the prior art (see e.g. Chiu et al (2001), Clinical Chemistry 47:9 1607-1613; Fan et al (2010) and US2010/0184069). Further, the amount and recoverability of available extracellular nucleic acids can decrease substantially over a period of time due to degradation. Besides mammalian extracellular nucleic acids that derive e.g. from tumor cells or the fetus, cell-containing samples may also comprise other nucleic acids of interest that are not comprised in cells. An important, non-limiting example is pathogen nucleic acids such as viral nucleic acids. Preservation of the integrity of viral nucleic acids in cell-containing samples such as in particular in blood specimens during shipping and handling is also crucial for the subsequent analysis and viral load monitoring.

The release from intracellular nucleic acids after sample collection particularly is an issue, if the sample comprises a high amount of cells as is the case e.g. with whole blood samples. Thus, in order to avoid respectively reduce the above described problems it is common to separate an essentially cell-free fraction of the sample from the cells contained in the sample basically immediately after the sample is obtained. E.g. it is recommended to obtain blood plasma from whole blood basically directly after the blood is drawn and/or to cool the whole blood and/or the obtained plasma or serum in order to preserve the integrity of the extracellular nucleic acids and to avoid contaminations of the extracellular nucleic acid population with intracellular nucleic acids that are released from the contained cells. However, obtaining an essentially cell-free fraction of a sample can be problematic and the separation is frequently a tedious and time consuming multi-step process as it is important to use carefully controlled conditions to prevent cell breakage during centrifugation which could contaminate the extracellular nucleic acids with cellular nucleic acids released during breakage. The need to directly separate e.g. the plasma from the blood is also a major disadvantage with respect to the handling of the samples as appropriate equipment is not necessarily available at the site where the sample is collected. Furthermore, it is often difficult to remove all cells. Thus, many processed samples that are often and commonly classified as "cell-free" such as plasma or serum in fact still contain residual amounts of cells that were not removed during the separation process. These cells may also become damaged or may die during handling of the sample, thereby releasing intracellular nucleic acids, in particular genomic DNA, as is described above. These remaining cells also pose a risk that they become damaged during the handling so that their nucleic acid content, particularly genomic (nuclear) DNA and cytoplasmic RNA, would merge with and thereby contaminate respectively dilute the extracellular, circulating nucleic acid fraction. To remove these remaining contaminating cells and to avoid/reduce the aforementioned problems, it was known to perform a second centrifugation step at higher speed. However, again, such powerful centrifuges are often not available at the facilities wherein the blood is obtained. Furthermore, even if plasma is obtained directly after the blood is drawn, it is recommended to freeze it at -80°C in order to preserve the nucleic acids contained therein if the nucleic acids can not be directly isolated. This too imposes practical constraints upon the processing of the samples as e.g. the plasma samples must be shipped frozen. This increases the costs and furthermore, poses a risk that the sample gets compromised in case the cold chain is interrupted.

With respect to the stabilization of blood, the following technologies are known in the art: Blood samples are usually collected in blood collection tubes containing spray-dried or liquid EDTA (e.g. BD Vacutainer K₂EDTA). EDTA chelates magnesium, calcium and other bivalent metal ions, thereby inhibiting enzymatic reactions, such as e.g. blood clotting or DNA degradation due to DNases. However, EDTA does not efficiently prevent the dilution respectively contamination of the extracellular nucleic acid population by released intracellular nucleic acids during storage. Thus, the extracellular nucleic acid population that is found in the cell-free portion of EDTA stabilised samples changes during the storage and becomes contaminated with large amounts of intracellular nucleic acids, in particular genomic DNA. Accordingly, EDTA is not capable of sufficiently stabilizing the extracellular nucleic acid population in particular because it can not avoid the contamination of the extracellular nucleic acid population with e.g. genomic DNA fragments which are generated after blood draw by cell degradation and cell instability during sample transportation and storage.

Blood collection tubes are known that contain reagents for an immediate stabilization of the RNA gene expression profile and thus the transcriptome at the point of sample collection (see for example US 6,617,170, US 7,270,953, Kruhoffer et al, 2007). However, these methods are based on the immediate lysis of the cells contained in the sample. Therefore, these methods and other methods that induce cell lysis are unsuitable for stabilizing the extracellular nucleic acid population in a cell-containing sample, because they induce the release of intracellular nucleic acids which become thereby mixed with the extracellular nucleic acid population.

Furthermore, methods are known in the prior art for stabilizing cell-containing samples, such as blood or tissue samples, which stabilize e.g. the cells, the transcriptome, genome and proteome. Such a method is e.g. disclosed in WO 2008/145710. Said method is based on the use of specific stabilizing compounds, such as for example N,N-Dimetyhlacetamide. However, N,N-dimetyhlacetamide is a toxic agent. Therefore, there is a need to provide alternative stabilization methods which avoid the use of toxic agents.

Methods are known in the prior art that specifically aim at stabilizing extracellular nucleic acids contained in whole blood. One method employs the use of formaldehyde to stabilize the cell membranes, thereby reducing the cell lysis and furthermore, formaldehyde inhibits nucleases. Respective methods are e.g. described in US 7,332,277 and US 7,442,506. To address the need of simultaneous cell stabilization and nucleic acid stabilization, stabilization systems were developed that are based on the use of formaldehyde releasers. Respective stabilization agents are commercially available from Streck Inc. under the name of cell-free RNA BCT (blood collection tube). The 10 ml blood collection tube is intended for the preservation and stabilization of cell-free RNA in plasma for up to 3 days at room temperature. The preservative stabilizes cell-free RNA in plasma and prevents the release of non-target background RNA from blood cells during sample processing and storage. US 2011/0111410 describes the use of formaldehyde releasing components to achieve cell and RNA stabilization in the same blood sample. Therefore, this document describes a technique wherein the stabilization agent stabilises the blood cells in the drawn blood thereby preventing contamination of cellular RNA with cell-free RNA or globin RNA, inhibits the RNA synthesis for at least 2 hours and cellular RNA that is within the blood cells is preserved to keep the protein expression pattern of the blood cells substantially unchanged to the time of the blood draw. The white blood cells can be isolated from the respectively stabilised sample and cellular RNA is than extracted from the white blood cells. However, the use of formaldehyde or formaldehyde-releasing substances has drawbacks, as they compromise the efficacy of extracellular nucleic acid isolation by induction of crosslinks between nucleic acid molecules or between proteins and nucleic acids. Methods to stabilize blood samples are also described e.g. in US 2010/0184069 and US 2010/0209930.

PCT/EP2012/070211and PCT/EP2012/068850 describe different methods for stabilizing the extracellular nucleic acid population in a cell-containing biological sample such as a whole blood sample. The stabilization compositions described in these applications are effective in stabilizing the extracellular nucleic acid population, in particular by preventing the release of intracellular nucleic acids into the extracellular nucleic acid population.

There is a continuous need to develop and improve methods that result in a stabilization of the extracellular nucleic acid population comprised in a cell-containing biological sample, including samples suspected of containing cells, in particular whole blood, plasma or serum, thereby making the handling, respectively processing of such samples easier. By providing efficient and reliable sample stabilization technologies which preferably do not impair the subsequent nucleic acid isolation, the isolation and testing of extracellular nucleic acids contained in such samples becomes more reliable and consequently, the diagnostic and prognostic application/use of extracellular nucleic acids is improved by such stabilization technologies. In particular, there is a continuous need for a solution for preserving the extracellular nucleic acid population in whole blood samples, e.g. for prenatal testing and/or for screening for diseases such as e.g. neoplastic, in particular premalignant or malignant diseases.

It is the object of the present invention to provide methods and composition for stabilizing the extracellular population comprised in a cell-containing sample. In particular, it is the object to overcome at least one of the drawbacks of the prior art sample stabilization methods. Furthermore, it is in particular an object of the present invention to provide a method suitable for stabilizing a cell-containing biological sample, in particular a whole blood sample, at room temperature. Furthermore, it is an object of the present invention to provide a sample collection container, in particular a blood collection tube, that is capable of effectively stabilizing a cell-containing biological sample and in particular is capable of stabilizing the extracellular nucleic acid population comprised in the sample. Furthermore, it is one object of the present invention to provide a stabilization technology which subsequently allows isolation of the extracellular nucleic acids from the stabilized sample with good yield.

### SUMMARY OF THE INVENTION

The present invention is based on the surprising finding that poly(oxyethylene) polymers such as polyethylene glycol are effective in stabilizing cell-containing biological samples comprising extracellular nucleic acids, in particular whole blood samples. It was found that poly(oxyethylene) polymers such as polyethylene glycol of different molecular weights and in various concentrations are capable of stabilizing the extracellular nucleic acid population of the cell-containing sample and in particular are capable to reduce the risk that the extracellular nucleic acid population becomes contaminated with genomic DNA, in particular fragmented genomic DNA, after the sample was collected. Using a poly(oxyetlylene) polymer such as polyethylene glycol as stabilizing agent reduces the risk that the extracellular nucleic acid population becomes diluted by intracellular nucleic acids such as in particular genomic DNA, what significantly contributes to the preservation of the profile of the extracellular nucleic acid population at the time of sample collection. The stabilization effect was stronger than that seen with many other stabilization agents. Furthermore, it was found that poly(oxyethylene) polymers such as preferably polyethylene glycol can be advantageously used in combination with other stabilizing agents such as caspase inhibitors and amides in order to further improve the stabilization effect on the cell-containing sample. Advantageously, poly(oxyethylene) polymers such as the preferred polyethylene glycol are not classified as toxic, harmful or irritant agent. In advantageous embodiments, two or more poly(oxyethylene) glycols are used that differ in their molecular weight. Preferably, a high molecular weight poly(oxyethylene) polymer having a molecular weight of at least 1500 is used in combination with a low molecular weight poly(oxyethylene) polymer having a molecular weight of 1000 or less for stabilization. It was found that a balanced combination of poly(oxyethylene) polymers that differ in their molecular weight, such as using a high and low molecular weight poly(oxyethylene) polymer, provides efficiently stabilized samples, from which the extracellular nucleic acids can be subsequently isolated with good yield using various standard nucleic acid isolation methods. Furthermore, the use of mono-ethylene glycol (1,2-ethanediol) as stabilizing agent for cell-containing samples is described herein. Mono-ethylene gylcol may also be used in combination with the poly(oxyethylene) polymer for stabilization.

According to a first aspect, a method suitable for stabilizing an extracellular nucleic acid population comprised in a cell-containing biological sample is provided, comprising contacting the cell-containing sample with at least one poly(oxyethylene) polymer as stabilizing agent or with mono-ethylene glycol as stabilizing agent.

According to a second aspect, a method for isolating extracellular nucleic acids from a cell-containing biological sample is provided, wherein said method comprises
a) stabilizing the cell-containing biological sample according to the method defined in the first aspect of the present invention; and
b) isolating extracellular nucleic acids from the stabilized sample.

According to a third aspect, a composition suitable for stabilizing a cell-containing biological sample is provided comprising
i) a poly(oxyethylene) polymer as stabilizing agent or
ii) mono-ethylene glycol as stabilizing agent
and one or more, preferably two or more further additives selected from the group consisting of
- one or more primary, secondary or tertiary amides;
- a caspase inhibitor;
- an anticoagulant and/or a chelating agent.

Preferably, the composition according to the third aspect comprises a poly(oxyethylene) polymer, which preferably is a high molecular weight poly(oxyethylene) polymer having a molecular weight of at least 1500, as stabilizing agent and furthermore comprises one or more, preferably two or more further additives selected from the group consisting of
- at least one further poly(oxyethylene) polymer having a molecular weight that is at least 100, preferably at least 200, at least 300 or at least 400 below the molecular weight of the first poly(oxyethylene) polymer, which preferably is a high molecular weight poly(oxyethylene) polymer, wherein said further poly(oxyethylene) polymer preferably is a low molecular weight poly(oxyethylene) polymer having a molecular weight of 1000 or less;
- one or more primary, secondary or tertiary amides;
- a caspase inhibitor;
- an anticoagulant and/or a chelating agent.

A respective stabilizing composition is particularly effective in stabilizing a cell-containing biological sample, such as blood, plasma and/or serum, by stabilizing cells and the extracellular nucleic acid population comprised in said sample. The extracellular nucleic acid population contained in the cell-containing biological sample is substantially preserved over the stabilization period in the state it had shown at the time the biological sample was contacted with said stabilizing composition. The release of genomic DNA and other intracellular nulceic acids is significantly reduced as is shown by the examples. Extracellular nucleic acids isolated from respectively stabilized samples comprise significantly less contamination with intracellular nucleic acids, in particular fragmented genomic DNA, compared to extracellular nucleic acids that are isolated from unstabilized samples. A respective stabilizing composition allows the storage and/or handling, e.g. shipping, of the stabilized sample, e.g. blood, at room temperature for days without substantially compromising the quality of the sample, respectively the extracellular nucleic acid population contained therein.

According to a fourth aspect, the present invention is related to the use of the composition according to third aspect for stabilizing the extracellular nucleic acid population in a cell-containing biological sample, preferably a blood sample.

According to a fifth aspect, a collection device for collecting a cell-containing biological sample is provided, wherein the collection device comprises
i) a poly(oxyethylene) polymer as stabilizing agent or
ii) mono-ethylene glycol as stabilizing agent
and one or more further additives selected from the group consisting of
- one or more primary, secondary or tertiary amides;
- a caspase inhibitor;
- an anticoagulant and/or a chelating agent.

Preferably, the collection device according to the fifth aspect comprises a poly(oxyethylene) polymer, which preferably is a high molecular weight poly(oxyethylene) polymer having a molecular weight of at least 1500, as stabilizing agent and furthermore comprises one or more, preferably two or more further additives selected from the group consisting of
- at least one further poly(oxyethylene) polymer having a molecular weight that is at least 100, preferably at least 200, at least 300 or at least 400 below the molecular weight of the first poly(oxyethylene) polymer which preferably is a high molecular weight poly(oxyethylene) polymer, wherein said further poly(oxyethylene) polymer preferably is a low molecular weight poly(oxyethylene) polymer having a molecular weight of 1000 or less;
- one or more primary, secondary or tertiary amides;
- a caspase inhibitor;
- an anticoagulant and/or a chelating agent.

The collection device for collecting a cell-containing biological sample, preferably a blood sample, may comprise a stabilizing composition according to the third aspect of the present invention. Providing a respective collection device, e.g. a sample collection tube, comprising the stabilizing composition has the advantage that the cell-containing biological sample is immediately stabilized as soon as the sample is collected in the respective device.

According to a sixth aspect, a method is provided comprising the step of collecting, preferably drawing, a biological sample, preferably blood, from a patient directly into a chamber of a container according to the fifth aspect of the present invention.

According to a seventh aspect, a method of producing a composition according to the third aspect of the present invention is provided, wherein the components of the composition are mixed, preferably are mixed in a solution. The term "solution" as used herein in particular refers to a liquid composition, preferably an aqueous composition. It may be a homogenous mixture of only one phase but it is also within the scope of the present invention that a solution comprises solid components such as e.g. precipitates.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

### DETAILED DESCRIPTION OF THIS INVENTION

The present invention is directed to methods, compositions and devices and thus to technologies for stabilizing the extracellular nucleic acid population comprised in a cell-containing biological sample that are based on the use of a poly(oxyethylene) polymer as stabilizing agent. It is shown that poly(oxyethylene) polymers such as polyethylene glycol of different molecular weights and in various concentrations are effective as stabilizing agents. Furthermore, advantageous combinations with other stabilizing agents are described. The stabilization that is achieved with the methods and compositions of the present invention allows the storage and/or handling of the stabilized sample for a prolonged period of time at room temperature.

The stabilization technologies described herein reduce the risk that the extracellular nucleic acid population comprised in the cell-containing sample becomes contaminated and thus becomes diluted with intracellular nucleic acids, in particular fragmented genomic DNA, originating from damaged and/or dying cells contained in the sample. As the composition of the extracellular nucleic acid population is stabilized and thus is substantially preserved at the time the sample is obtained, the time between sample collection and nucleic acid isolation can vary within the suitable stabilization period without significant negative effect on the composition of the extracellular nucleic acid population. This is an important advantage as it reduces variabilities in the extracellular nucleic acid population attributable to different handling procedures. Extracellular nucleic acids isolated from respectively stabilized samples comprise significantly less contamination with intracellular nucleic acids, in particular fragmented genomic DNA, compared to extracellular nucleic acids that are isolated from unstabilized samples. The described stabilization technologies improve the standardization of diagnostic or prognostic extracellular nucleic acid analyses because variations in the handling/storage of the samples have less influence on the quality, respectively the composition and thus profile of the extracellular nucleic acid population comprised in the cell-containing biological sample, thereby making diagnostic or prognostic applications that are based on the extracellular nucleic acid fraction more reliable and more independent from the used storage/handling conditions. The achieved substantial preservation is an important advantage because it enhances the accuracy of any subsequent tests that aims at analysing extracellular nucleic acids. Thereby, the diagnostic and prognostic applicability of the respectively isolated extracellular nucleic acids is improved. Specific embodiments described herein have the advantage that the ratio of certain extracellular nucleic acid molecules comprised in the population of extracellular nucleic acids can be kept more constant and thus more comparable to the ratio present at the time the biological sample was collected. Thus, advantageously, the profile of the extracellular nucleic acid population can be preserved. Hence, the analysis of respective cell containg biological samples, respectively the extracellular nucleic acids obtained from respectively stabilized samples, becomes more comparable.

Furthermore, the teachings of the present invention obviate the necessity to directly separate cells contained in the biological sample from the cell-free portion of the sample after sample collection in order to avoid, respectively reduce, contaminations of the extracellular nucleic acids with intracellular nucleic acids, in particular fragmented genomic DNA, that is otherwise released from cells that die or decay during storage/shipping. This advantage considerably simplifies the handling of cell-containing biological samples, such as whole blood samples. However, the teachings of the invention are also advantageous when processing cell-depleted biological samples, or samples commonly referred to as being "cell-free" such as e.g. blood plasma or serum. Respective cell-depleted or "cell-free" biological samples may still (also depending on the used separation process) comprise residual cells, in particular white blood cells which comprise genomic DNA. Said residual cells pose a risk that the extracellular nucleic acid population becomes increasingly contaminated with intracellular nucleic acids, in particular fragmented genomic DNA, if the (potentially) remaining cells are damaged or die during the shipping or storing process. This risk is considerably reduced when using the stabilization methods taught by the present invention. Thus, the present invention has many advantages when stabilizing biological samples which comprise large amounts of cells such as e.g. blood samples, but also has important advantages when stabilizing biological samples which comprise smaller or only a small amount of cells or which may only be suspected of containing cells such as e.g. plasma, serum, urine, saliva, synovial fluids, amniotic fluid, lachrymal fluid, lymphatic fluid, liquor, cerebrospinal fluid and the like.

Furthermore, the use of mono-ethylene glycol is described for stabilizing the extracellular nucleic acid population in a cell-containing sample, wherein optionally, mono-ethylene glycol is used in combination with the poly(oxyethylene) polymer and/or one or more of the further stabilizing agents described herein.

### A. METHOD OF STABILIZATION

According to a first aspect, a method suitable for stabilizing an extracellular nucleic acid population comprised in a cell-containing biological sample is provided which comprises a step of contacting the cell-containing biological sample with at least one poly(oxyethylene) polymer as stabilizing agent or with mono-ethylene glycol as stabilizing agent. The advantages of using a poly(oxyethylene) polymer as stabilizing agent were described above.

Preferably, the method according to the first aspect comprises a step of contacting the cell-containing biological sample with at least one poly(oxyethylene) polymer as stabilizing agent. The term poly(oxyethylene) polymer in particular refers to an an oligomer or polymer of ethylene oxide. It comprises at least two ethylene oxide units. Poly(oxyethylene) polymers are known in low and high molecular weights. Their molecular weight are usually multitutes of 44, the molecular weight of its monomer, and can range up to 100000. The molecular weight is indicated in Da. The poly(oxyethylene) polymer may be linear or branched or may have other geometries. A linear poly(oxyethylene) polymer is preferred. The poly(oxyethylene) polymer may be unsubstituted or substituted and preferably is polyethylene glycol. As is demonstrated in the examples, polyethylene glycol has in various molecular weights and in various concentrations a stabilization effect on cells and therefore can be used alone or in combination with other stabilization agents in order to stabilize the extracellular nucleic acid population in a cell-containing sample, in particular by reducing dilution of the extracellular nucleic acid population by intracellular nucleic acids such as in particular genomic DNA. However, also other poly(oxyethylene) polymers may be used that achieve a stabilization effect as was shown for polyethylene glycol. As mentioned, also substituted poly(oxyethylene) polymers having a stabilizing effect may be used such as alkyl poly(oxyethylene) polymers, e.g. alkylpolyethylene glycols, but also poly(oxyethylene) esters, poly(oxyethylene) amines, poly(oxyethylene) thiol compounds, poly(oxyethylene) glycerides and others. The preferred embodiment of the poly(oxyethylene) polymer that is used as stabilizing agent is polyethylene glycol. It preferably is unbranched and may be unsubstituted or substituted. Known substituted forms of polyethylene glycol include alkylpolyethylene glycols that are e.g. substituted at one or both ends with an C1-C5 alkyl group. Preferably, unsubstituted polyethylene glycol of the formula HO-(CH₂CH₂O)ₙ-H is used. All disclosures described in this application for the poly(oxyethylene) polymer in general, specifically apply and particularly refer to the preferred embodiment polyethylene glycol even if not explicitly stated. The poly(oxyethylene) polymer can be used in various molecular weights. Preferably, the term polyethylene glycol refers to oligo or polymers as is also evident from the molecular weights specified herein as suitable and preferred for the poly(oxyethylene) polymer which specifically also apply to the preferred embodiment polyethylene glycol.

A correlation was found between the stabilization effect of the poly(oxyethylene) polymer and its molecular weight. Higher molecular weight poly(oxyethylene) polymers were found to be more effective stabilizing agents than lower molecular weight poly(oxyethylene) polymers. To achieve an efficient stabilization with a lower molecular weight poly(oxyethylene) polymer, generally higher concentrations are recommendable compared to a higher molecular weight poly(oxyethylene) polymer. For some applications such as blood samples it is preferred though to keep the amount of additives used for stabilization low. Therefore, in embodiments, higher molecular weight poly(oxyethylene) polymers are used as stabilizing agents, as they allow to use lower concentrations of the poly(oxyethylene) polymer while achieving a strong stabilization effect on the extracellular nucleic acid population.

Thus, according to one embodiment, a high molecular weight poly(oxyethylene) polymer having a molecular weight of at least 1500 is used as stabilizing agent and is contacted with the cell-containing sample. The high molecular weight poly(oxyethylene) polymer may have a molecular weight that lies in a range selected from 1500 to 50000, 1500 to 40000, 2000 to 40000, 3000 to 40000, 2000 to 30000, 2500 to 30000, 2500 to 25000, 3000 to 20000, 4000 to 20000, 3500 to 15000, 3000 to 10000, 4000 to 10000, 4500 to 10000, 4500 to 9000, 4500 to 8000, 5000 to 8000, 5000 to 7000 and 5500 to 7000. As is demonstrated by the examples, many different high molecular weight poly(oxyethylene) polymers can be used in conjunction with the invention. Suitable high molecular weight poly(oxyethylene) polymers are also described in conjunction with different aspects and embodiments of the invention. These molecular weights are particularly preferred for the use of a polyethylene glycol, in particular an unsubstituted polyethylene glycol. Unsubstituted polyethylene glycol was also used in the examples. The molecular weight of a poly(oxyethylene) polymer having a specific molecular weight may vary within certain ranges conditional to manufacturing as is well-known to the skilled person.

The high molecular weight poly(oxyethylene) polymer is used in a concentration, wherein it exerts or supports the stabilization of the extracellular nucleic acid population that is contained in the cell-containing sample. Suitable concentrations for different sample types can be determined by the skilled person, for example by testing different concentrations of a specific high molecular weight poly(oxyethylene) polymer in the test assays described in the examples. As is demonstrated by the examples, the high molecular weight poly(oxyethylene) polymer is effective in various concentrations. The achieved stabilization effect and the preferred concentration also depends on whether one or more additional stabilizing agents are used. Preferred combinations are described herein. According to one embodiment, the mixture that is obtained after contacting the cell-containing biological sample with the high molecular weight poly(oxyethylene) polymer and optionally further additives comprises the high molecular weight poly(oxyethylene) polymer in a concentration range that is selected from 0.05% to 4% (w/v), 0.1% to 3% (w/v), 0.2% to 2.5% (w/v), 0.25% to 2% (w/v), 0.3% to 1.75% (w/v) and 0.35% to 1.5% (w/v). According to one embodiment, the high molecular weight poly(oxyethylene) polymer is used in lower concentration ranges such as 0.25% to 1.5% (w/v), 0.3% to 1.25% (w/v), 0.35% to 1% (w/v) and 0.4% to 0.75% (w/v). The above concentrations ranges are particularly suitable for the stabilization of blood. Using a high molecular weight poly(oxyethylene) polymer in a concentration of 1.5% (w/v) or less, 1.25% (w/v) or less, 1% (w/v) or less and in particular in a concentration of 0.75% (w/v) or less is advantageous in certain embodiments. It was found in embodiments wherein a high molecular weight poly(oxyethylene) polymer was used in certain higher concentrations in the resulting mixture comprising the stabilizing agent and the cell-containing sample, such as a blood sample, the subsequent isolation of the extracellular nucleic acids may become impaired when using certain standard nucleic acid isolation procedures that involve e.g. the use of silica columns. It is however advantageous to use a stabilization technology that is compatible with most standard nucleic acid isolation methods and the use of silica columns for isolating extracellular nucleic acids is widely used and established. Using the high molecular weight poly(oxyethylene) polymer in a concentration of 1.5% (w/v) or less, 1.25% (w/v) or less, 1% (w/v) or less or 0.75% or less in the stabilization mixture containing the sample supports that the extracellular nucleic acids can be efficiently isolated from the stabilized samples using such standard methods with good yield even if higher volumes of stabilization composition is used. This is advantageous, because extracellular nucleic acids and in particular specific target nucleic acids comprised in the extracellular nucleic acid populations are often present in only few copies. The stabilization technology described herein is also compatible with other nucleic acid isolation methods, including those that are based on ion exchange. As is demonstrated in the examples, the observed impairment also depends on the used volume of the the stabilization composition that contains the high molecular weight (polyoxyethylene) polymer. Using a lower volume of stabilizing composition for stabilization can compensate the impairment even if a high molecular weight poly(oxyethylene) polymer is used in higher concentrations so that the subsequent nucleic acid isolation is not impaired. Thus, reducing the overall concentration of the high molecular weight poly(oxyethylene) polymer in the mixture containing the sample and/or reducing the volume of stabilsation composition containing the high molecular weight poly(oxyethylene) polymer are alternative options to reduce or even avoid impairment. This volume dependent effect that is demonstrated in the examples is significant and was highly surprising as the overall concentration in the mixture containing the sample was the same.

According to one embodiment, the poly(oxyethylene) polymer used for stabilization has a molecular weight below 1500 and may be a low molecular weight poly(oxyethylene) polymer having a molecular weight of 1000 or less. It is used in a concentration, wherein it can exert or support the stabilizing effect on the extracellular nucleic acid population of the cell-containing biological sample. Suitable concentrations for different sample types can be determined by the skilled person, e.g. by testing different concentrations in the test assays described in the examples. A respective poly(oxyethylene) polymer, such as a low molecular weight poly(oxyethylene) polymer having a molecular weight of 1000 or less, can be present in the mixture that is obtained after contacting the cell-containing biological sample with said poly(oxyethylene) polymer and optionally further additives in a concentration range that is selected from 0.5% to 10%, 1.5% to 9%, 2% to 8%, 2 to 7%, 2.5% to 7% and 3% to 6%. The percentage values refer to (w/v) in case the poly(oxyethylene) polymer is a solid and to (v/v) in case the poly(oxyethylene) polymer is a liquid. The indicated concentrations are particularly suitable for the use in case of blood samples. Higher concentration of at least 1%, preferably at least 1.5% are advantageous for a low molecular weight poly(oxyethylene) polymer to achieve (or support) the stabilization of the extracellular nucleic acid population. As is demonstrated in the examples, also poly(oxyethylene) polymers such as polyethylene glycol having a molecular weight of 1500 or less such as 1000 or less show a stabilizing effect on the extracellular nucleic acid population, in particular by reducing dilutuions with genomic DNA. The examples demonstrate that poly(oxyethylene) polymers such as polyethylene glycol having a molecular weight of 1500 or less, such as 1000 or less are effective stabilizers, in particular when used in combination with one or more further stabilizing agents described herein such as a caspase inhibitor and at least one primary, secondary or tertiary amide. The low molecular weight poly(oxyethylene) polymer may have a molecular weight that lies in a range selected from 100 to 1000, 150 to 800, 150 to 700, preferably 200 to 600 and more preferably 200 to 500 such as 200 to 400.

It was found that a low molecular weight poly(oxyethylene) polymer having a molecular weight of 1000 or less, preferably of 800 or 700 or less, can be used in substantially higher concentrations, because it does not substantially hinder the subsequent isolation of the extracellular nucleic acids from the stabilized sample even if a higher volume of the stabilizing composition is used. However, if the amount required to achieve a stabilization effect is too high, this can be inconvenient for the processing and handling of the samples. It is generally preferred to stabilize the sample with a rather low amount or volume of stabilizing agents. This particularly, as in case of certain samples such as blood samples, the amount of stabilizing agent that can be added to the sample is restricted by the standard collection tubes that are used. E.g. for a standard collection device that is used for collecting 10ml blood, approx. 2ml stabilizing agent can be added as maximum.

According to one embodiment, at least two poly(oxyethylene) polymers are used for stabilization, which differ in their molecular weight. They may be of the same kind and preferably both are a polyethylene glycol such as an unsubstituted polyethylene glycol. According to one embodiment, the difference in the molecular weight is at least 100, at least 200, at least 300, at least 400, at least 500, at least 600, at least 700, at least 800, at least 900 or at least 1000. According to one embodiment, the difference in the molecular weight is at least 2500, at least 3500, at least 5000 or at least 7500. As is described subsequently in detail for specific embodiments of this embodiment, it is advantageous to use two poly(oxyethylene) polymers that differ in their molecular weight. As is described herein, the stabilization effect of poly(oxyethylene) polymers appears to depend on their molecular weight. In the tested examples it was found that the higher the molecular weight, the higher the stabilization efficiency. However, poly(oxyethylene) polymers may differ in their effect on the subsequent nucleic acid isolation method depending on their molecular weight. As described above, it was found that in certain embodiments that involve the use of higher molecular weight poly(oxyethylene) polymers as stabilizing agents, in particular where a higher volume of stabilization composition and a higher concentration of the polymer in the stabilizing mixture containing the sample was used, that the nucleic acid isolation was less efficient with certain nucleic acid isolation methods. As is demonstrated by the examples, such issues that occur in certain scenarios can be overcome when using a mixture of poly(oxyethylene) polymers that differ in their molecular weight. Therefore, this embodiment wherein at least two poly(oxyethylene) polymers are used for stabilization that differ in their molecular weight is advantageous, because it allows to provide balanced compositions of poly(oxyethylene) polymers having the desired characteristics with respect to the stabilization effect to be achieved and the characteristics required e.g. for certain downstream uses.

According to one embodiment, a high molecular weight poly(oxyethylene) polymer as defined above which has a molecular weight of at least 1500 is used in combination with a low molecular weight poly(oxyethylene) polymer having a molecular weight of 1000 or less and the cell-containing sample is contacted with both types of poly(oxyethylene) polymers. Suitable embodiments are described herein. Using a high molecular weight poly(oxyethylene) polymer in combination with a low molecular weight poly(oxyethylene) polymer is advantageous, because the low molecular weight poly(oxyethylene) polymer allows to reduce the concentration of the high molecular weight poly(oxyethylene) polymer required to achieve an effective stabilization of the sample. Therefore, the high molecular weight poly(oxyethylene) polymer can be used in the mixture with the sample in a concentration wherein it does not impair the subsequent nucleic acid isolation using certain standard methods such as those involving silica columns. This embodiment is andvantageous because it provides more freedom with respect to the volume or amount of stabilization composition that can be used. The low molecular weight poly(oxyethylene) polymer assists in the stabilization but in contrast to the high molecular weight poly(oxyethylene) polymer, was in the tested examples not found to significantly impair the subsequent isolation of the nucleic acids when using methods, such as those involving silica columns, where the higher molecular weight poly(oxyethylene) polymers showed in certain concentrations and/or volumes an impairing effect in the examples. Therefore, stabilizing the extracellular nucleic acid population in a cell-containing sample using a combination of a high and a low molecular weight poly(oxyethylene) polymer is a particular preferred embodiment that has important advantages. The low molecular weight poly(oxyethylene) polymer can be of the same kind as the high molecular weight poly(oxyethylene) polymer which was described above. Also for the low molecular weight poly(oxyethylene) polymer it is preferred that a polyethylene glycol is used, such as an unsubstituted polyethylene glycol. Suitable molecular weights for the high molecular weight poly(oxyethylene) polymer were described above. It may have e.g. a molecular weight that lies in a range selected from 1500 to 50000, 2000 to 40000, 2500 to 30000, 2500 to 25000 and 3000 to 20000. The low molecular weight poly(oxyethylene) polymer may have a molecular weight that lies in a range selected from 100 to 1000, 150 to 800, 150 to 700, preferably 200 to 600 and more preferably 200 to 500 such as 200 to 400. Suitable and preferred concentrations and concentration ranges for the high and low molecular weight poly(oxyethylene) polymer are described above and may also be used in the embodiment wherein both types of polymers are used in combination.

According to one embodiment, the mixture that is obtained after contacting the cell-containing biological sample, which preferably is blood, with the high and the low molecular weight poly(oxyethylene) polymer and optionally further additives used for stabilization, comprises the high molecular weight poly(oxyethylene) polymer, which may have e.g. a molecular weight in the range of 3000 to 40000, preferably 4000 to 20000, in a concentration that lies in a range of 0.2% to 1.5% (w/v), preferably 0.3% to 1.25% (w/v) and in embodiments in a range of 0.4 (w/v) to 0.75% (w/v) and the low molecular weight poly(oxyethylene) polymer, which preferably has a molecular weight that lies in a range of 200 to 800, preferably 200 to 600, in a concentration that lies in a range selected from 1.5% to 8%, preferably 2% to 7%, more preferred 2.5% to 6%. The high and low poly(oxyethylene) polymer is preferably a polyethylene glycol, such as an unsubstituted polyethylene glycol. Suitable embodiments for the high and low molecular weight poly(oxyethylene) polymer are also described above and in conjunction with the different aspects and embodiments. The cell-containing biological sample may be blood in this embodiment and the blood sample is additionally contacted with an anticoagulant. Suitable examples for anticoagulants are described below.

As is shown by the provided examples, using a poly(oxyethylene) polymer, such as a high molecular weight poly(oxyethylene) polymer, alone is in embodiments already effective in stabilizing a cell-containing sample and preserving the extracellular nucleic acid population from changes in its composition, in particular changes arising from a contamination with fragmented genomic DNA released from damaged or dying cells during storage. The stabilization effect can be significantly improved though if the poly(oxyethylene) polymer which preferably is a polyethylene glycol is used in combination with further stabilization agents and/or additives. It was found that using a poly(oxyethylene) polymer in combination with further stabilizing agents siginificantly improves the achieved stabilization effect. As is demonstrated in the examples, it is significantly supporting and improving the stabilizing effect when being used in combination with other stabilizing agents such as amides and a caspase inhinitor. Such balanced compositions significantly improve the achieved stabilization effect which is also superior to existing prior art technologies. Suitable and preferred examples of additional stabilizing agents and innovative combinations are described subsequently.

According to one embodiment, the cell-containing sample is additionally contacted with one or more primary, secondary or tertiary amides as further stabilizing agent. Primary, secondary and tertiary amides have an advantageous stabilization effect on cell-containing samples and therefore, are in one embodiment used to support the stabilization of the extracellular nucleic acid population. Also combinations or two or more primary, secondary or tertiary amides can be used. The amide preferably is a carboxylic acid amide.

Suitable concentrations for different primary, secondary or tertiary amides and/or for different sample types can be determined by the skilled person, e.g. by testing different concentrations in the test assays described in the examples. Generally, the mixture that is obtained when contacting the cell-containing biological sample with the at least one poly(oxyethylene) polymer and the one or more primary, secondary or tertiary amides and optionally further additives may comprise said amide (or combination of amides) in a concentration of at least 0.05%, at least 0.1%, at least 0.25%, at least 0.5% or at least 0.75%. Suitable concentration ranges include but are not limited to 0.1% to 10%, 0.25% to 7.5%, 0.3% to 5%, 0.4% to 3%, 0.5% to 2%, 0.6% to 1.8% and 0.75% to 1.5%. Concentrations or concentration ranges indicated in percentage values as used herein are in particular given as percentage weight per volume (w/v) for solid amides and as percentage volume per volume (v/v) for liquid amides.

According to one embodiment, the primary, secondary or tertiary amide is a compound according to formula 1 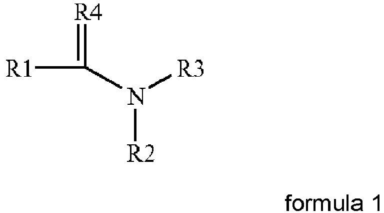 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue, preferably R4 is oxygen.

Also a combination of one or more compounds according to formula 1 can be used in addition to the poly(oxyethylene) polymer for stabilization.

In embodiments, wherein R1 is an alkyl residue, a chain length of 1 or 2 is preferred for R1.

R2 and/or R3 of the compound according to formula 1 are identical or different and are selected from a hydrogen residue and a hydrocarbon residue, which preferably is an alkyl residue. According to one embodiment, R2 and R3 are both hydrogen. According to one embodiment, one of R2 and R3 is a hydrogen and the other is a hydrocarbon residue. According to one embodiment, R2 and R3 are identical or different hydrocarbon residues. The hydrocarbon residues R2 and/or R3 can be selected independently of one another from the group comprising alkyl, including short chain alkyl and long-chain alkyl, alkenyl, alkoxy, long-chain alkoxy, cycloalkyl, aryl, haloalkyl, alkylsilyl, alkylsilyloxy, alkylene, alkenediyl, arylene, carboxylates and carbonyl. The chain length n of R2 and/or R3 can in particular have the values 1, 2, 3, 4, 5, 6, 7, 8, 9, 10, 11, 12, 13, 14, 15, 16, 17, 18, 19 and 20.

According to one embodiment, R2 and R3 have a length of the carbon chain of 1-10, preferably 1 to 5, more preferred 1 to 2. According to one embodiment, R2 and/or R3 are alkyl residues, preferably C1-C5 alkyl residues.

Preferably, the compound according to formula 1 is a carboxylic acid amide so that R4 is oxygen. It can be a primary, secondary or tertiary carboxylic acid amide.

According to one embodiment, the compound according to formula 1 is a N,N-dialkylcarboxylic acid amide. Preferred R1, R2, R3 and R4 groups are described above. Using a respective compound according to formula 1 has the advantage that additionally, intracellular nucleic acids such as in particular RNA, e.g. mRNA and/or miRNA transcripts can be stabilized in the cell-containing sample. The additional stabilization of intracellular nucleic acids, in particular gene transcript levels, is advantageous as it e.g. allows the subsequent analysis of target transcripts or transcript profiles in the contained cells. According to one embodiment, the compound according to formula 1 is selected from the group consisting of N,N-dimethylacetamide, N,N-diethylacetamide, N,N-dimethylformamide and N,N-diethylformamide. Also suitable are the respective thio analogues, which comprise sulphur instead of oxygen as R4. N,N-dimethlylacetamide (DMAA) e.g. achieves good stabilization results, however, is a toxic agent. Preferably, at least one compound according to formula 1 is used in combination with the poly(oxyethylene) polymer for stabilizing the cell-containing biological sample which is not a toxic agent according to the GHS classification.

According to one embodiment, the cell-containing biological sample is contacted for stabilization with a poly(oxyethylene) polymer and a compound according to formula 1 which is a N,N-dialkylpropanamide, such as N,N-dimethylpropanamide. As is demonstrated in the examples, a respective combination is particularly suitable for stabilizing the extracellular nucleic acid population in a cell-containing sample such as a blood sample.

According to one embodiment, the cell-containing sample is contacted with a poly(oxyethylene) polymer and at least one compound according to formula 1, which is a primary or secondary carboxylic acid amide. As can be seen from the examples of unpublished US 61/803,107 and EP 13 160 896.0 (see published WO 2014/146781), herein incorporated by reference, primary and secondary carboxylic acid amides are capable of stabilizing the extracellular nucleic acid population over a broad concentration range. According to one embodiment, the primary carboxylic acid amide is selected from the group consisting of formamide, acetamide, propanamide and butanamide. Preferably, the primary carboxylic acid is butanamide, as butanamide is particularly effective for stabilizing the extracellular nucleic acid population as demonstrated in the present examples and additionally, is non-toxic. The stabilization effect of butanamide on the extracellular nucleic acid population of cell-containing samples is also described in detail in unpublished applications EP 13 159 834.4 and EP 13 180 086.4 (see published WO 2014/146780 and WO 2014/146782), herein incorporated by reference.

According to a preferred embodiment, the cell-containing sample is contacted for stabilization with the poly(oxyethylene) polymer, which preferably is a polyethylene glycol, and butanamide and/or an N,N-dialkylpropanamide, wherein said N,N-dialkylpropanamide preferably is N,N-dimethylpropanamide. Suitable and preferred concentrations and concentration ranges described above in general for the primary, secondary and tertiary amides in general also specifically apply to this embodiment. As is shown by the examples, using butanamide and/or a N,N-dialkylpropanamide in a concentration that lies in these ranges provides an advantageous stabilizing effect on cell-containing samples such as blood samples.

According to one embodiment, the cell-containing biological sample is contacted with a poly(oxyethylene) polymer as described above and at least one caspase inhibitor. It is also within the scope of the present invention to use a combination of different caspase inhibitors. As is shown by the examples, using a poly(oxyethylene) polymer such as preferably a high molecular weight polyethylene glycol in combination with a caspase inhibitor significantly improves the achieved stabilization effect. Furthermore, it was found that in particular with biological samples that contain large amounts of cells and furthermore, differ in their composition, such as e.g. blood samples, the achieved stabilization effect was stronger and more uniform. E.g. blood samples derived from different donors may differ in the changes in the extracellular nucleic acid population that occur during ex *vivo* handling. Some samples show strong alterations in the profile of the extracellular nucleic acid population (in particular strong increases in genomic DNA) while in other samples the effects are less prominent. Such samples can also react differently to stabilization. When using a poly(oxyethylene) polymer in combination with a caspase inhibitor and preferably also one or more primary, secondary or tertiary amide as described above for stabilizing blood samples obtained from different donors, a more uniform stabilization effect could be achieved. The caspase inhibitor present in the resulting mixture significantly supports the stabilization of the extracellular nucleic acid population. Furthermore, the degradation of nucleic acids, in particular genomic DNA, present in the sample is reduced by said combination of stabilizing agents. Thus, using a poly(oxyethylene) polymer such as polyethylene glycol in combination with at least one caspase inhibitor significantly improves the stabilization effect thereby supporting that the extracellular nucleic acid population contained in the sample is substantially preserved in the state it had shown at the time the biological sample was obtained, respectively collected, even during prolonged storage periods.

Preferably, the caspase inhibitor is cell-permeable. Members of the caspase gene family play a significant role in apoptosis. The substrate preferences or specificities of individual caspases have been exploited for the development of peptides that successfully compete caspase binding. It is possible to generate reversible or irreversible inhibitors of caspase activation by coupling caspase-specific peptides to e.g. aldehyde, nitrile or ketone compounds. E.g. fluoromethyl ketone (FMK) derivatized peptides such as Z-VAD-FMK act as effective irreversible inhibitors with no added cytotoxic effects. Inhibitors synthesized with a benzyloxycarbonyl group (BOC) at the N-terminus and O-methyl side chains exhibit enhanced cellular permability. Further suitable caspase inhibitors are synthesized with a phenoxy group at the C-terminus. An example is Q-VD-OPh which is a cell permeable, irreversible broad-spectrum caspase inhibitor that is even more effective in preventing apoptosis and thus supporting the stabilization than the caspase inhibitor Z-VAD-FMK.

According to one embodiment, the caspase inhibitor is a pancaspase inhibitor and thus is a broad spectrum caspase inhibitor. According to one embodiment, the caspase inhibitor comprises a modified caspase-specific peptide. Preferably, said caspase-specific peptide is modified by an aldehyde, nitrile or ketone compound. According to one embodiment, the caspase specific peptide is modified, preferably at the carboxyl terminus, with an O-Phenoxy (OPh) or a fluoromethyl ketone (FMK) group. According to one embodiment, the caspase inhibitor is selected from the group consisting of Q-VD-OPh and Z-VAD(OMe)-FMK. In a preferred embodiment, Q-VD-OPh, which is a broad spectrum inhibitor for caspases, is used for stabilization. Q-VD-OPh is cell permeable and inhibits cell death by apoptosis. Q-VD-OPh is not toxic to cells even at extremely high concentrations and comprises a carboxy terminal phenoxy group conjugated to the amino acids valine and aspartate. It is equally effective in preventing apoptosis mediated by the three major apoptotic pathways, caspase-9 and caspase-3, caspase-8 and caspase-10, and caspase-12 (Caserta et al, 2003). Examples of caspase inhibitors are also listed in Table 1 of WO 2013/045457, herein incorporated by reference.

The mixture that is obtained after contacting the biological sample with the at least one poly(oxyethylene) polymer and the at least one caspase inhibitor and optionally further additives may comprise the caspase inhibitor (or combination of caspase inhibitors) in a concentration of at least at least 0.05µM, at least 0,1µM, at least 0.5µM, at least 0.75µM, at least 1µM, at least 1.25µM, at least 1.5µM, at least 1.75µM, at least 2µM, at least 2.25 µM, at 2.5µM, at least 2.75µM, at least 3µM, at least 3.25µM or at least 3.5µM. Suitable concentration ranges for the caspase inhibitor(s) when mixed with the cell-containing biological sample and the further additives include but are not limited 0.1 µM to 25µM, 0.75µM to 20µM, 1µM to 15µM, 1.5µM to 12.5µM, and 2µM to 10µM and 3µM to 7.5µM. The above mentioned concentrations apply to the use of a single caspase inhibitor as well as to the use of a combination of caspase inhibitors. The aforementioned concentrations are in particular suitable when using a pancaspase inhibitor, in particular a modified caspase specific peptide such as Q-VD-OPh and/or Z-VAD(OMe)-FMK. The above mentioned concentrations are e.g. suitable for stabilizing whole blood. Suitable concentration ranges for individual caspase inhibitors and/or for other cell-containing biological samples can be determined by the skilled person, e.g. by testing different concentrations of the respective caspase inhibitor in the test assays described in the examples.

The stabilizing effect observed with combinations of stabilizing agents is stronger than the effect observed for any of the individual stabilizing agents when used alone and/or allows using lower concentrations of individual stabilizers, thereby making combinatorial use of stabilizing agents an attractive option. The combinations of stabilizing agents involving a poly(oxyethylene) polymer such as a polyethylene glycol described herein have a better stabilizing effect than the individual stabilizers and therefore, are advantageous embodiments. Furthermore, additional additives can be used for stabilization such as e.g. anticoagulants and chelating agents which are particularly useful when stabilizing a blood sample.

As discussed in the background of the invention, extracellular nucleic acids are usually not present "naked" in the extracellular portion of the cell-containing sample but are e.g. stabilized to a certain extent by being released protected in complexes or by being contained in vesicles and the like. This has the effect that extracellular nucleic acids are already to a certain extent stabilized by nature and thus, are usually not degraded rapidly by nucleases in cell-containing samples such as whole blood, plasma or serum. Thus, when intending to stabilize extracellular nucleic acids that are comprised in a cell-containg biological sample, one of the primary problems after obtaining or collecting the cell-containing biological sample is the contamination and dilution of the extracellular nucleic acid population comprised in the collected cell-containing biological sample by intracellular nucleic acids, in particular fragmented genomic DNA, that originates from damaged or dying cells that are contained in the cell-containing biological sample. The stabilization technology according to the present invention is of particular advantage in this respect because it not only substantially preserves the extracellular nucleic acids present in the sample and e.g. inhibits degradation of the comprised extracellular nucleic acids (preferably at least by 60%, at least by 70%, at least by 75%, at least by 80%, at least by 85%, at least by 90% or most preferably at least by 95% over the stabilization period compared to an unstabilized sample or e.g. an EDTA stabilized sample in the case of blood) but furthermore, efficiently reduces the release of genomic DNA from cells contained in the obtained cell-containing biological sample and/or reduces the fragmentation of respective genomic DNA. According to one embodiment, using a poly(oxyethylene) polymer for stabilizing the extracellular nucleic acid population in a cell-containing sample, optionally but preferably in combination with one or more of the further stabilization agents described above, has the effect that the increase of DNA that results from a release of genomic DNA from cells contained in the sample during the stabilization period is reduced compared to a non-stabilized sample. According to one embodiment, said release of genomic DNA is reduced by at least 3-fold, at least 4-fold, at least 5-fold, at least 6-fold, at least 7-fold, at least 10-fold, at least 12-fold, at least 15-fold, at least 17-fold or at least 20-fold over the stabilization period compared to the non-stabilized sample or a corresponding sample that is stabilized with EDTA (in particular in case of a blood sample or a sample derived from blood such as plasma or serum). According to one embodiment, said release of genomic DNA is reduced by at least 60%, at least 70%, at least 75%, at least 80%, at least 85%, at least 90% or at least 95% over the stabilization period compared to the non-stabilized sample or a corresponding sample that is stabilized with EDTA (in particular in case of a blood sample or a sample derived from blood such as plasma or serum). The release of DNA can be determined e.g. by quantifying the ribosomal 18S DNA as is described herein in the example section. As is shown in the examples, the stabilization achievable with the teachings of the present invention remarkably reduces this release of DNA over the stabilization period. Thus, according to one embodiment, the stabilization effect that is achieved with a poly(oxyethylene) polymer such as a polyethylene glycol, optionally in combination with one or more of the additional stabilization agents described above such as preferably one or more primary, secondary or tertiary amides and a caspase inhibitor, results in that the release of DNA from cells contained in the stabilized sample is over the stabilization period reduced at least down to a maximum of 8-fold, at least down to a maximum of 7-fold, at least down to a maximum of 5-fold, more preferably is reduced e.g. at least down to a maximum of 4-fold, more preferably is reduced at least down to a maximum of 3-fold or preferably down to a maximum of 2-fold or even less as is e.g. determinable in the 18S DNA assay described in the examples. As is shown by the examples, an effective stabilization of the extracellular nucleic acid population is achievable with the method of the invention for a period of at least three days and when using a combination of stabilizing agents as described herein even up to 6 days and longer. During shorter as well as during longer storage of the stabilized samples the DNA release can be reduced in embodiments at least down to a maximum of two-fold and lower as is e.g. determinable in the 18S DNA assay described in the examples. Thus, according to embodiments of the stabilization technology described herein, the DNA release can be reduced down to 3fold or less or even 2fold or less up to three or even up to 6 days of storage and longer when using the stabilizing methods according to the present invention. As is demonstrated by the examples, in embodiments values between 1 and 1.5 are achieved. This is a remarkable improvement in the stabilization of the extracellular nucleic acid population compared to prior art methods. However, of course, the samples may also be further processed earlier, if desired. It is not necessary to make use of the full achievable stabilization period. Furthermore, nucleic acids can be efficiently isolated from respectively stabilized samples using different standard methods as no cross-linking of the sample occurs due to the stabilization. This greatly simplifies and improves the standardization of molecular analysis that relies on the analysis of extracellular nucleic acids.

The selection of suitable additives that may also contribute to the stabilization effect may also depend on the type of cell-containing sample to be stabilized. E.g. when processing blood as cell-containing biological sample, an anticoagulant is additionally used to prevent blood clotting. The anticoagulant is used in a concentration wherein it can prevent clotting of the amount of blood to be stabilized. The anticoagulant may be e.g. selected from the group consisting of heparin, chelating agents such as ethylenediamine tetraacetic acid, salts of carboxylic acids such as citrate or oxalate and any combination thereof. In an advantageous embodiment, the anticoagulant is a chelating agent. A chelating agent is an organic compound that is capable of forming coordinate bonds with metals through two or more atoms of the organic compound. Chelating agents according to the present invention include, but are not limited to diethylenetriaminepentaacetic acid (DTPA), ethylenedinitrilotetraacetic acid (EDTA), ethylene glycol tetraacetic acid (EGTA) and N,N-bis(carboxymethyl)glycine (NTA) and furthermore, e.g. citrate or oxalate. According to a preferred embodiment, EDTA is used as anticoagulant. As used herein, the term "EDTA" indicates *inter alia* the EDTA portion of an EDTA compound such as, for example, K₂EDTA, K₃EDTA or Na₂EDTA. Using a chelating agent such as EDTA also has the advantageous effect that nucleases such as DNases and RNases are inhibited, thereby e.g. preventing a degradation of extracellular nucleic acids by nucleases. Therefore, the use of a chelating agent such as EDTA is also advantageous when using cell-containing samples different from blood. Furthermore, it was found that EDTA used/added in higher concentrations supports the stabilizing effect. However, EDTA alone does not achieve a sufficient stabilization effect for the purposes described herein. However, used in combination with the teachings of the present invention, in particular in combination with a poly(oxyethylene) polymer (and one or more of the further stabilizing agents as described herein, such as preferably a primary, secondary or tertiary amide and/or a caspase inhibitor), it can further improve the stabilization effect for the above discussed reasons.

According to one embodiment, the concentration of the chelating agent, preferably EDTA, in the mixture that is obtained when contacting the cell-containing biological sample with the poly(oxyethylene) polymer and optionally one or more additional additives lies in a range selected from the group consisting of 0.5 to 40mg/ml, 1 to 30 mg/ml, 1.6 to 25 mg/ml, 5 to 20 mg/ml and 7.5 to 17.5 mg/ml. Respective concentrations are particularly effective when stabilizing blood, plasma and/or serum samples. Suitable concentrations can also be determined by the skilled person.

Additional additives can also be used in order to further support the stabilization of the cell-containing sample, respectively support the preservation of the extracellular nucleic acid population. Examples of respective additives include but are not limited to nuclease inhibitors, in particular RNase and DNase inhibiting compounds. When choosing a respective further additive for supporting stabilization, care should be taken not to compromise and/or counteract the stabilizing effect. Thus, no additives such as e.g. chaotropic agents should be used in concentrations that result in or support the lysis and/or degradation of nucleated cells contained in the cell-containing biological sample that is stabilized and/or which support the degradation of the nucleic acids contained in the cell-free fraction of said biological sample. Therefore, preferably, the stabilization method described herein does not involve the use of additives (i) that induce or promote lysis of nucleated cells, (ii) that induce or promote lysis of cells in general and/or (iii) that lead to a degradation of nucleic acids contained in the cell-free fraction of the cell-containing biological sample. As the stabilization method described herein is not based on cell lysis but preserve cells, cells can be separated from the cell-containing sample after the stabilization period, thereby allowing to obtain a cell-free or cell-depleted fraction which comprises the extracellular nucleic acid population. Due to the poly(oxyethylene) polymer based stabilization described herein, said extracellular nucleic acid population substantially corresponds to or at least closely resembles the extracellular nucleic acid population present at the time of sample collection and stabilization. Furthermore, nucleic acids can be isolated from the separated cells and are available for analysis. As described above, a combination comprising a compound according to formula 1 also has transcriptome stabilizing properties. By stabilizing the transcriptome in addition to the extracellular nucleic acid population, the respectively stabilized samples are also suitable for gene expression profiling. Furthermore, respectively stabilized samples allow, if desired, the separate analysis of the extracellular and intracellular nucleic acid populations from the same stabilized sample.

In an advantageous embodiment, the cell-containing biological sample, which preferably is a blood sample or a sample derived from blood such as plasma or serum, is contacted with:
a) at least one high molecular weight poly(oxyethylene) polymer having a molecular weight of at least 1500, preferably in a range of 1500 to 50000, 2000 to 40000, 2500 to 30000, 2500 to 25000, more preferred 3000 to 20000 or 4500 to 10000;
b) one or more compounds according to formula 1, preferably in a concentration so that the concentration in the mixture with the cell-containing biological sample lies in a range of 0.25% to 5%, 0.3% to 4%, 0.4% to 3%, 0.5% to 2% or 0.75% to 1.5%;
c) at least one caspase inhibitor, preferably a pancaspase inhibitor, more preferred Q-VD-OPh, preferably in a concentration so that the concentration of the caspase inhibitor in the mixture with the cell-containing biological sample lies in a range of 0.1µM to 20µM, more preferred 0.5µM to 10µM, more preferred 1µM to 10µM, more preferred 3µM to 7.5µM or 3µM to 5µM;
d) optionally at least one further poly(oxyethylene) polymer having a molecular weight that is at least 100, preferably at least 200, at least 300 or at least 400 below the molecular weight of the high molecular weight poly(oxyethylene) polymer used and wherein said further poly(oxyethylene) polymer preferably is a low molecular weight poly(oxyethylene) having a molecular weight of 1000 or less, preferably having a molecular weight in a range of 200 to 800 or 200 to 600;
e) optionally a chelating agent, more preferably EDTA.

In an advantageous embodiment, the cell-containing biological sample, which preferably is a blood sample or a sample derived from blood such as plasma or serum, is contacted with:
a) at least one high molecular weight poly(oxyethylene) polymer, preferably polyethylene glycol, having a molecular weight in a range of 2000 to 40000, 2500 to 30000, 3000 to 25000, 3500 to 20000 or 4000 to 15000;
b) one or more compounds according to formula 1, preferably butanamide and/or an N,N-dialkyl propanamide;
c) at least one caspase inhibitor, preferably a pancaspase inhibitor, more preferred Q-VD-OPh;
d) at least one low molecular weight poly(oxyethylene), preferably polyethylene glycol, having a molecular weight in a range of 100 to 1000, 150 to 800 or 200 to 600;
e) optionally a chelating agent, more preferably EDTA,
wherein after the cell-containing biological sample has been contacted with said additives and optionally further additives used for stabilization the resulting mixture comprises
- the high molecular weight poly(oxyethylene) polymer in a concentration that lies in a range of 0.25% to 1.25% (w/v), 0.3% to 1% (w/v) or 0.4% to 0.75% (w/v),
- the one or more compounds according to formula 1 in a concentration that lies in a range of 0.3% to 4%, 0.4 to 3% or 0.5 to 2%,
- the caspase inhibitor in a concentration that lies in a range of 1µM to 10µM, preferably 3µM to 7.5µM, and
- the low molecular weight poly(oxyethylene) polymer in a concentration that lies in the range of 1.5% to 10%, 2% to 8%, 2.5 to 7% and 3% to 6%.

According to one embodiment, the method according to the present invention is for stabilizing an extracellular nucleic acid population comprised in a blood sample and comprises contacting the blood sample with at least one poly(oxyethylene) polymer, preferably a high molecular weight poly(oxyethylene) polymer, preferably a polyethylene glycol, and an anticoagulant, wherein during the stabilization period, the release of genomic DNA from cells contained in the blood sample into the cell-free portion of the blood sample is reduced. In particular, the present invention provides a method for stabilizing an extracellular nucleic acid population comprised in a blood sample which comprises contacting the blood sample with at least one poly(oxyethylene) polymer, one or more primary, secondary or tertiary amides (suitable and preferred examples and concentrations are described above), at least one caspase inhibitor and an anticoagulant and wherein the release of genomic DNA from nucleated cells contained in the blood sample into the cell-free portion of the blood sample is reduced. In particular, lysis of white blood cells is prevented/reduced during stabilization. Furthermore, degradation of nucleic acids present in the sample is reduced due to the stabilization.

In one embodiment, the cell-containing biological sample is a blood sample which is contacted with:
a) at least one high molecular weight poly(oxyethylene) polymer having a molecular weight that lies in a range of 2000 to 40000, 2500 to 30000, 2500 to 25000, 3000 to 20000, 3500 to 15000 or 3000 to 10000;
b) one or more compounds according to formula 1;
c) at least one caspase inhibitor, preferably a pancaspase inhibitor, more preferred Q-VD-OPh;
d) at least one low molecular weight poly(oxyethylene) polymer having a molecular weight of 1000 or less, preferably in a range of 100 to 800, 200 to 600 or 200 to 500;
e) an anticoagulant which preferably is a chelating agent, more preferably EDTA,
wherein after the blood sample has been contacted with said additives and optionally further additives used for stabilization the resulting mixture comprises
- the high molecular weight poly(oxyethylene) polymer in a concentration that lies in a range of 0.2% to 1.5% (w/v), 0.25% to 1.25% (w/v), 0.3% to 1% (w/v) or 0.4% to 0.75% (w/v),
- the one or more compounds according to formula 1 in a concentration that lies in a range of 0.3% to 4%, preferably 0.5 to 3%, more preferred 0.5% to 2% or 0.75% to 1.5%,
- the caspase inhibitor in a concentration that lies in a range of 1µM to 10µM, preferably 3µM to 7.5µM, and
- the low molecular weight poly(oxyethylene) polymer in a concentration that lies in the range of 1.5% to 10%, preferably 2% to 6%.

According to one embodiment, the method is for stabilizing an extracellular nucleic acid population comprised in a blood sample and comprises contacting the blood sample with at least one poly(oxyethylene) polymer, butanamide and/or an N,N-dialkylpropanamide such as N,N-dimethylpropanamide, at least one caspase inhibitor, and an anticoagulant, wherein the release of genomic DNA from cells contained in the blood sample into the cell-free portion of the blood sample is reduced. Preferably, said stabilization effect is achieved for at least three days, more preferred up to 6 days and longer. As described above, the release of DNA from the contained cells throughout the stabilization period preferably does not exceed a maximum of 2-fold, preferably is even approx. 1.5-fold or less.

In embodiments, a further advantage when stabilizing blood samples using the method according to the present invention is that hemolysis can be significantly reduced during the stabilization period. In one embodiment, the poly(oxyethylene) polymer and the one or more additional agents used for stabilization are comprised in a stabilization composition that contains water. The examples demonstrate that this embodiment signicficantly reduces the hemolysis of the red blood cells compared to unstabilized samples or standard EDTA blood samples and even compared to other stabilized samples. Hemolysis is the rupturing of erythrocytes and the release of their cytoplasm into surrounding extracellular fluid, e.g. blood plasma. The degree of hemolysis can be analysed e.g. by visual inspection, as the released hemoglobin will cause the serum or plasma to appear red. Most causes of *in vitro* hemolysis are related to specimen collection. However, *in vitro* hemolysis usually also occurs in a blood sample during ex *vivo* storage if no proper stabilization method is used. Depending on the extracellular nucleic acid of interest, hemolysis can be a considerable problem. If the extracellular nucleic acid of interest is DNA, hemolysis is less of a problem because red blood cells do not contain a nucleus and consequently, do not contain genomic DNA. Therefore, no intracellular DNA is released from the red blood cells during hemolysis. When the extracellular nucleic acid of interest is DNA, in particular the lysis or decay of white blood cells is a problem because in this case genomic DNA is released in addition to intracellular RNA. Therefore, when the extracellular nucleic acid of interest is extracellular DNA, in particular the lysis of white blood cells must be avoided. White blood cells may differ among each other in their stability characteristics. Thus, some types of white blood cells are more stable than others. However, generally, white blood cells are significantly more stable than red blood cells. Therefore, the lysis of red blood cells does not necessarily indicate that white blood cells were lysed. The different susceptibility of white blood cells and red blood cells to lysis is also used in the art to e.g. specifically lyse red blood cells, while preserving white cells in order to allow e. g. the collection of white blood cells. However, if the extracellular nucleic acid of interest is RNA, hemolysis and thus the lysis of red blood cells does constitute a problem. Mature red blood cells also do not contain RNA, however, their precursors (reticulocytes) do. Reticulocytes make up approximately 0.5% to 1% of the red blood cells and contain large amounts of globin RNA. Therefore, in particular when the extracellular nucleic acid of interest is RNA, it is advantageous to reduce or prevent lysis of red blood cells and thus reticulocytes during storage in order to reduce a dilution of the extracellular nucleic acid population, in particular the extracellular RNA population, with globin mRNA. Furthermore, as described above, it is advantageous to maintain the composition and thus profile of the extracellular nucleic acid population. As is shown by the examples, in embodiments, hemolysis is efficiently reduced when using the stabilization method according to the present invention. This is particularly the case where a stabilization composition comprising water is used. Thereby, the extracellular nucleic acid population is substantially preserved and furthermore, the stabilized blood sample, in particular the plasma or serum obtained from the stabilized blood sample, is due to the prevention of hemolysis and cell lysis in general also suitable for other standard laboratory analyses.

The poly(oxyethylene) polymer may be comprised in a stabilizing composition. If one or more further stabilizing agents and additives are additionally used, they may and preferably are also be comprised in the stabilization composition. Preferably, a stabilizing composition according to the third aspect of the present invention is used for stabilization of the cell-containing biological sample.

Furthermore, as described according to one alternative of the first aspect, the cell-containing biological sample to be stabilized is contacted with mono-ethylene glycol (1,2-ethanediol) as stabilizing agent. Mono-ethylene glycol may be used in the concentrations described above for the low molecular weight poly(oxyethylene) polymer. It is referred to the respective disclosure which also applies here. In particular, the cell-containing biological sample to be stabilized may be contacted with mono-ethylene glycol and any one or more of the other stabilizing agents described herein, preferably with at least one caspase inhibitor and/or with at least one primary, secondary or tertiary amide, which preferably is a compound according to formula 1 as defined above. Mono-ethylene glycol may be used in combination with the at least one poly(oxyethylene) polymer. Suitable embodiments and concentration ranges for the respective stabilizing agents are described above and also apply to the embodiment, wherein mono-ethylene glycol is used in order to stabilize or support the stabilization of a cell-containing sample and the exracellular nucleic acid population comprised therein.

The components of the stabilizing composition can be comprised, respectively can be dissolved in a solvent, e.g. water, a buffer, e.g. a biological buffer such as MOPS, TRIS, PBS and the like. Furthermore, the components may be dissolved in or the stabilizing composition may comprise a polar aprotic solvent such as dimethyl sulfoxide (DMSO). As is demonstrated by the examples, using a stabilization composition that contains water is particularly preferred when stabilizing a blood sample, as this embodiment significantly reduces hemolysis.

The poly(oxyethylene) polymer and the optionally used further stabilizing agents and/or additives can be present in a device, preferably a container, for collecting the cell-containing biological sample. The poly(oxyethylene) polymer and optionally the one or more compounds additionally used for stabilization can be present in a stabilizing composition that is present in a respective device or can be present as separate entities. Furthermore, they can be added to a respective collection device prior to collection of the cell-containing biological sample, or can be added to the collection device after the cell-containing biological sample was collected therein. It is also within the scope of the present invention to add the stabilizing agents and optionally, further additive(s) used separately to the cell-containing biological sample. However, for the ease of handling, it is preferred that the poly(oxyethylene) polymer and any further stabilizing agents and/or additives used are provided in the respective collection device, e.g. in form of a single composition. However, they may also be present as separate components or compositions in the collection device. In an advantageous embodiment, the at least one poly(oxyethylene) polymer, the one or more primary, secondary or tertiary amides, the at least one caspase inhibitor, and optionally further additive(s) such as e.g. an anticoagulant such as EDTA, are present in the collection device prior to adding the cell-containing biological sample. This ensures that the cell-containing biological sample is rapidly stabilized upon contact with the stabilizing agents used according to the teachings of the present invention. The stabilization agents are present in the container in an amount effective to stabilize the amount of cell-containing biological sample to be collected, respectively contained in said collection device. Suitable and preferred embodiments for a respective collection device are also described subsequently in conjunction with the fifth embodiment and it is referred to said disclosure. The same applies when using mono-ethylene glycol as stabilizing agent.

Preferably, the cell-containing biological sample is contacted with the poly(oxyethylene) polymer and optionally further additives directly after and/or during the collection of the cell-containing biological sample. Therefore, as described above, preferably, the agents used for stabilization are provided in form of a stabilizing composition. Preferably, said stabilizing composition is provided in a liquid form. It can be e.g. pre-filled in the sample collection device so that the cell-containing biological sample is rapidly stabilized during collection. According to one embodiment, the stabilizing composition is contacted with the cell-containing sample in a volumetric ratio selected from 10:1 to 1:20, 5:1 to 1:15, 1:1 to 1:10, 1:10 to 1:5 and 1:7 to 1:5, in particular about 1:6. These ratios are particularly useful for stabilizing blood samples. For stabilizing blood samples it is preferred that the stabilization composition contains water. Suitable and preferred concentrations of the additives in the resulting mixture with the cell-containing sample, in particular a blood sample, were described above and it is referred to the respective disclosure. It is a particular advantage of teachings of the present invention that stabilization of a large sample volume can be achieved with a small volume of the stabilizing composition according to the present invention. This particularly, if a high molecular weight poly(oxyethylene) polymer as described above is used as this allows to use lower concentrations compared to a low molecular weight poly(oxyethylene) polymer. Therefore, preferably, the ratio of stabilizing composition to sample lies in a range from 1:10 to 1:5, in particular 1:7 to 1:5, such as about 1.6.

The term "cell-containing biological sample", "cell-containing sample" and similar terms as used herein, in partiuclar refers to a sample which comprises at least 50, 250, at least 500, at least 1000, at least 1500, at least 2000 or at least 5000 cells. According to one embodiment, the cellular portion makes up at least 1%, at least 2%, at least 2.5%, at least 5%, preferably at least 10%, at least 15%, at least 20%, more preferably at least 25%, at least 30%, at least 35% or at least 40% of the cell-containing biological sample. Cell-containing samples comprising considerably more cells, wherein the cellular fraction makes up more than 40% can also be stabilized using the teachings described herein. However, the term "cell-containing biological sample" also refers to and thus encompasses cell-depleted samples, including cell-depleted samples that are commonly referred to as "cell-free" such as e.g. blood plasma as respective samples often include residual cells. At least, it can often not be fully excluded that even so-called "cell-free" samples such as blood plasma comprise residual amounts of cells which accordingly, pose a risk that the extracellular nucleic acid population becomes contaminated with intracellular nucleic acids released from said residual cells. Therefore, respective cell-depleted and "cell-free" samples are according to one embodiment also encompassed by the term "cell-containing biological sample". Thus, the "cell-containing sample" may comprise large amounts of cells, as is the case e.g. with whole blood, but may also only comprise merely minor amounts of cells. Hence, the term "cell containing biological sample" also encompasses samples that may only be suspected of or pose a risk of containing cells. As discussed above, also with respect to biological samples which only comprise minor, respectively residual amounts of cells such as e.g. blood plasma (blood plasma contains - depending on the preparation method - usually small residual amounts of cells, even though it is commonly referred to as being cell-free), the method according to the present invention has considerable advantages as these residual cells may also result in a undesired contamination of the comprised extracellular nucleic acids. Using the stabilizing method according to the present invention has the advantage that substantially irrespective of the composition of the cell-containing biological sample and the number of cells contained therein, the extracellular nucleic acid population contained in said sample can be substantially preserved, respectively stabilized, thereby allowing for standardizing the subsequent isolation and/or analysis of the contained extracellular nucleic acids.

According to one embodiment, the cell-containing biological sample is selected from the group consisting of body fluids and cell-containing samples derived from body fluids, in particular, whole blood, samples derived from blood such as plasma or serum, buffy coat, urine, sputum, lachrymal fluid, lymphatic fluid, sweat, liquor, cerebrospinal fluid, ascites, milk, stool, bronchial lavage, saliva, amniotic fluid, nasal secretions, vaginal secretions, semen/seminal fluid, wound secretions, cell culture and swab samples. According to one embodiment, the cell-containing biological sample is a body fluid, a body secretion or body excretion, preferably a body fluid, most preferably urine, lymphatic fluid, blood, buffy coat, plasma or serum. In particular, the cell-containing biological sample can be a circulating body fluid such as blood or lymphatic fluid. Preferably, the cell-containing biological sample that is stabilized using the teachings described herein is a blood sample, sometimes also referred to whole blood. The blood sample preferably has not been diluted or fractionated prior to stabilization. According to one embodiment, the blood sample is peripheral blood. According to one embodiment, the cell-containing biological sample was obtained from a human. The cell-containing biological sample comprises extracellular nucleic acids in the extracellular portion.

The term "extracellular nucleic acids" or "extracellular nucleic acid" as used herein, in particular refers to nucleic acids that are not contained in cells. Respective extracellular nucleic acids are also often referred to as cell-free nucleic acids. These terms are used as synonyms herein. Hence, extracellular nucleic acids usually are present exterior of a cell or exterior of a plurality of cells within a sample. The term "extracellular nucleic acids" refers e.g. to extracellular RNA as well as to extracellular DNA. Examples of typical extracellular nucleic acids that are found in the cell-free fraction (respectively portion) of biological samples such as e.g. body fluids include but are not limited to mammalian extracellular nucleic acids such as e.g. extracellular tumor-associated or tumor-derived DNA and/or RNA, other extracellular disease-related DNA and/or RNA, epigenetically modified DNA, fetal DNA and/or RNA, small interfering RNA such as e.g. miRNA and siRNA, and non-mammalian extracellular nucleic acids such as e.g. viral nucleic acids, pathogen nucleic acids released into the extracellular nucleic acid population e.g. from prokaryotes (e.g. bacteria), viruses, eukaryotic parasites or fungi. The extracellular nucleic acid population usually comprises certain amounts of intracellular nucleic acids that were released from damaged or dying cells. E.g. the extracellular nucleic acid population present in blood usually comprises intracellular globin mRNA that was released from damaged or dying cells. This is a natural process that occurs *in vivo.* Such intracellular nucleic acid present in the extracellular nucleic acid population can even serve the purpose of a control in a subsequent nucleic acid detection method. The stabilization method described herein in particular reduces the risk that the amount of intracellular nucleic acids, such as genomic DNA, that is comprised in the extracellular nucleic acid population is significantly increased after the cell-containing sample was collected due to the ex *vivo* handling of the sample. Thus, alterations of the extracellular nucleic acid population because of the ex *vivo* handling are reduced and can even be prevented. According to one embodiment, the cell-containing biological sample is or is derived from a body fluid such as e.g. blood, plasma, serum, saliva, urine, liquor, cerebrospinal fluid, sputum, lachrymal fluid, sweat, amniotic fluid or lymphatic fluid. Herein, we refer to extracellular nucleic acids that are obtained from a circulating body fluid such as blood or lymphatic fluid as circulating extracellular nucleic acids or circulating cell-free nucleic acids. According to one embodiment, the term extracellular nucleic acids in particular refers to mammalian extracellular nucleic acids. Examples include but are not limited to diseaseassociated or disease-derived extracellular nucleic acids such e.g. as tumor-associated or tumor-derived extracellular nucleic acids, extracellular nucleic acids released due to inflammations or injuries, in particular traumata, extracellular nucleic acids related to and/or released due to other diseases, or extracellular nucleic acids derived from a fetus. The term "extracellular nucleic acids" or "extracellular nucleic acid" as described herein also refers to extracellular nucleic acids obtained from other cell-containing biological samples, in particular biological samples other than body fluids. Usually, a sample comprises more than one kind or type of extracellular nucleic acids.

The term "extracellular nucleic acid population" as used herein in particular refers to the collective of different extracellular nucleic acids that are comprised in a cell-containing sample. A cell-containing sample usually comprises a characteristic and thus unique extracellular nucleic acid population. Thus, the type, kind, ratio and/or the the amount of one or more extracellular nucleic acids comprised in the extracellular nucleic acid population of a specific sample may be important sample characteristics. As discussed above, it is therefore important to stabilize and thus to substantially preserve said extracellular nucleic acid population at the state wherein the sample is collected, as its composition and/or the amount of one or more extracellular nucleic acids comprised in the extracellular nucleic acid population of a sample can provide valuable medical, prognostic or diagnostic information. Therefore, it is advantageous if the profile of the extracellular nucleic acid population is efficiently stabilized over the intended stabilization period. The stabilization technologies described herein reduce contaminations and hence a dilution of the extracellular nucleic acid population by intracellular nucleic acids, in partiuclar by genomic DNA, after sample collection and stabilization. Thus, a substantial preservation of the extracellular nucleic acid population is achieved. As is shown by the examples, changes in the extracellular nucleic acid population with respect to the quantity, the quality and/or the composition of the comprised extracellular nucleic acids, in particular changes attributable to an increase of released genomic DNA, are over the stabilization period considerably reduced compared to an unstabilized sample or a corresponding sample that is e.g. stabilized by EDTA in case of a blood sample or a sample derived from blood. According to one embodiment the increase in genomic DNA from T₀ (stabilization point) to a end of the stabilization period (preferably 48h, 72h or 96h after T₀) is reduced by at least 60%, at least 70%, at least 75%, at least 80%, at least 85%, at least 90% or at least 95% compared to an unstabilized sample or a corresponding sample that is e.g. stabilized by EDTA in case of a blood sample (e.g. 1.5 mg EDTA/ml stabilized blood sample) or a sample derived from blood. As is demonstrated by the examples, values above 80% and higher are achieved, in particular in embodiments, wherein a caspase inhibitor and at least one primary, secondary or tertiary amide is used in combination.

As is described above and as is demonstrated by the examples, using the methods of the present invention allows for stabilizing the cell-containing sample without refrigeration or freezing for a prolonged time period. Thus, the samples can be kept at room temperature or even at elevated temperatures e.g. up to 30°C or even up to 40°C. According to one embodiment, a stabilization effect is achieved for at least three days, preferably at least four days, more preferred at least 6 days. Preferably, during said stabilization periods the stabilization method according to the invention has the effect that cells contained in the sample are stabilized, that the release of genomic DNA from cells contained in the sample into the cell-free portion of the sample is reduced and/or that a degradation of nucleic acids present in the sample is reduced due to the stabilization. In particular, during the described stabilization periods, the stabilization reduces the dilution of the extracellular DNA population comprised in the biological sample with genomic DNA originating from cells contained in the stabilized sample during the stabilization period. The stabilizing effects that can be achieved with the method according to the present invention were described in detail above and it is referred to the above disclosure. Preferably, during said stabilization periods, the stabilization reduces the contamination of the extracellular nucleic acid population comprised in the biological sample with intracellular nucleic acids, in particular genomic DNA, originating from cells contained in the stabilized sample during the stabilization period. As is shown in the examples, blood samples could be stabilized up to 3 days or longer at room temperature.

Even during longer storages at room temperature for up to 6 days and even longer, the extracellular nucleic acid population was substantially stabilized (in particular compared to non-stabilized samples or e.g. compared to samples that were stabilized using standard methods such as an EDTA treatment) when using a poly(oxyethylene) polymer, preferably in combination with a caspase inhibitor and one or more primary, secondary or tertiary amides. As is demonstrated by the examples, even over very long stabilization periods of 10 days the stabilization effect was maintained. Generally, it may occur though that the stabilization effect may decrease over time, which may also depend on the source, e. g. the donor from which the cell-containing biological sample is derived, it generally will be still sufficient to preserve the composition of the extracellular nucleic acid population to allow the analysis and/or further processing of the extracellular nucleic acids. Thus, cell-containing biological samples that were stabilized according to the methods of the present invention and in particular samples that were stabilized with a poly(oxyethylene) polymer, one or more primary, secondary or tertiary amides and a caspase inhibitor were still suitable for isolating and analysing the extracellular nucleic acids contained therein even after prolonged storage at room temperature. Thus, even longer storage/shipping times are conceivable. However, usually, longer periods are not necessary, as the regular storage and e.g. shipping time to the laboratory, wherein the nucleic acid isolation and optionally the analysis is performed, usually does not exceed 6 or 7 days, but usually is even completed after two or three days. As is shown in the examples, the stabilization efficiency is particularly good during this time period. However, the long stabilization times and stabilization efficiencies that are achievable with the method according to the present invention provides an important safety factor.

The methods and also the subsequently described stabilizing compositions according to the present invention allow the stabilization of large volumes of cell-containing biological samples with small volumes/amounts of added stabilizer because the at least one poly(oxyethylene) polymer which preferably is a polyethylene glycol and the described combinations of stabilizers used according to the teachings of the present invention for stabilization are highly active in particular in combination. This is an important advantage because the size/volume of the sample poses considerable restrains on the subsequent nucleic acid isolation procedure in particular when intending to use automated processes for isolating the extracellular nucleic acids contained in the samples. Furthermore, one has to consider that extracellular nucleic acids are usually only comprised in small amounts in the cell-containing biological sample. Thus, processing larger volumes of a cell-containing biological sample such as e.g. a blood sample has the advantage that more extracellular nucleic acids can be isolated from the sample and thus are available for a subsequent analysis. According to one embodiment, the sample volume that is stabilized using the method of the first aspect is selected from 1 to 50ml, 2 to 35ml, 3 to 25ml, 4 to 20ml and 5 to 15ml.

The stabilization methods as disclosed herein, provide a significant advantage over state-ofthe-art stabilization methods that are used for stabilizing the extracellular nucleic acid population in a cell-containing sample which are based on the use of cross-linking reagents, such as formaldehyde, formaline, formaldehyde releasers and the like. Crosslinking reagents cause inter- or intra-molecular covalent bonds between nucleic acid molecules or between nucleic acids and proteins. This cross-linking effect can significantly impair the subsequent isolation of nucleic acids from such stabilized samples and usually requires specifically adapted nucleic acid isolation procedures that allow the isolation from such samples. As, for example, the concentration of circulating nucleic acids in a whole blood sample is already relatively low, any measure which further reduces the yield of such nucleic acids should be avoided. This may be of particular importance when detecting and analyzing very rare nucleic acid molecules derived e.g. from malignant tumors or from a developing fetus in the first trimester of pregnancy. As is shown by the examples, the method of the invention does not require cross-linking agents for stabilization. Therefore, according to one embodiment, the stabilization method according to the present invention does not involve the use of a cross-linking agent that induces protein-nucleic acid and/or protein-protein crosslinks. In particular, the stabilization does not involve the use of formaldehyde, formaline, paraformaldehyde or a formaldehyde releaser. Furthermore, as described above, according to one embodiment, the stabilization method according to the invention does not involve the use of additives that classify as toxic agents.

After the stabilization period, the method may comprise one or more of the following
a) the stabilized sample is subjected to a nucleic acid analysis and/or detection method;
b) extracellular nucleic acids are isolated from the stabilized sample;
c) extracellular nucleic acids are isolated from the stabilized sample and the isolated nucleic acids are analysed and/or detected;
d) cells comprised in the stabilized sample are removed;
e) cells comprised in the stabilized sample are removed prior to performing an nucleic acid isolation, analysis and/or detection step;
f) cells are removed from the stabilized sample and extracellular nucleic acids are isolated from the cell-free or cell-depleted portion of the stabilized sample;
g) (i) the stabilized sample, (ii) the stabilized sample from which cells have been removed and/or (iii) cells removed from the sample are stored;
h) cells are removed from the stabilized sample and are discarded; and/or
i) cells are removed from the stabilized sample and nucleic acids are isolated from cells that were removed from the stabilized sample;
j) cells are removed from the stabilized sample and extracellular nucleic acids are isolated from the cell-free or cell-depleted portion of the stabilized sample using a size selective nucleic acid isolation method.

Hence, the cell-containing biological sample that was stabilized using the method of the present invention can be analysed in a nucleic acid analytic and/or detection method and/or may be further processed. The stabilization of the biological sample may either be followed directly by techniques for analysing nucleic acids, or nucleic acids may first be isolated from the stabilized sample. Details regarding the nucleic acid isolation and analysis are also described below in conjunction with the second aspect of the present invention and it is referred to said disclosure.

### B. NUCLEIC ACID ISOLATION METHOD

According to a second aspect, a method for isolating extracellular nucleic acids from a cell-containing biological sample is provided comprising the steps of
a) stabilizing the cell-containing biological sample according to the method defined in the first aspect of the present invention; and
b) isolating extracellular nucleic acids from the stabilized sample.

In step a), the extracellular nucleic acid population comprised in the cell-containing sample is stabilized according to the method described in the first aspect of the present invention. As discussed above, the stabilization according to the present invention has the effect that the extracellular nucleic acid population contained in the sample may be substantially preserved in the state it had shown at the time the biological sample was obtained, respectively collected, over the stabilization period. In particular, the usually observed high increase in nucleic acids that results from intracellular nucleic acids, in particular genomic DNA, more specifically fragmented genomic DNA, during storage/handling is efficiently reduced or even prevented as is demonstrated in the examples. Without being bound in theory, it is believed that the poly(oxyethylene) polymer based stabilization described herein stabilizes cells and/or reduces the destruction of cells during the stabilization period, thereby reducing the release of intracellular nucleic acids. The method allows to separate a cell fraction from the stabilized sample after the desired stabilization period. Hence, extracellular nucleic acids obtained from a respectively stabilized sample comprise significantly less contamination with intracellular nucleic acids originating from degraded or dying cells and in particular comprise less amounts of fragmented genomic DNA compared to non-stabilized samples. Furthermore, the stabilization according to the present invention does not require and preferably does not involve the use of cross-linking agents. This is an important advantage over prior art methods which involve the use of cross-linking agents such as formaldehyde, formaline or formaldehyde releasers, because these reagents often reduce the recoverable amount of extracellular nucleic acids due to cross-linking when using standard nucleic acid isolation techniques. Furthermore, as described above, the stabilization described herein allows the sample to be stored and/or handled, e.g. transported, - even at room temperature - for a prolonged period of time prior to separating the cells contained in the sample and/or prior to isolating nucleic acids comprised therein in step b). With respect to the details of the stabilization that is performed in step a), it is referred to the above disclosure which also applies here. Non-limiting embodiments are again described briefly in the following.

According to one embodiment, the cell-containing biological sample such as e.g. a whole blood sample is stabilized in step a) using
- a high molecular weight poly(oxyethylene) polymer having a molecular weight of at least 1500 as described above, optionally in combination with a further poly(oxyethylene) polymer having a molecular weight that is at least 100 lower than the high molecular weight polymer, such as a low molecular weight poly(oxyethylene) polymer having a molecular weight of 1000 or less,
- at least one caspase inhibitor,
- one or more primary, secondary and tertiary amides and
- optionally, further stabilizing agents and/or additives.

Suitable and preferred embodiments of the stabilization method according to the present invention that is performed in step a) and the compounds used for stabilization were described above and it is referred to the above disclosure which also applies here. Particularly preferred is the additional use of a low molecular weight poly(oxyethylene) polymer, at least one caspase inhibitor, and butanamide and/or an N,N-dialkylpropanamide such as preferably N,N-dimethlypropanamide for stabilization. Preferred is the combination with an anticoagulant, preferably a chelating agent such as EDTA, when stabilizing a whole blood sample. Furthermore, as described avove, according to one alternative of the first aspect, the cell-containing biological sample to be stabilized is contacted with mono-ethylene glycol (1,2-ethanediol) as stabilizing agent. It is referred to the above disclosure.

If the cell-containing biological sample comprises large amounts of cells as is e.g. the case with whole blood, the cells are separated from the remaining sample in order to obtain a cell-free, respectively cell-reduced or cell-depleted fraction of the stabilized sample from which the extracellular nucleic acids are then isolated in step b). Thus, according to one embodiment, cells are removed from the cell-containing sample between step a) and step b). This intermediate step may be obsolete if samples are processed which merely comprise minor amounts of residual cells such as e.g. plasma or serum and/or wherein the extracellular nucleic acid of interest is DNA. Due to the stabilization of the invention, the release of genomic DNA during the stabilization period from the contained cells is reduced or even prevented and furthermore, in particular when using a caspase inhibitor in addition, the fragmentation of genomic DNA is reduced. As described herein, due to its considerably larger size, unfragmented genomic DNA can be distinguished from the smaller extracellular DNA. This allows to selectively isolate extracellular DNA even in the presence of unfragmented genomic DNA by using a size selective isolation protocol. However, in order improve the results, it is preferred that cells (or potentially remaining cells) are removed from the stabilized sample prior to isolating the extracellular nucleic acids in step b) in order to reduce contaminations of the extracellular nucleic acid population with intracellular nucleic acids that would otherwise be released from the cells during nucleic acid isolation. To remove the contained cells is also advantageous if the extracellular nucleic acids of interest are RNA, because it can be difficult to distinguish intracellular RNA from extracellular RNA and furthermore, a dilution of the extracellular RNA can thereby be prevented. A cell removal step prior to step b) is generally advantageous and thus preferred, also if the extracellular nucleic acid of interest is DNA, because this allows to use standard nucleic acid isolation procedures in step b).

Depending on the type of cell-containing biological sample, cells, including residual cells, can be separated and removed e.g. by centrifugation, preferably high speed centrifugation, or by using means other than centrifugation, such as e.g. filtration, sedimentation or binding to surfaces e.g. on (optionally magnetic) particles if a centrifugation step is to be avoided. Respective cell separation methods are well-known in the prior art and thus, do not need to be described in detail. Respective cell removal steps can also be easily included into an automated sample preparation protocol. Respectively removed cells may also be processed further if desired. The cells can e.g. be stored, analysed and/or biomolecules such as e.g. nucleic acids or proteins can be isolated from the removed cells. Furthermore, it was found that intracellular nucleic acids such as intracellular RNA can be stabilized in particular when additionally using a compound according to formula 1 such as DMPA for stabilizing the cell-containing sample. The additional stabilization of the transcriptome is advantageous as it allows e.g. to analyse profiles of transcripts in the isolated intracellular nucleic acids which can also be important biomarkers for *in vitro* diagnostics.

Furthermore, it is also within the scope of the present invention to include further intermediate steps to work up the stabilized sample.

Extracellular nucleic acids are isolated in step b), preferably from the cell-free, respectively cell-depleted fraction of the stabilized sample, e.g. from supernatants or from plasma and/or serum in case the stabilized cell-containing sample was a blood sample. For isolating extracellular nucleic acids, any known nucleic acid isolation method can be used that is suitable for isolating nucleic acids from the stabilized sample, respectively the obtained cell-depleted sample. Examples for respective purification methods include but are not limited to extraction, solid-phase extraction, silica-based purification methods, magnetic particle-based purification, phenol-chloroform extraction, chromatography, anion-exchange chromatography (using anion-exchange surfaces), electrophoresis, filtration, precipitation and combinations thereof. It is also within the scope of the present invention to specifically isolate specific target extracellular nucleic acids, e.g. by using appropriate probes coupled to a solid support that enable a sequence specific binding. Also any other nucleic acid isolating technique known by the skilled person can be used.

According to one embodiment, nucleic acids are isolated in step b) using a chaotropic agent and/or alcohol. Preferably, the nucleic acids are isolated by binding them to a solid phase, preferably a solid phase comprising silica or carrying anion exchange functional groups. Respective methods are well-known in the prior art and thus, do not need any detailed description. Suitable methods and kits for isolating extracellular nucleic acids are also commercially available such as the QIAamp^{®} Circulating Nucleic Acid Kit (QIAGEN), the Chemagic Circulating NA Kit (Chemagen), the NucleoSpin Plasma XS Kit (Macherey-Nagel), the Plasma/Serum Circulating DNA Purification Kit (Norgen Biotek), the Plasma/Serum Circulating RNA Purification Kit (Norgen Biotek), the High Pure Viral Nucleic Acid Large Volume Kit (Roche) and other commercially available kits suitable for extracting and purifying extracellular nucleic acids. As described above, in particular the embodiment wherein a high molecular weight poly(oxyethylene) polymer is used in combination with a low molecular weight poly(oxyethylene) polymer is advantageous, as it allows to isolate the extracellular nucleic acids with high yield using a broad range of nucleic acid isolation procedures. As described above, e.g. in combination with a subsequent nucleic acid isolation method that involves the use of a silica column, it is preferred to use a high molecular weight poly(oxyethylene) polymer having a molecular weight of at least 2000, preferably at least 3000, more preferred in a range of 4500 to 10000, in combination with a low molecular weight poly(oxyethylene) polymer having a molecular weight of 1000 or less, preferably in a range of 150 to 700, more preferred 200 to 600. As described above, this combination of polymers allows to reduce the amount of high molecular weight poly(oxyethylene) polymer required for efficient stabilization to e.g. 1.5% (w/v) or less, 1.25% (w/v) or less, 1% (w/v) or less or also 0.75% (w/v) or less in the stabilized mixture that contains the cell-containing sample to be stabilized. In the tested examples, these lower concentrations of the high molecular weight polymer in combination with the low molecular weight polymer substantially showed no impairment on the subsequent nucleic acid isolation even when using silica columns, while achieving a strong stabilization effect. Furthermore, as a combination of polymers was used, also the amount of low molecular weight polymer could be lowered so that the required volume of stabilization composition was kept in an acceptable range. However, as is demonstrated in the examples, respective lower concentrations of the high molecular weight poly(oxyethylene) polymer of 1.5% (w/v) or less, 1.25% (w/v) or less, 1% (w/v) or less or also 0.75% (w/v) or less may also be used in the absence of a low molecular weight poly(oxyethylene) polymer, if further stabilizing agents are used, preferred embodiments of such stabilizing agents are described herein.

According to one embodiment, nucleic acids are isolated in step b) by binding them to a solid phase comprising anion exchange groups. Suitable anion exchange groups are for example provided by amine groups. Binding preferably occurs at a pH below 7. Such anion exchange based nucleic acid isolation methods are known to the skilled person. According to one embodiment, the nucleic acids are extracellular nucleic acids. Suitable anion exchange based methods are e.g. described in WO 2013/045432, herein incorporated by reference. The described method is particularly suitable for isolating exctracellular nucleic acids, such as extracellular DNA, from plasma that was obtained from a blood sample that was stabilized using the stabilization method described herein.

According to one embodiment, total nucleic acids are isolated from the stabilized cell-containing sample that is obtained after step a) or optionally the sample that is obtained after cells were removed from the stabilized cell-containing sample in an intermediate step. Preferably, the nucleic acids are isolated from the stabilized sample, or a cell-free, respectively cell-depleted fraction of the stabilized sample. E.g. total nucleic acids can be isolated from plasma or serum and the extracellular nucleic acids will be comprised as portion in these extracted nucleic acids. If the cells contained in the stabilized sample are efficiently removed prior to nucleic acid isolation, the isolated total nucleic acids will predominantly comprise or even consist of extracellular nucleic acids.

It is also within the scope of the present invention to isolate at least predominantly a specific target nucleic acid. A target nucleic acid can be e.g. a certain type of extracellular nucleic acid, e.g. DNA or RNA, including mRNA, microRNA, other non-coding nucleic acids, epigenetically modified nucleic acids, and other nucleic acids. E.g. the target extracellular nucleic acid can be DNA and the non-target extracellular nucleic acid can be RNA or *vice versa.* Target specific nucleic acid isolation methods which specifically aim at isolating DNA or RNA are also well known in the prior art and thus, do not need any detailed description herein. According to one embodiment, the non-target nucleic acid is destroyed by adding an appropriate enzyme which specifically destroys the non-target nucleic acid, e.g. a RNase if the target nucleic acid is DNA or a DNase if the target nucleic acid is RNA. Said enzyme can be added to the lysis or binding mixture or can be added after extracellular nucleic acids were bound to a solid phase. Suitable embodiments for performing a respective non-target nucleic acid digestion step are known in the prior art and thus, do not need any further description herein. According to one embodiment which is feasible if DNA and RNA are bound to a solid support, elution conditions selective for the target nucleic acid can be applied to predominantly and thus selectively recover the target nucleic acid from the solid support. According to one embodiment, an isolation method is used, wherein the target nucleic acid, e.g. DNA, is selectively bound to a solid phase under conditions wherein non-target nucleic acids, e.g. RNA do not bind. Suitable binding conditions are well-known in the prior art and are e.g. described in WO 95/21849. According to one embodiment, the non-target nucleic acid is removed by binding at least a portion of the non-target nucleic acid under appropriate conditions to a solid phase and then separating the non-target nucleic acid bound to the solid phase from the remaining sample comprising the target extracellular nucleic acid. This can be achieved e.g. by the addition of a suitable solid phase under conditions wherein mainly the non-target nucleic acids e. g. DNA are bound to the solid phase while the non-target nucleic acid, e.g. RNA, remains in the sample and is recovered therefrom in a separate step. Suitable methods for selectively removing a non-target nucleic acid from a target nucleic acid are for example described in EP 0 880 537 and WO 95/21849, herein incorporated by reference. If desired, said non-target nucleic acid may also be further used, e.g. further processed such as e.g. eluted from the solid phase. However, it may also be discarded. It is also within the scope of the present invention to e.g. digest the non-target nucleic acid or remainders thereof using nucleases after isolation of the target nucleic acid.

The term target nucleic acid may also refer to a specific kind of nucleic acid, e.g. a specific extracellular nucleic acid that is known to be a certain disease marker. As discussed above, the isolation of extracellular nucleic acids may also comprise the specific isolation of a respective target nucleic acid e.g. by using appropriate capture probes which support the selective isolation of the target nucleic acid.

The term target nucleic acid may also refer to nucleic acids having a certain length, e.g. a nucleic acid having a length of 5000nt or less, 2000nt or less, 1000nt or less, 900nt or less, 800nt or less, 700nt or less, 600nt or less, 500nt or less, 400nt or less or 350nt or less. Isolating target nucleic acids of a certain maximum size can be advantageous in the context of the present invention. It is known that extracellular nucleic acids usually have a size of 1000nt or less and usually even 500nt or less. The sizes, respectively size ranges indicated herein refer to the chain length. I.e. in case of double-stranded nucleic acids such as double-stranded DNA it refers to bp. Selectively isolating smaller nucleic acids in step b) can increase the portion of extracellular nucleic acids obtained in the isolated nucleic acids. The stabilization methods according to the present invention allow, in particular due to the inhibition of the release of genomic DNA and/or the inhibition of the fragmentation of released genomic DNA, for a more efficient separation of such high molecular weight genomic DNA from the smaller extracellular nucleic acid population. As the substantial size difference between genomic DNA and extracellular nucleic acids is essentially preserved using the stabilization technology according to the present invention, genomic DNA can be removed more efficiently e.g. using a size selective nucleic acid isolation protocol. As the size difference between genomic DNA (usually larger than >10,000 bp) and extracellular nucleic acids (usually <1000 nt/bp) in a sample stabilized as described herein is usually relatively large due to the efficient stabilization, known methods for selectively isolating nucleic acids of a certain target length can be used. Thus, according to one embodiment, step b) comprises selectively isolating target nucleic acids having a length of 2000nt or less, 1500nt or less, 1000nt or less, 900nt or less, 800nt or less, 700nt or less, 600nt or less or 500nt or less. Suitable methods to achieve a respective size selective isolation of nucleic acids e.g. by depleting high molecular weight genomic DNA, are known in the prior art and thus, need no detailed description herein. A classic method for isolating DNA of a target size involves the separation of the DNA in a gel, cutting out the desired gel band(s) and then isolating the DNA of the target size from the gel fragment(s). Another widely used technology is the size selective precipitation with polyethylene glycol based buffers (Lis and Schleif Nucleic Acids Res. 1975 Mar;2(3):383-9) or the binding/precipitation on carboxylfunctionalized beads (DeAngelis et al, Nuc. Acid. Res. 1995, Vol 23(22), 4742-3; US 5,898,071 und US 5,705,628, commercialized by Beckman-Coulter (AmPure XP; SPRIselect) and US 6,534,262). Furthermore, size selective isolation methods that are based on the use of solid supports comprising anion exchange groups and varying pH values are known. A size selective isolation provides further opportunities in order to reduce the amount of intracellular nucleic acids in the isolated extracellular nucleic acids. For example, when the target extracellular nucleic acid of interest is DNA, the removal of genomic DNA during nucleic acid isolation step b) could also supplement or even replace a separate high g-force centrifugation of a plasma sample before starting the nucleic acid extraction in order to remove residual cells. Genomic DNA that is released from said residual cells is prevented from becoming massively degraded due to the stabilization according to the present invention, in particular if a caspase inhibitor is additionally used, and accordingly, said unfragmented or less fragmented genomic DNA can be depleted by using a size-selective nucleic acid isolation protocol in step b). This option is of particular advantage, as many clinical laboratories do not have a centrifuge capable of performing such a high g-force centrifugation or other means for removing in particular trace amounts of residual cells.

The isolated extracellular nucleic acids can then be analysed and/or further processed in a step c) using suitable assay and/or analytical methods. E.g. they can be identified, modified, contacted with at least one enzyme, amplified, reverse transcribed, cloned, sequenced, contacted with a probe, be detected (their presence or absence) and/or can be quantified. Respective methods are well-known in the prior art and are commonly applied in the medical, diagnostic and/or prognostic field in order to analyse extracellular nucleic acids (see also the detailed description in the background of the present invention). Thus, after extracellular nucleic acids were isolated in step b), optionally as part of total nucleic acids, total RNA and/or total DNA (see above), they can be analysed e.g. to identify the presence, absence or severity of a disease state including but not being limited to a multitude of neoplastic diseases, in particular premalignancies and malignancies such as different forms of tumors or cancers. E.g. the isolated extracellular nucleic acids can be analysed in order to detect diagnostic and/or prognostic markers (e.g., fetal- or tumor-derived extracellular nucleic acids) in many fields of application, including but not limited to non-invasive prenatal genetic testing respectively screening, disease screening, pathogen screening, oncology, cancer screening, early stage cancer screening, cancer therapy monitoring, genetic testing (genotyping), infectious disease testing, injury diagnostics, trauma diagnostics, transplantation medicine or many other diseases and, hence, are of diagnostic and/or prognostic relevance. According to one embodiment, the isolated extracellular nucleic acids are analyzed to identify and/or characterize a disease or a fetal characteristic. Thus, as discussed above, the isolation method described herein may further comprise a step c) of nucleic acid analysis and/or processing.

Therefore, according to one embodiment, the isolated extracellular nucleic acids are analysed in a step c) to identify, detect, screen for, monitor or exclude a disease and/or at least one fetal characteristic. The analysis/further processing of the isolated extracellular nucleic acids can be performed using any nucleic acid analysis/processing method including, but not limited to amplification technologies, polymerase chain reaction (PCR), isothermal amplification, reverse transcription polymerase chain reaction (RT-PCR), quantitative real time polymerase chain reaction (Q-PCR), digital PCR, gel electrophoresis, capillary electrophoresis, mass spectrometry, fluorescence detection, ultraviolet spectrometry, hybridization assays, DNA or RNA sequencing, next generation sequencing, restriction analysis, reverse transcription, nucleic acid sequence based amplification (NASBA), allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, or any combination thereof. Respective technologies are well-known to the skilled person and thus, do not need further description here.

According to one embodiment, either or both of isolation step b) and analysis step c) occur at least one day up to 3 days or two days up to 10 days after the cell-containing biological sample has been collected, respectively was stabilized according to the teachings of the present invention. Suitable time periods for which the cell-containing biological sample, in particular a blood sample, respectively the extracellular nucleic acid population contained therein can be stabilized using the method according to the present invention are also described above in conjuncton with the stabilization method and the respective disclosure also applies here. According to one embodiment, nucleic acid isolation step b) is performed at least one day, at least 2 days or at least 3 days after the cell-containing sample was collected and stabilized according to the method according to the present invention.

### C. STABILIZATION COMPOSITION

According to a third aspect, a composition suitable for stabilizing a cell-containing biological sample is provided comprising
i) a poly(oxyethylene) polymer as stabilizing agent or
ii) mono-ethylene glycol as stabilizing agent
and one or more, preferably two or more, further additives selected from the group consisting of
- one or more primary, secondary or tertiary amides;
- a caspase inhibitor;
- an anticoagulant and/or a chelating agent.

Preferably, the composition according to the third aspect comprises a poly(oxyethylene) polymer, which preferably is a high molecular weight poly(oxyethylene) polymer having a molecular weight of at least 1500, as stabilizing agent and furthermore comprises one or more, preferably two or more further additives selected from the group consisting of
- at least one further poly(oxyethylene) polymer having a molecular weight that is at least 100, preferably at least 200, at least 300 or at least 400 below the molecular weight of the first poly(oxyethylene) polymer which preferably is a high molecular weight poly(oxyethylene) polymer, wherein said further poly(oxyethylene) polymer preferably is a low molecular weight poly(oxyethylene) polymer having a molecular weight of 1000 or less;
- one or more primary, secondary or tertiary amides;
- a caspase inhibitor;
- an anticoagulant and/or a chelating agent.

The advantages and suitable and preferred embodiments are discussed above in conjunction with the stabilization method according to the first aspect and it is referred to the above disclosure which also applies here. As discussed above, the stabilizing compositions provided by the invention, in particular those comprising a poly(oxyethylene) polymer, which preferably is a high molecular weight poly(oxyethylene) polymer, and at least one caspase inhibitor, optionally but preferably in combination with one or more primary, secondary or tertiary amides, are particularly effective in stabilizing a cell-containing biological sample, in particular blood, plasma and/or serum, by stabilizing comprised cells and the comprised extracellular nucleic acids thereby substantially preserving, respectively stabilizing the extracellular nucleic acid population at the time of stabilization. A respective stabilizing composition allows the storage and/or handling, e.g. shipping, of the cell-containing biological sample, which preferably is blood, at room temperature for at least two, preferably at least three days or even longer without substantially compromising the quality of the blood sample, respectively the extracellular nucleic acid population contained therein. In particular, dilutions, respectively contaminations of the extracellular nucleic acid composition with intracellular nucleic acids, in particular fragmented genomic DNA, are reduced or even prevented over the stabilization period. Preferably, the stabilization composition is contacted with the cell-containing sample immediately after or during collection of the cell-containing biological sample. Furthermore, as described above, mono-ethylene glycol can be used either alternatively or in addition to the poly(oxyethylene) polymer as stabilizing agent. The preferably used combinations with the other stabilizing agents are essentially the same.

The stabilization composition comprises according to one embodiment a high molecular weight poly(oxyethylene) polymer. Details are described above in conjunction with the stabilization method according to the first aspect and it is referred to the respective disclosure. The high molecular weight poly(oxyethylene) polymer is preferably a polyethylene glycol, such as a unsubstituted polyethylene glycol. In embodiments, the molecular weight may lie in a range selected from 1500 to 40000, 2000 to 30000, 2500 to 25000, 3000 to 20000, 3500 to 15000, 4000 to 10000, 4500 to 9000 and 5000 to 8000. As described above, also higher molecular weights exceeding 40000 can be used.

According to one embodiment, the stabilization composition comprises a poly(oxyethylene) polymer that has a molecular weight of 1500 or less and in embodiments is a low molecular poly(oxyethylene) polymer having a molecular weight of 1000 or less. The low molecular weight poly(oxyethylene) polymer may have a molecular weight that lies in a range selected from 100 to 1000, 150 to 800, 150 to 700, preferably 200 to 600 and more preferably 200 to 500 such as 200 to 400. As is demonstrated by the examples, a stabilization composition comprising such a poly(oxyethylene) polymer, which preferably is a polyethylene glycol, are effective stabilizers when being used in combination with one or more further stabilizing agents, such as preferably a caspase inhibitor and one or more primary, secondary or tertiary amides as described herein. The stabilization effect of these stabilizing agents is improved if the stabilization composition comprises a respective poly(oxyethylene) polymer having a molecular weight of 1500 or less, such as a low molecular weight poly(oxyethylene) polymer having a molecular weight of 1000 or less.

According to one embodiment, the stabilization composition comprises a high molecular weight poly(oxyethylene) polymer and additionally comprises at least one further poly(oxyethylene) polymer having a molecular weight that is at least 100, preferably at least 200, at least 300 or at least 400 below the molecular weight of the high molecular weight poly(oxyethylene) polymer. According to one embodiment, the difference in the molecular weight is at least 2500, at least 3500, at least 5000 or at least 7500. As described above in conjunction with the stabilization method according to the first aspect, using a combination of poly(oxyethylene) polymers that differ in their molecular weights is advantageous. Preferably, both poly(oxyethylene) polymers are polyethylene glycols such as unsubstituted polyethylene glycol. According to an advantageous embodiment, the stabilization composition comprising a high molecular weight poly(oxyethylene) polymer having a molecular weight of at least 1500 additionally comprises a low molecular weight poly(oxyethylene) polymer having a molecular weight of 1000 or less. Details are described above in conjunction with the stabilization method according to the first aspect and it is referred to the respective disclosure which also applies here. The low molecular weight poly(oxyethylene) polymer is preferably a polyethylene glycol, such as a unsubstituted polyethylene glycol. The molecular weight of the low molecular weight poly(oxyethylene) polymer may lie in a range selected from 100 to 1000, 150 to 800 and preferably lies in the range of 200 to 600.

According to one embodiment, the stabilization composition comprising a poly(oxyethylene) polymer, which preferably is a high molecular weight poly(oxyethylene) polymer having a molecular weight of at least 1500, additionally comprises one or more primary, secondary or tertiary amides. The advantages of additionally using one or more of such amides in combination with a poly(oxyethylene) polymer and suitable and preferred embodiments were described in detail above in conjunction with the stabilization method according to the first aspect and it is referred to the above disclosure which also applies here. According to one embodiment, the at least one primary, secondary or tertiary amide comprised in the stabilization composition is a compound according to formula 1 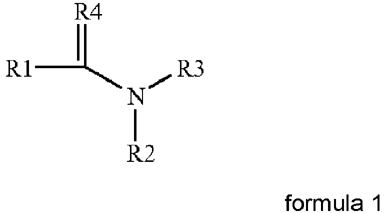 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue. Preferably, the amide is a carboxylic acid amide so that R4 is oxygen. Preferred embodiments were described above in conjunction with the stabilization method and it is referred to the above disclosure which also applies here. Preferably, a compound according to formula 1 is used which is not classified as toxic agent. Preferably, said stabilization composition comprising a poly(oxyethylene) polymer, which preferably is a high molecular weight poly(oxyethylene) polymer and comprising a compound according to formula 1 additionally comprises a caspase inhibitor.

The compound according to formula 1 may be a carboxylic acid amide selected from primary carboxylic acid amides and secondary carboxylic acid amides. According to one embodiment, the composition comprises a primary carboxylic acid amide selected from the group consisting of formamide, acetamide, propanamide and butanamide. Preferably, the carboxylic acid is selected from butanamide and formamide. More preferred, it is butanamide, as this agent is particularly effective for stabilizing the extracellular nucleic acid population.

According to one embodiment, the at least one compound according to formula 1 is a N,N-dialkyl-carboxylic acid amide. According to one embodiment, the compound according to formula 1 is a N,N-dialkylpropanamide, preferably N,N-dimethlypropanamide. N,N-dimethylpropanamide is not classified as toxic agent. Furthermore, N,N-dimethylpropanamide has the advantageous effect that it is additionally capable of stabilizing intracellular nucleic acids, and in particular may stabilize transcript profiles if used in an appropriate concentration.

According to one embodiment, the stabilization composition comprises butanamide and/or an N,N-dialkylpropanamide, wherein said N,N-dialkylpropanamide preferably is N,N-dimethylpropanamide. As is demonstrated by the examples, both amides alone and in combination significantly improve the observed stabilization effect.

According to one embodiment, the stabilization composition comprising a poly(oxyethylene) polymer additionally comprises a caspase inhibitor. The advantages of using a caspase inhibitor in combination and suitable and preferred embodiments of the caspase inhibitor were described in detail above in conjunction with the stabilization method according to the first aspect and it is referred to the above disclosure which also applies here. Preferably, the caspase inhibitor is a pancaspase inhibitor. Preferably, the caspase inhibitor is a modified caspase specific peptide, preferably modified at the C-terminus with an O-phenoxy group such as Q-VD-OPh. According to an advantageous embodiment, a high molecular weight poly(oxyethylene) polymer having a molecular weight of at least 1500 is used as poly(oxyethylene) polymer. Preferably, polyethylene glycol is used. Preferably, unsubstituted polyethylene glycol is used.

According to one embodiment, the stabilization composition comprises a poly(oxyethylene) polymer, which preferably is a high molecular weight poly(oxyethylene) polymer, and at least one anticoagulant. This embodiment is particularly suitable for stabilizing a blood sample or a cell-containing sample derived from blood. According to one embodiment, the stabilization composition comprises a poly(oxyethylene) polymer, which preferably is a high molecular weight poly(oxyethylene) polymer, and a chelating agent. Suitable chelating agents which also function as anticoagulant as well as suitable concentrations for stabilization were described above in conjunction with the method according to the present invention and it is referred to the above disclosure which also applies here. Preferably, EDTA is used as chelating agent.

The stabilization composition may also comprise further additives as described above in conjunction with the stabilization method.

For stabilizing blood, the stabilization composition preferably comprises a poly(oxyethylene) polymer, which preferably is a high molecular weight poly(oxyethylene) polymer, at least one caspase inhibitor and an anticoagulant and optionally at least one primary, secondary or tertiary amide as described above. The poly(oxyethylene) polymer is preferably polyethylene glycol. As described, said amide is preferably a compound according to formula 1. The use of butanamide and/or a N,N-dialkylpropanamide, preferably N,N-dimethlypropanamide is preferred. According to one embodiment, said composition comprises a high molecular weight poly(oxyethylene) polymer, which preferably is a polyethylene glycol, and additionally comprises a low molecular weight poly(oxyethylene) polymer, which preferably is a polyethylene glycol. Preferred molecular weights were described above and it is referred to the respective disclosure.

According to one embodiment, the stabilization composition comprises
a) at least one high molecular weight poly(oxyethylene) polymer having a molecular weight of at least 1500, preferably in a range of 1500 to 50000, 2000 to 40000, 2500 to 30000, 2500 to 25000, more preferred 3000 to 20000, 3500 to 15000 or 4500 to 10000;
b) one or more compounds according to formula 1;
c) at least one caspase inhibitor, preferably a pancaspase inhibitor, more preferred Q-VD-OPh;
d) at least one further poly(oxyethylene) polymer having a molecular weight that is at least 100, preferably at least 200, at least 300 or at least 400 below the molecular weight of the high molecular weight poly(oxyethylene) polymer used and wherein said further poly(oxyethylene) polymer preferably is a low molecular weight poly(oxyethylene) having a molecular weight of 1000 or less, preferably having a molecular weight in a range of 200 to 800 or 200 to 600;
e) optionally an anticoagulant and/ or a chelating agent, more preferably EDTA.

According to one embodiment, the stabilization composition comprises
a) at least one high molecular weight poly(oxyethylene) polymer having a molecular weight that lies in the range of 2000 to 40000, 2500 to 30000, 2500 to 25000, 3000 to 20000 or 3500 to 15000;
b) one or more compounds according to formula 1, preferably butanamide and/or N,N-dimethlypropanamide;
c) at least one caspase inhibitor;
d) at least one low molecular weight poly(oxyethylene) polymer having a molecular weight that lies in a range of 100 to 1000, 150 to 800 or 200 to 600;
e) an anticoagulant and/or a chelating agent, preferably EDTA.

According to one embodiment, the stabilization composition comprises
a) at least one high molecular weight poly(oxyethylene) polymer having a molecular weight that lies in the range of 4500 to 10000;
b) one or more compounds according to formula 1, preferably butanamide and/or N,N-dimethlypropanamide;
c) at least one caspase inhibitor;
d) at least one low molecular weight poly(oxyethylene) polymer having a molecular weight that lies in a range of 100 to 800, preferably 200 to 600;
e) an anticoagulant and/or a chelating agent, preferably EDTA.

Suitable and preferred embodiments of the high and low molecular weight poly(oxyethylene) polymer, the caspase inhibitor, the compound according to formula 1 and anticoagulants as well as chelating agents were described in detail above in conjunction with the stabilization method and it is referred to the above disclosure which also applies here. Preferably, the anticoagulant is a chelating agent, more preferably EDTA.

Suitable and preferred concentrations of the individual agents that can be used for stabilization in the stabilization mixture comprising the stabilizing composition and the cell-containing biological sample were described above and also apply here. The skilled person can chose appropriate concentrations of said agents in the stabilization composition to achieve said concentrations in the mixture comprising the cell-containing sample when the intended amount of the stabilization composition is mixed with the intended amount of cell-containing sample to be stabilized. It is referred to the above disclosure which also applies here with respect to the stabilization composition.

According to one embodiment, the stabilization composition is a liquid. Subsequently, concentrations of the individual agents are indicated, if present in the stabilization composition, that are particularly preferred for the stabilisation of blood samples. E.g. a liquid stabilisation composition of 0.5ml to 2.5ml, 0.5ml to 2ml, preferably 1ml to 2ml or 1ml to 1.5ml can be used. Such stabilization composition comprising the stabilizing agents in the concentrations indicated below, can be used for stabilizing e.g. 10ml blood.

According to one embodiment, said liquid stabilization composition comprises a high molecular weight poly(oxyethylene) polymer which preferably is a polyethylene glycol in a concentration selected from 0.4% to 35% (w/v), 0.8% to 25% (w/v), 1.5% to 20% (w/v), 2.5% to 17.5% (w/v), 3% to 15% (w/v), 4% to 10% (w/v) and 3% to 5% (w/v). Suitable concentrations can be determined by the skilled person and may *inter alia* depend on whether the high molecular weight poly(oxyethylene) glycol is used as alone or in combination with a further poly(oxyethylene) polymer such as a low poly(oxyethylene) polymer and the amount, e.g. the volume, of the stabilization composition used to stabilize a certain amount of cell-containing sample. Examples of concentration ranges suitable when using a high molecular weight poly(oxyethylene) polymer alone include but are not limited to concentrations selected from 2.2% to 33.0% (w/v), 4.4% to 22.0 (w/v)%, 6.6% to 16.5% (w/v) and 8.8% to 13.2% (w/v). Examples of concentration ranges suitable when using a high molecular weight poly(oxyethylene) polymer in combination with a low molecular weight poly(oxyethylene) polymer include but are not limited to concentrations selected from 0.4% to 30.7%, 0.8% to 15.3%, 1% to 10%, 1.5% to 7.7%, 2.5% to 6%, 3.1% to 5.4% and 3% to 4%.

In a specific embodiment, the liquid stabilization composition comprises 5 mg to 500 mg, in particular 10 mg to 250 mg, 25 mg to 150 mg, or 40 mg to 100 mg of the high molecular weight poly(oxyethylene) polymer which preferably is a polyethylene glycol. In particular, the liquid stabilization composition may comprise 0,5 µmol to 50 µmol, in particular 1 µmol to 25 µmol, 2 µmol to 20 µmol, or 3 µmol to 10 µmol of the high molecular weight poly(oxyethylene) polymer. Such liquid stabilization composition can be filled e.g. in a collection device and is e.g. suitable for stabilizing a sample unit.

According to one embodiment, said liquid stabilization composition comprises a low molecular weight poly(oxyethylene) polymer, which preferably is a polyethylene glycol in a concentration selected from 0.8% to 92.0%, 3.8% to 76.7%, 11.5% to 53.7%, 19.2% to 38.3%, 20% to 30% and 20% to 27.5%. The aforementioned concentrations refer to (w/v) or (v/v) depending on whether the low molecular weight poly(oxyethylene) polymer is a liquid or not. As is demonstrated in the examples, low molecular weight poly(oxyethylene) polymers can efficiently support the stabilization of cell-containing samples, in particular when being used in combination with one or more further stabilizing agents as described herein.

In a specific embodiment, the liquid stabilization composition comprises 40 µl to 4000 µl, in particular 100 µl to 2000 µl, 150 µl to 1500 µl, 200µl to 1000µl or 250 µl to 750 µl of the low molecular weight poly(oxyethylene) polymer. In particular, the liquid stabilization composition may comprise 0.2 mmol to 15 mmol, in particular 0.5 mmol to 10 mmol, 0.75 mmol to 5 mmol, 1 mmol to 3 mmol, or 1.2 mmol to 2 mmol of the low molecular weight poly(oxyethylene) polymer. Such liquid stabilization composition can be filled e.g. in a collection device and is e.g. suitable for stabilizing a sample unit.

According to one embodiment, said liquid stabilization composition comprises one or more primary, secondary or tertiary amides in a concentration selected from 0.4% to 38.3%, 0.8% to 23.0%, 2.3% to 11.5%, 3.8% to 9.2%, 5% to 15% and 7.5% to 12.5%. The aforementioned concentrations refer to (w/v) or (v/v) depending on whether the primary, secondary or tertiary amide is a liquid or not. As described above, it is preferred that the stabilizing composition additionally comprises one or more primary, secondary or tertiary amides and suitable and preferred examples were described above.

In a specific embodiment, the liquid stabilization composition comprises 10 µl to 2000 µl, in particular 50 µl to 1000 µl, 100 µl to 750 µl, or 125 µl to 500 or 150 to 250 µl of the primary, secondary or tertiary amide. In particular, the liquid stabilization composition may comprise 0,2 mmol to 15 mmol, in particular 0.5 mmol to 10 mmol, 0.75 mmol to 5 mmol, 1 mmol to 3 mmol, or 1.2 mmol to 2 mmol of the primary, secondary or tertiary amide. Such liquid stabilization composition can be filled e.g. in a collection device and is e.g. suitable for stabilizing a sample unit.

According to one embodiment, said liquid stabilization composition comprises a caspase inhibitor in a concentration selected from 0.1 µM to 220 µM, 0.8 µM to 115.0 µM, 7.7 µM to 76.7 µM and 23.0 µM to 50 µM. In a specific embodiment, the liquid stabilization composition comprises 1 nmol to 1000 nmol, in particular 5 nmol to 500 nmol, 10 nmol to 200 nmol or 25 nmol to 100 nmol of the caspase inhibitor. Such liquid stabilization composition can be filled e.g. in a collection device and is e.g. suitable for stabilizing a sample unit.

According to one embodiment, said liquid composition comprises a chelating agent, preferably EDTA such as K₂EDTA in a concentration selected from 9.5mM to 1100mM, 20mM to 750mM, 50mM to 600mM, 75mM to 550mM, 100mM to 500mM, 125mM to 450mM, 130mM to 300mM and 140mM to 250mM. In a specific embodiment, the liquid stabilization composition comprises 10 nmol to 3000 nmol, in particular 50 nmol to 1500 nmol, 100 nmol to 1000 nmol or 150 nmol to 500 nmol of the chelating agent. Such liquid stabilization composition can be filled e.g. in a collection device and is e.g. suitable for stabilizing a sample unit.

As described above, said liquid stabilization composition comprises a poly(oxyethylene) polymer which preferably is a high molecular weight poly(oxyethylene) polymer, and preferably one or more primary, secondary or tertiary amides, a caspase inhibitor and the chelating agent. According to one embodiment, said liquid composition comprises a high molecular weight poly(oxyethylene) polymer and comprises additionally a low molecular weight poly(oxyethylene) glycol.

According to one alternative, the composition comprises mono-ethylene glycol (1,2-ethanediol) as stabilizing agent. Mono-ethylene glycol may be used in the concentrations described above for the low molecular weight poly(oxyethylene) polymer. It is referred to the repective disclosure which also applies here. In particular, the composition may comprise mono-ethylene glycol and any one or more of the other stabilizing agents described above in conjunction with the composition. According to one embodiment, the composition acomprises additionally at least one caspase inhibitor and/or at least one primary, secondary or tertiary amide, in particular a compound according to formula 1 as described above. Preferably, the composition comprises both. The composition comprising mono-ethylene glycol may also comprise an anticoagulant, preferably a chelating agent, and/or a poly(oxyethylene) polymer, such as a high and/or low molecular weight poly(oxyethylene) polymer, as described above. Suitable embodiments and concentration ranges for the respective stabilizing agents are described above and also apply to the embodiment, wherein the composition comprises mono-ethylene glycol as stabilizing agent.

The stabilizing composition provided by the present invention stabilizes the cell-containing biological sample and thus, does not induce the lysis and/or disruption of nucleated cells and preferably also anucleated cells, contained in the sample. Therefore, the stabilization composition does not comprise additives in a concentration wherein said additives would induce or promote cell lysis of respective cells and preferably cells in general. The stabilizing composition reduces the damage of cells comprised in the sample as can be e.g. determined by the assay methods described in the example section. In particular, the stabilization composition described herein is capable of reducing the release of genomic DNA from cells contained in the cell-containing biological sample into the cell-free portion of the sample. Furthermore, in particular when additionally comprising a caspase inhibitor what is preferred, the stabilization composition may be capable of reducing the degradation of nucleic acids, in particular genomic DNA, present in the stabilized sample. As described, the stabilization composition is capable of reducing or preventing the contamination of the extracellular DNA population comprised in the biological sample with genomic DNA originating from cells contained in the stabilized sample. Preferably, it is capable of reducing or preventing the contamination of the extracellular nucleic acid population comprised in the biological sample with intracellular nucleic acids, in particular DNA and RNA, originating from cells contained in the stabilized sample. Preferably, the stabilization composition does not comprise a crosslinking agent that induces protein-DNA and/or protein-protein crosslinks. In particular, the stabilization composition does not comprise formaldehyde, formaline, paraformaldehyde or a formaldehyde releaser or similar crosslinking agents. Preferably, the stabilization composition does not comprise agents that are classified as toxic agents according to GHS. Preferably, the stabilization composition of the invention is capable of stabilizing the extracellular nucleic acid population comprised in the cell-containing biological sample without refrigeration, preferably at room temperature, for a time period selected from at least two days, at least three days, at least four days, at least five days and/or at least six days. In particular, one or more, preferably all of the above-described stabilizing effects are achieved during the defined stabilization periods.

According to one embodiment, the stabilizing composition is for the stabilization of blood and consists essentially of the one or more poly(oxyethylene) polymers, one or more primary, secondary or tertiary amides, the at least one caspase inhibitor, and an anticoagulant, which preferably is a chelating agent such as EDTA and optionally, a solvent and/or buffering agent. As described, preferably water is used as solvent as it reduces hemolysis during the storage period. The same applies mutatis mutandis to the embodiment wherein mono-ethylene glycol is used as stabilizing agent.

The stabilization composition may be provided in a solid form, a semi-liquid form or as liquid. A solid composition is e.g. a suitable option if the cell-containing biological sample to be stabilized contains liquid to dissolve the solid (such as for example cell-containing body fluids, cells in medium, urine) or if liquid, e.g. water is added thereto to dissolve the solid composition. As is demonstrated by the examples, the stabilising composition can be used in solid-form. The advantage of using a solid stabilizing composition is that solids are usually chemically more stable. However, also a liquid stabilization composition may be used. Liquid compositions have the advantage that the mixture with the sample to be stabilised can be quickly achieved, thereby basically providing an immediate stabilizing effect as soon as the sample comes into contact with the liquid stabilizing composition. Preferably, stabilizing agent(s) present in the liquid stabilizing composition remain stable in solution and require no pre-treatment-such as for example the dissolving of precipitates of limited solubility-by the user because pre-treatments of this kind pose a risk of variations in the stabilizing efficiency. As is demonstrated by the examples, a stabilization composition comprising water is particularly advantageous when stabilizing blood samples, as hemolysis is reduced during the storage period.

The present invention also provides a mixture comprising the stabilizing composition according to the third aspect of the invention mixed with a cell-containing biological sample. Suitable and preferred examples of cell-containing biological samples as well as suitable concentrations of the stabilizing agent(s) when mixed with the cell-containing biological sample are described above *inter alia* in conjunction with the stabilizing method according to the invention. It is referred to the above disclosure which also applies here. As described, preferably the cell-containing sample is a blood sample.

According to one embodiment, the stabilizing composition of the invention is pre-filled in a sample collection device so that the sample is immediately stabilized upon or during collection. According to one embodiment, the stabilizing composition is contacted with the cell-containing biological sample in a volumetric ratio selected from 10:1 to 1:20, 5:1 to 1:15, 1:1 to 1:10, 1:10 to 1:5 and 1:7 to 1:5, in particular about 1:6. It is a particular advantage of the stabilizing composition of the present invention that stabilization of a large sample volume can be achieved with a small volume of the stabilizing composition. Therefore, preferably, the ratio of stabilizing composition to sample lies in a range from 1:10 to 1:5, in particular 1:7 to 1:5, such as e.g. about 1:6.

The stabilizing composition according to the third aspect of the present invention can be used to stabilize the extracellular nucleic acid population comprised in a cell-containing sample, such as preferably a blood sample. Furthermore, as described above, the stabilizing composition stabilizes the contained cells and thereby *inter alia* reduce the release of genomic DNA and other intracellular nucleic acids from cells comprised in the cell-containing biological sample. Thereby, a contamination of the extracellular nucleic acid population with genomic DNA and other intracellular nucleic acids is reduced.

The stabilizing composition of the present invention may also be incorporated into a sample collection device, in particular a blood collection assembly, such as a blood collection container thereby providing for a new and useful version of such a device. Such devices typically include a container having an open and a closed end. The container is preferably a blood collection tube. The container type also depends on the sample to be collected, other suitable formats are described below.

### D. USE

According to fourth aspect, the present invention is directed to the use of a poly(oxyethylene) polymer, which preferably is a polyethylene glycol, and/or the use of mono-ethylene glycol for stabilizing a cell-containing biological sample and in particular the extracellular nucleic acid population comprised in a cell-containing biological sample. In particular, the stabilizing composition according to the third aspect can be used for said purpose, e.g. in the method according to the first aspect of the present invention. Details of said method were described above and it is referred to the above disclosure which also applies here. Preferably, as described above, the composition comprises an anticoagulant if the cell-containing biological sample is blood what is a preferred embodiment.

### E. COLLECTION DEVICE

According to a fifth aspect, the present invention provides a collection device for collecting a cell-containing biological sample, wherein the collection device comprises
i) a poly(oxyethylene) polymer as stabilizing agent or
ii) mono-ethylene glycol as stabilizing agent
and one or more, preferably two or more, further additives selected from the group consisting of
- one or more primary, secondary or tertiary amides;
- a caspase inhibitor;
- an anticoagulant and/or a chelating agent.

Preferably, the collection device according to the fifth aspect comprises a poly(oxyethylene) polymer, which preferably is a high molecular weight poly(oxyethylene) polymer having a molecular weight of at least 1500, as stabilizing agent and furthermore comprises one or more, preferably two or more further additives selected from the group consisting of
- at least one further poly(oxyethylene) polymer having a molecular weight that is at least 100, preferably at least 200, at least 300 or at least 400 below the molecular weight of the first poly(oxyethylene) polymer which preferably is a high molecular weight poly(oxyethylene) polymer, wherein said further poly(oxyethylene) polymer preferably is a low molecular weight poly(oxyethylene) polymer having a molecular weight of 1000 or less;
- one or more primary, secondary or tertiary amides;
- a caspase inhibitor;
- an anticoagulant and/or a chelating agent.

Providing a respective collection device, e.g. a sample collection tube, has the advantage that the sample is quickly stabilized when the sample is collected in said collection device. The collection device for collecting a cell-containing biological sample, preferably a blood sample, may comprise a stabilizing composition according to the third aspect of the present invention. Details with respect to the the use of a poly(oxyethylene) polymer and optionally the one or more further additives for stabilization as well as the stabilizing composition were described above in conjunction with the other aspects, it is referred to the above disclosure which also applies here. The same applies with respect to the alternative wherein mono-ethylene glycol is used as stabilizing agent, details of that embodiment were described already above. Mono-ethylene glycol can also be used in combination with the at least one poly(oxyethylene)polymer. The collection device is subsequently also referred to as container.

According to one embodiment, the collection device comprises a poly(oxyethylene) polymer, preferably a polyethylene glycol, one or more primary, secondary or tertiary amides, preferably one or more compounds according to formula 1 and a caspase inhibitor. Furthermore, if the collection container is for collection blood, it preferably also comprises an anticoagulant, which preferably is a chelating agent.

According to one embodiment, a collection device for receiving and collecting a cell-containing biological sample is provided which comprises:
a) at least one high molecular weight poly(oxyethylene) polymer having a molecular weight of at least 1500, at least 2000, preferably at least 3000, wherein the molecular weight preferably lies in a range selected from 2000 to 40000, 2500 to 30000, 2500 to 25000, 3000 to 20000, 3500 to 15000, 4000 to 10000, 4500 to 8000 and 5000 to 7000;
b) one or more primary, secondary or tertiary amides;
c) at least one caspase inhibitor, preferably a pancaspase inhibitor, more preferred Q-VD-OPh;
d) optionally at least one low molecular weight poly(oxyethylene) polymer having a molecular weight of 1000 or less, wherein the molecular weight preferably lies in a range selected from 100 to 800, 150 to 700, 150 to 600, 200 to 500 and 200 to 400;
e) optionally an anticoagulant and/or chelating agent, more preferably EDTA.

In this embodiment, the high molecular weight poly(oxethylene) polymer (component a)) which preferably is a polyethylene glycol, may be comprised in the collection device in a concentration so that when the cell-containing biological sample is collected into said device, the concentration of the high molecular weight poly(oxyethylene) polymer in the resulting mixture lies in a range selected from 0.05% to 4% (w/v), 0.1% to 3% (w/v), 0.2% to 2.5% (w/v), 0.25% to 2% (w/v), 0.3% to 1.75% (w/v) and 0.35% to 1.5% (w/v). According to one embodiment, the high molecular weight poly(oxyethylene) polymer is comprised in the collection device in a concentration so that when the cell-containing biological sample is collected into said device, the concentration of the high molecular weight poly(oxyethylene) polymer in the resulting mixture lies in a range as 0.25% to 1.5% (w/v), 0.3% to 1.25% (w/v), 0.35% to 1% (w/v) and 0.4% to 0.75% (w/v). These concentrations ranges are particularly suitable for the stabilization of blood and the advantages were discussed above in conjunction with the stabilization method.

In this embodiment, the one or more primary, secondary or tertiary amide (component b)) is comprised in the collection device in a concentration so that when the cell-containing biological sample is collected into said device, the concentration of the amide (or combination of amides) in the resulting mixture lies in a range of 0.25% to 5%, 0.3% to 4%, 0.4% to 3%, 0.5% to 2% or 0.75% to 1.5%. The at least one amide preferably is a compound according to formula 1 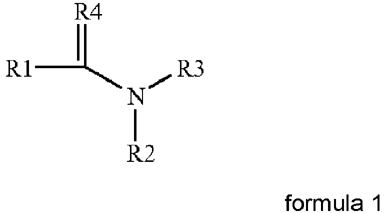 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue. Preferably, the amide is a carboxylic acid amide so that R4 is oxygen. Preferred embodiments of the compound according to formula 1 are described above in conjunction with the stabilization method and it is referred to the above disclosure which also applies here. Preferably, the collection device comprises butanamide and/or a an N,N-dialkylpropanamide which preferably is N,N-dimethylproanamide as compound according to formula 1.

In this embodiment, the at least one caspase inhibitor (component c)) is advantageously comprised in the collection device in a concentration so that when the cell-containing biological sample is collected into said device, the concentration of the caspase inhibitor in the resulting mixture lies in a range of 0.1µM to 20µM, more preferred 0.5µM to 10µM, more preferred 1µM to 10µM, more preferred 3µM to 7.5µM or 3µM to 5µM. As is shown by the examples, a stabilizing composition comprising a poly(oxyethylene) polymer and a caspase inhibitor is very effective in stabilizing a cell-containiner biological sample, in particular a whole blood sample. Preferably, one or more primary, secondary or tertiary amides as described above are additionally used.

If in this embodiment the collection device additionally comprises a low molecular weight poly(oxyethylene) polymer having a molecular weight of 1000 or less as component d), it is advantageously comprised in the collection device in a concentration so that when the cell-containing biological sample is collected into said device, the concentration of low molecular weight poly(oxyethylene) polymer in the resulting mixture lies in a range selected from 0.5% to 10%, 1.5% to 9%, 2% to 8% and 2.5% to 7% and 3% to 6%. The percentage values refer to (w/v) in case the poly(oxyethylene) polymer is a solid and to (v/v) in case the poly(oxyethylene) polymer is a liquid. Preferably, the poly(oxyethylene) polymer is a polyethylene glycol. The advantages associated with this embodiment, wherein a high molecular weight poly(oxyethylene) polymer is used in combination with a low molecular weight poly(oxyethylene) polymer was described in detail above. In an advantageous embodiment, the collection container comprises the high molecular weight poly(oxyethylene) polymer (component a)) and the low molecular weight poly(oxyethylene) polymer (component d)) in a concentration so that when the cell-containing biological sample is collected into said device, the concentration of the high molecular weight poly(oxyethylene) polymer in the resulting mixture lies for the high molecular weight poly(oxyethylene glycol in a range selected from 0.2% to 1.5% (w/v), 0.3% to 1.25% (w/v) and 0.4% to 0.75% (w/v) and for the low molecular weight poly(oxyethylene) glycol in a range selected from 1.5% to 8%, 2% to 7% and 2.5% to 6%. Preferably, the high as well as the low molecular weight poly(oxyethylene) polymer is a polyethylene glycol as described above.

According to one embodiment, the collection device additionally comprises an anticoagulant and/or a chelating agent agent. This embodiment is particularly suitable if the container is for collecting blood or a sample derived from blood such as plasma or serum. The anticoagulant is comprised in a concentration wherein it is capable of preventing the coagulation of blood. Suitable anticoagulants were described above in conjunction with the method according to the first aspect and it is referred to the above disclosure which also applies here. As described, the anticoagulant is preferably a chelating agents and suitable embodiments were described in detail above and it is referred to the respective disclosure. According to one embodiment, the container comprises a chelating agent, preferably EDTA, in a concentration so that when the cell-containing biological sample is collected into the container, the concentration of the chelating agent in the resulting mixture lies in a concentration range selected from 0.5 to 40mg/ml; 1 to 30 mg/ml, 1.6 to 25 mg/ml, 5 to 20 mg/ml and 7.5 to 17.5 mg/ml.

According to one embodiment, the collection device comprises mono-ethylene glycol as stabilizing agent. Mono-ethylene glycol may be used in the concentrations described above for the low molecular weight poly(oxyethylene) polymer. It is referred to the repective disclosure which also applies here. In particular, the collection device may comprise mono-ethylene glycol and any one or more of the other stabilizing agents described herein, preferably at least one caspase inhibitor and/or at least one primary, secondary or tertiary amide, which preferably is a compound according to formula 1 as defined above. The collection device may also comprise mono-ethylene glycol and at least one poly(oxyethylene) polymer. Suitable embodiments and concentration ranges for the respective stabilizing agents are described above and also apply to the embodiment, wherein mono-ethylene glycol is comprised in the collection container in order to stabilize or support the stabilization of a cell-containing sample and the exracellular nucleic acid population comprised therein.

The stabilizing composition and/or the individual compounds used for stabilization comprised in the collection container can be provided in a liquid; semi-liquid or in a dry form. As discussed above, the poly(oxyethylene) polymer and the further additives used for stabilization may be provided in form of a stabilizing composition. The compounds used for stabilization may also be provided as separate entities in the container and may also be provided in different forms in the container. E.g. one component may be provided in dry form while the other compound may be provided as liquid. Other combinations are also feasible. Suitable formulation and manufacturing options are known to the skilled person.

For stabilizing whole blood it is preferred to encompass an anticoagulant such as EDTA into the container, e.g. in the stabilizing compositon. A dry form is e.g. a suitable option if the biological sample to be stabilized contains liquid to dissolve the solid (such as for example cell-containing body fluids, cells in medium, urine) or if liquid, e.g. water or other solvent is added thereto to dissolve the solid. The advantage of using a solid stabilizing composition is that solids are usually chemically more stable than liquids. According to one embodiment, the inner wall of the container is treated/covered with a stabilizing composition according to the present invention or with individual components thereof, such as e.g. the anticoagulant. Said composition or component can be applied to the inner walls using e.g. a spray-dry-method. Liquid removal techniques can be performed on the stabilizing composition in order to obtain a substantially solid state protective composition. Liquid removal conditions may be such that they result in removal of at least about 50% by weight, at least about 75% by weight, or at least about 85% by weight of the original amount of the dispensed liquid stabilizing composition. Liquid removal conditions may be such that they result in removal of sufficient liquid so that the resulting composition is in the form of a film, gel or other substantially solid or highly viscous layer. For example it may result in a substantially immobile coating (preferably a coating that can be re-dissolved or otherwise dispersed upon contact with the cell-containing sample which preferably is a blood product sample). It is possible that lyophilization or other techniques may be employed for realizing a substantially solid form of the protective agent (e.g., in the form of one or more pellet). Thus, liquid removal conditions may be such that they result in a material that upon contact with the sample under consideration (e.g., a whole blood sample) the protective agent will disperse in the sample, and substantially preserve components (e.g., extracellular nucleic acids) in the sample. Liquid removal conditions may be such that they result in a remaining composition that is substantially free of crystallinity, has a viscosity that is sufficiently high that the remaining composition is substantially immobile at ambient temperature; or both.

According to one embodiment, a liquid composition is used. This has advantages for specific samples such as e.g. blood samples. Liquid compositions have the advantage that the mixture with the cell-containing biological sample to be stabilised can be quickly achieved, thereby basically providing an immediate stabilizing effect as soon as the sample comes into contact with the liquid stabilizing composition. Furthermore, liquid compositions are advantageous if larger amounts of stabilization compositions are used which accordingly, can not or are difficult to spray-dry or if the composition hampers providing a dry composition. Preferably, the stabilizing agents present in the liquid stabilizing composition remain stable in solution and require no pre-treatment - such as for example the dissolving of precipitates of limited solubility - by the user because pre-treatments of this kind pose a risk of variations in the stabilizing efficiency. For stabilizing blood, according to one embodiment, all compounds are present in the stabilizing composition. As discussed above, in case of blood it is advantageous to use a stabilization composition comprising a sufficient amount of water to reduce hemolysis during storage of the stabilized sample.

The stabilizing composition is comprised in the container in an amount effective to provide the stabilization of the amount of sample to be collected in said container. According to one embodiment, the liquid stabilizing composition is contacted with the biological sample in a volumetric ratio selected from 10:1 to 1:20, 5:1 to 1:15, 1:1 to 1:10, 1:10 to 1:5 and 1:7 to 1:5, in particular about 1:6. It is a partiuclar advantage of the stabilizing composition of the present invention that stabilization of a large sample volume can be achieved with a small volume of the stabilizing composition. Therefore, preferably, the ratio of stabilizing composition to sample lies in a range from 1:10 to 1:5, in particular 1:7 to 1:5, such as about 1:6.

According to one embodiment, the collection device is evacuated. The evacuation is preferably effective for drawing a specific volume of a fluid cell-containing biological sample into the interior. Thereby, it is ensured that the correct amount of sample is contacted with the pre-filled amount of the stabilizing composition comprised in the container, and accordingly, that the stabilization is efficient. According to one embodiment, the container comprises a tube having an open end sealed by a septum. E.g. the container is pre-filled with a defined amount of the stabilizing composition either in solid or liquid form and is provided with a defined vacuum and sealed with a septum. The septum is constructed such that it is compatible with the standard sampling accessories (e.g. cannula, etc.). When contacted with e.g. the canula, a sample amount that is predetermined by the vacuum is collected in the container. A respective embodiment is in particular advantageous for collecting blood. A suitable container is e.g. disclosed in US 6,776,959.

The container according to the present invention can be made of glass, plastic or other suitable materials. Plastic materials can be oxygen impermeable materials or may contain an oxygen impermeable layer. Alternatively, the container can be made of air-permeable plastic material. The container according to the present invention preferably is made of a transparent material. Examples of suitable transparent thermoplastic materials include polycarbonates, polyethylene, polypropylene and polyethyleneterephthalate. The container may have a suitable dimension selected according to the required volume of the biological sample being collected. As described above, preferably, the container is evacuated to an internal pressure below atmospheric pressure. Such an embodiment is particularly suitable for collecting body fluids such as whole blood. The pressure is preferably selected to draw a predetermined volume of a biological sample into the container. In addition to such vacuum tubes also non-vacuum tubes, mechanical separator tubes or gel-barrier tubes can be used as sample containers, in particular for the collection of blood samples. Examples of suitable containers and capping devices are disclosed in US 5,860,397 and US 2004/0043505. As container for collecting the cell-containing sample also further collection devices, for example a syringe, a urine collection device or other collection devices can be used. The type of the container may also depend on the sample type to be collected and suitable containers are also available to the skilled person.

According to one embodiment, the container has an open top, a bottom, and a sidewall extending therebetween defining a chamber, wherein the poly(oxyethylene) polymer, which preferably is a high molecular weight poly(oxyethylene) polymer and the one or more further stabilizing agents mentioned above or the stabilization composition according to the third aspect is comprised in the chamber. It may be comprised therein in liquid or solid form. According to one embodiment, it is a liquid. According to one embodiment the container is a tube, the bottom is a closed bottom, the container further comprises a closure in the open top, and the chamber is at a reduced pressure. The advantages of a reduced pressure in the chamber were described above. Preferably, the closure is capable of being pierced with a needle or cannula, and the reduced pressure is selected to draw a specified volume of a liquid sample into the chamber. According to one embodiment, the chamber is at a reduced pressure selected to draw a specified volume of a liquid sample into the chamber, and the stabilizing composition is a liquid and is disposed in the chamber such that the volumetric ratio of the stabilizing composition to the specified volume of the cell-containing sample is selected from 10:1 to 1:20, 5:1 to 1:15 and 1:1 to 1:10 and preferably is 1:10 to 1:5, more preferably 1:7 to 1:5. The associated advantages were described above.

Preferably, the container is for drawing blood from a patient. According to one embodiment, it is for drawing 10ml blood from a patient. According to one embodiment, the stabilisation composition is a liquid and the volume is 2ml or less and may lie in a range of 0.5ml to 2ml, 0.75ml to 1.75ml and 1ml to 1.5ml.

### F. METHOD FOR COLLECTING A CELL CONTAINING SAMPLE

According to a sixth aspect, a method is provided comprising the step of collecting a cell-containing biological sample from a patient directly into a chamber of a container according to the fifth aspect of the present invention. Details with respect to the container and the cell-containing biological sample were described above. It is referred to the respective disclosure. According to one embodiment, a blood sample is collected, preferably it is drawn from the patient into the container.

### G. MANUFACTURING METHOD

According to a seventh aspect, a method of manufacturing a stabilizing composition according to the third aspect of the present invention is provided, wherein the components of the stabilizing composition are mixed. Preferably, they are mixed to provide a liquid solution. As described, a stabilization composition comprising water is particularly preferred for stabilizing blood samples, because hemolysis can be effectively reduced.

This invention is not limited by the exemplary methods and materials disclosed herein, and any methods and materials similar or equivalent to those described herein can be used in the practice or testing of embodiments of this invention. Numeric ranges are inclusive of the numbers defining the range. The headings provided herein are not limitations of the various aspects or embodiments of this invention which can be read by reference to the specification as a whole.

Unless the context indicates otherwise, percentage values indicated herein refer to (w/v) in case of solid compounds contained in a liquid mixture or composition and to (v/v) in case of liquid compounds contained in a liquid mixture or composition such as e.g. the mixture resulting from contacting the stabilizing agents or the stabilizing composition containing said agents with the cell-containing sample.

As used in the subject specification, the singular forms "a", "an" and "the" include plural aspects unless the context clearly dictates otherwise. Thus, for example, reference to "a poly(oxyethylene) polymer" includes a single type of poly(oxyethylene) polymer, as well as two or more poly(oxyethylene) polymers. Likewise, reference to an "agent", "additive", "compound" and the like includes single entities and combinations of two or more of such entities. Reference to "the disclosure" and "the invention" and the like includes single or multiple aspects taught herein; and so forth. Aspects taught herein are encompassed by the term "invention".

The term "solution" as used herein in particular refers to a liquid composition, preferably an aqueous composition. It may be a homogenous mixture of only one phase but it is also within the scope of the present invention that a solution comprises solid additives such as e.g. precipitates, in particular of contained chemicals such as stabilizing agents..

The sizes, respectively size ranges indicated herein with reference to nucleotides (nt), refer to the chain length and thus are used in order to describe the length of single-stranded as well as double-stranded molecules. In double-stranded molecules said nucleotides are paired.

According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

### FURTHER ITEMS OF THE INVENTION

Also disclosed are the following items of the invention:
1. A method for stabilizing an extracellular nucleic acid population comprised in a cell-containing biological sample comprising contacting the cell-containing biological sample with at least one poly(oxyethylene) polymer as stabilizing agent or with mono-ethylene glycol as stabilizing agent.
2. The method according to item 1, wherein the poly(oxyethylene) polymer is polyethylene glycol.
3. The method according to item 1 or 2, wherein the poly(oxyethylene) polymer is a high molecular weight poly(oxyethylene) polymer having a molecular weight of at least 1500.
4. The method according to item 3, wherein the high molecular weight poly(oxyethylene) polymer has a molecular weight that lies in a range selected from 1500 to 50000, 2000 to 40000, 2500 to 30000, 2500 to 25000, 3000 to 20000 and 3500 to 15000.
5. The method according to item 3 or 4, wherein after the cell-containing biological sample has been contacted with the high molecular weight poly(oxyethylene) polymer and optionally further additives used for stabilization, the resulting mixture comprises the high molecular weight poly(oxyethylene) polymer in a concentration range that is selected from 0.05% to 4% (w/v), 0.1% to 3% (w/v), 0.2% to 2.5% (w/v), 0.25% to 2% (w/v), 0.3% to 1.75% (w/v) and 0.35% to 1.5% (w/v) or is selected from 0.25% to 1.5% (w/v), 0.3% to 1.25% (w/v), 0.35% to 1% (w/v) and 0.4% to 0.75% (w/v).
6. The method according to item 1 or 2, wherein the poly(oxyethylene) polymer has a molecular weight below 1500 and preferably is a low molecular weight poly(oxyethylene) polymer having a molecular weight of 1000 or less.
7. The method according to item 6, wherein the poly(oxyethylene) polymer is a low molecular weight poly(oxyethylene) polymer having a molecular weight of 1000 or less and preferably, the molecular weight lies in a range selected from 100 to 800, 150 to 700, 200 to 600 and 200 to 500.
8. The method according to item 6 or 7, wherein after the cell-containing biological sample has been contacted with the poly(oxyethylene) polymer and optionally further additives used for stabilization, the resulting mixture comprises the poly(oxyethylene) polymer, such as a low molecular weight poly(oxyethylene) polymer having a molecular weight of 1000 or less, in a concentration range that is selected from 0.5% to 10%, 1.5% to 9%, 2% to 8%, 2 to 7%, 2.5% to 7% and 3% to 6%.
9. The method according to one or more of items 3 to 8, wherein the cell-containing biological sample is contacted with a high molecular weight poly(oxyethylene) polymer having a molecular weight of at least 1500 and a low molecular weight poly(oxyethylene) having a molecular weight of 1000 or less.
10. The method according to item 9, wherein after the cell-containing biological sample has been contacted with the high molecular weight poly(oxyethylene) polymer and optionally further additives used for stabilization, the resulting mixture comprises
   - the high molecular weight poly(oxyethylene) polymer in a concentration range selected from 0.1% to 3% (w/v), 0.2% to 2.5% (w/v), 0.25% to 2% (w/v), 0.3% to 1.75% (w/v) and 0.35% to 1.5% (w/v) or selected from 0.25% to 1.5% (w/v), 0.3% to 1.25% (w/v), 0.35% to 1% (w/v) and 0.4% to 0.75% (w/v); and
   - the low molecular weight poly(oxyethylene) polymer in a concentration range selected from 0.5% to 10%, 1.5% to 9%, 1.75% to 8%, 2% to 7% and 2.5% to 6%.
11. The method according to one or more of items 1 to 10, wherein the cell-containing biological sample is blood and wherein the blood sample is additionally contacted with an anticoagulant, preferably a chelating agent.
12. The method according to item 11, wherein the blood sample is contacted with a high molecular weight poly(oxyethylene) polymer having a molecular weight that lies in a range selected from 3000 to 40000, 2500 to 25000 and 4000 to 20000, a low molecular weight poly(oxyethylene) polymer having a molecular weight that lies in a range selected from 200 to 800, 200 to 600 and 200 to 500 and an anticoagulant, and wherein after the blood sample has been contacted with the high and low molecular weight poly(oxyethylene) polymer, the anticoagulant and optionally further additives used for stabilization, the resulting mixture comprises the high molecular weight poly(oxyethylene) polymer in a concentration that lies in a range selected from 0.2% to 1.5% (w/v), 0.3% to 1.25% (w/v) and 0.4 (w/v) to 0.75% (w/v) and the low molecular weight poly(oxyethylene) polymer in a concentration that lies in the range of 2% to 7%, preferably 2.25% to 6%.
13. The method according to one or more of items 1 to 12, wherein for stabilization, the cell-containing sample is additionally contacted with one or more primary, secondary or tertiary amides and/or at least one caspase inhibitor as stabilizing agent.
14. The method according to item 13, wherein the primary, secondary or tertiary amide is a compound according to formula 1 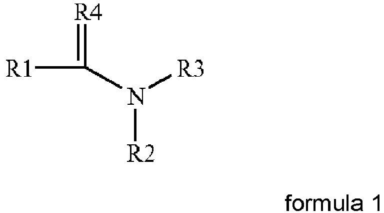 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue, preferably R4 is oxygen.
15. The method according to one or more of items 1 to 14, wherein the cell-containing sample is contacted with butanamide and/or an N,N-dialkylpropanamide, wherein said N,N-dialkylpropanamide preferably is N,N-dimethylpropanamide.
16. The method according to one or more of items 1 to 15, wherein the cell-containing biological sample, which preferably is a blood sample or a sample derived from blood such as plasma or serum, is contacted with:
   a) at least one high molecular weight poly(oxyethylene) polymer having a molecular weight of at least 1500, preferably in a range of 2000 to 40000, more preferred 2000 to 30000, 2500 to 25000 or 3000 to 20000;
   b) one or more compounds according to formula 1, preferably in a concentration so that the concentration in the mixture with the cell-containing biological sample lies in a range of 0.25% to 5%, 0.3% to 4%, 0.4% to 3%, 0.5% to 2% or 0.75% to 1.5%;
   c) at least one caspase inhibitor, preferably a pancaspase inhibitor, more preferred Q-VD-OPh, preferably in a concentration so that the concentration of the caspase inhibitor in the mixture with the cell-containing biological sample lies in a range of 0.1µM to 20µM, more preferred 0.5µM to 10µM, more preferred 1µM to 10µM, more preferred 3µM to 7.5µM;
   d) optionally at least one further poly(oxyethylene) polymer having a molecular weight that is at least 100, preferably at least 200, at least 300 or at least 400 below the molecular weight of the high molecular weight poly(oxyethylene) polymer used and wherein said further poly(oxyethylene) polymer preferably is a low molecular weight poly(oxyethylene) having a molecular weight of 1000 or less, preferably having a molecular weight in a range of 200 to 800 or 200 to 600;
   e) optionally a chelating agent, more preferably EDTA.
17. The method according to one or more of items 14 to 16, wherein for stabilization, the cell-containing sample which preferably is a blood sample, is contacted with:
   a) at least one high molecular weight poly(oxyethylene) polymer having a molecular weight of at least 3000;
   b) one or more compounds according to formula 1;
   c) at least one caspase inhibitor;
   d) optionally at least one low molecular weight poly(oxyethylene) polymer having a molecular weight of 1000 or less;
   e) optionally a chelating agent, preferably EDTA,
   wherein the release of genomic DNA from cells contained in the cell-containing sample into the cell-free portion of the sample is reduced due to the stabilization.
18. The method according to one or more of items 1 to 17, wherein the cell-containing sample is a blood sample which is contacted with:
   a) at least one high molecular weight poly(oxyethylene) polymer having a molecular weight that lies in a range of 3000 to 40000, 3000 to 30000 or 3500 to 25000;
   b) one or more compounds according to formula 1;
   c) at least one caspase inhibitor, preferably a pancaspase inhibitor, more preferred Q-VD-OPh;
   d) at least one low molecular weight poly(oxyethylene) polymer having a molecular weight of 1000 or less, preferably in a range of 100 to 800, 200 to 600 or 200 to 500;
   e) an anticoagulant which preferably is a chelating agent, preferably EDTA,
   wherein after the blood sample has been contacted with said additives and optionally further additives used for stabilization the resulting mixture comprises
   - the high molecular weight poly(oxyethylene) polymer in a concentration that lies in a range of 0.2% to 1.5% (w/v), 0.25% to 1.25% (w/v), 0.3% to 1% (w/v) or 0.4% to 0.75% (w/v),
   - the one or more compounds according to formula 1 in a concentration that lies in a range of 0.3% to 4%, preferably 0.5 to 3%, 0.5 to 2% or 0.75 to 1.5%,
   - the caspase inhibitor in a concentration that lies in a range of 1µM to 10µM, preferably 3µM to 7.5µM, and
   - the low molecular weight poly(oxyethylene) polymer in a concentration that lies in the range of 1.5% to 10%, preferably 2% to 6%.
19. The method according to one or more of items 1, 11 and 13 to 15, comprising contacting the cell-containing biological sample with mono-ethylenglycol as stabilizing agent and wherein optionally,
   - the cell-conatining sample is additionally contacted with at least one poly(oxyethylene) polymer, preferably as defined in one or more of items 1 to 10 and item 12; and/or
   - wherein the cell-containing sample is additionally contacted with one or more primary, secondary or tertiary amides, wherein preferably said amide is as defined in item 14 or 15 and/or at least one caspase inhibitor.
20. The method according to one or more of items 1 to 19, in particular 11 to 19, wherein the compounds used for stabilization are contained in an stabilization composition comprising water.
21. The method according to one or more of items 1 to 20, having one or more of the following characteristics:
   i) the stabilization does not involve the use of additives in a concentration wherein said additives would induce or promote lysis of nucleated cells;
   ii) the stabilization does not involve the use of a cross-linking agent that induces protein-nucleic acid and/or protein-protein crosslinks such as formaldehyde, formaline, paraformaldehyde or a formaldehyde releaser;
   iii) the stabilization does not involve the use of toxic agents.
22. A method for isolating extracellular nucleic acids from a stabilized cell-containing biological sample comprising the steps of
   a) stabilizing the cell-containing biological sample according to the method defined in one or more of items 1 to 21; and
   b) isolating extracellular nucleic acids.
23. A composition suitable for stabilizing a cell-containing biological sample, comprising
   i) a poly(oxyethylene) polymer as stabilizing agent or
   ii) mono-ethylene glycol as stabilizing agent
      and one or more further additives selected from the group consisting of
      - one or more primary, secondary or tertiary amides;
      - a caspase inhibitor;
      - an anticoagulant and/or a chelating agent.
24. The composition according to item 23, comprising a poly(oxyethylene) polymer which is a high molecular weight poly(oxyethylene) polymer having a molecular weight of at least 1500 as stabilizing agent and additionally comprising one or more, preferably two or more further additives selected from the group consisting of
   - at least one further poly(oxyethylene) polymer having a molecular weight that is at least 100, preferably at least 200, at least 300 or at least 400 below the molecular weight of the a high molecular weight poly(oxyethylene) polymer, wherein said further poly(oxyethylene) polymer preferably is a low molecular weight poly(oxyethylene) polymer having a molecular weight of 1000 or less;
   - one or more primary, secondary or tertiary amides;
   - a caspase inhibitor;
   - an anticoagulant and/or a chelating agent.
25. The composition according to item 23 or 24, wherein the poly(oxyethylene) polymer is a polyethylene glycol, preferably unsubstituted polyethylene glycol.
26. The composition according to one or more of items 23 to 25, comprising a caspase inhibitor and one or more primary, secondary or tertiary amides according to formula 1 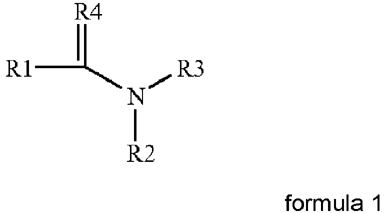 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue, preferably R4 is oxygen and wherein preferably, the composition comprises butanamide and/or an N,N-dialkylpropanamide, wherein said N,N-dialkylpropanamide preferably is N,N-dimethylpropanamide.
27. The composition according to any one of items 24 to 26, wherein the high molecular weight poly(oxyethylene) polymer has a molecular weight that lies in a range selected from 1500 to 50000, 1500 to 40000, 2000 to 30000, 2500 to 25000, 3000 to 20000, 3500 to 15000 and 4000 to 10000.
28. The composition according to one or more of items 24 to 27, wherein said further poly(oxyethylene) polymer is a low molecular weight poly(oxyethylene) polymer having a molecular weight of 1000 or less and wherein preferably, the molecular weight lies in a range selected from 100 to 1000, 150 to 800, 150 to 700, 200 to 600, 200 to 500 and 200 to 400.
29. The composition according to one or more of items 23 to 28, wherein the composition comprises a poly(oxyethylene) polymer, which preferably is a high molecular weight poly(oxyethylene) polymer, at least one caspase inhibitor and an anticoagulant and optionally comprises at least one primary, secondary or tertiary amide.
30. The composition according to item 29, comprising at least one primary, secondary or tertiary amide, wherein preferably, said amide is a compound according to formula 1, more preferred butanamide and/or a N,N-dialkylpropanamide, preferably N,N-dimethlypropanamide.
31. The composition according to one or more of items 24 to 30, comprising
   a) at least one high molecular weight poly(oxyethylene) polymer having a molecular weight of at least 1500, preferably in a range of 2000 to 40000, more preferred 2500 to 30000, 2500 to 25000 or 3000 to 20000;
   b) one or more compounds according to formula 1;
   c) at least one caspase inhibitor, preferably a pancaspase inhibitor, more preferred Q-VD-OPh;
   d) at least one further poly(oxyethylene) polymer having a molecular weight that is at least 100, preferably at least 200, at least 300 or at least 400 below the molecular weight of the high molecular weight poly(oxyethylene) polymer used and wherein said further poly(oxyethylene) polymer preferably is a low molecular weight poly(oxyethylene) having a molecular weight of 1000 or less, preferably having a molecular weight in a range of 200 to 800 or 200 to 600;
   e) optionally an anticoagulant and/ or a chelating agent, more preferably EDTA.
32. The composition according to item 23, comprising mono-ethylene glycol and optionally
   - additionally comprising at least one caspase inhibitor and at least one primary, secondary or tertiary amide, preferably, as defined in item 26; and/or
   - comprising an anticoagulant, preferably a chelating agent, and/or a poly(oxyethylene) polymer as defined in one or more of the above claims.
33. The composition according to one or more of items 23 to 32, having one or more of the following characteristics:
   a) it is capable of stabilizing cells and reducing the release of genomic DNA from cells contained in the cell-containing biological sample into the cell-free portion of the sample;
   b) it is capable of reducing the degradation of nucleic acids, in particular genomic DNA, present in the stabilized sample;
   c) it is capable of reducing or preventing the contamination of the extracellular DNA population comprised in the biological sample with genomic DNA originating from cells contained in the stabilized sample;
   d) it is capable of reducing or preventing the contamination of the extracellular nucleic acid population comprised in the biological sample with intracellular nucleic acids originating from cells contained in the stabilized sample;
   e) the stabilization composition does not comprise additives in a concentration wherein said additives would induce or promote cell lysis;
   f) the stabilization composition does not comprise a cross-linking agent that induces protein-DNA and/or protein-protein crosslinks such as formaldehyde, formaline, paraformaldehyde or a formaldehyde releaser;
   g) the stabilization composition does not comprise a toxic agent;
   h) it is capable of stabilizing extracellular nucleic acid population comprised in the cell-containing biological sample without refrigeration, preferably at room temperature, for a time period selected from at least three days, at least four, at least five or at least six days; and/or
   i) the composition additionally comprises the cell-containing sample to be stabilized which preferably is blood.

The present application claims priority of EP 14 000 990.3 and US 61/955, 200 (filed: March 18, 2014), the disclosure of both applications is herewith incorporated by reference.

### EXAMPLES

It should be understood that the following examples are for illustrative purpose only and are not to be construed as limiting this invention in any manner.

### Abbreviations used:

BA: Butanamide
ccfDNA: circulating, cell free DNA
DMPA: Dimethylpropionamide
EDTA: Ethylenediaminetetraacetic acid
PEG: Polyethylene glycol

Polyethylene glycol (PEG) was tested for its ability to stabilize a cell-containing biological sample, here a blood sample, either alone or in combination with different stabilizing agents, including a caspase inhibitor and/or different primary and/or tertiary amides. Compared to the reference samples (EDTA blood), PEG was found to be able to efficiently stabilize white blood cells in whole blood samples in a way, that it prevents the release of genomic DNA into the extracellular nucleic acid population. This stabilization effect was demonstrated for PEG of different molecular weights and when used in different concentrations. It is demonstrated that PEG can be added as a water-free powder or liquid, as pure reagent or dissolved in an aqueous solution. PEG alone has a strong stabilization effect on its own, but it also significantly improves the stabilization in combination with other stabilizing agents, including caspase inhibitors and different amides to a level that the increase of DNA released from white blood cells into plasma between day 0 (directly after blood draw) and day 6 (6 days of storage at room temperature) is reproducible reduced to 2fold or even lower. This achieved prolonged, efficient stabilization is an important advantage, as it provides a uniform, reliable stabilization method for cell-containing samples such as blood samples. Furthermore, the examples demonstrate that it is advantageous to use a combination of a high molecular weight PEG and a low molecular weight PEG as strong stabilizing effects are achieved and extracellular nucleic acids can be efficiently isolated from stabilized samples using e.g. silica column based nucleic acid isolation methods.

### I. Materials and Methods

The following procedure was followed in the examples if not indicated otherwise.

### 1. Blood collection and stabilization

Blood was drawn into 10ml spray dried EDTA tubes (BD) with 1.8mg K2EDTA per ml of whole blood. Within 30min after draw, stabilization reagents were either directly added or the blood was decanted into a new tube containing stabilization reagents. Blood and reagents were mixed by inverting the tube ten times. Stabilized blood samples were stored at room temperature standing in an upright position.

### 2. Preparation of plasma

Whole blood samples were centrifuged at ambient temperature for 15min at 3.000rpm (resolutions per minute). Clear plasma fraction was removed by pipetting and transferred into a fresh centrifuge tube. In a second round, plasma samples were centrifuged at 4°C for 10min at 16.000xg. Supernatant was transferred into a new tube and either directly used for purification of ccfDNA or stored at -20°C until use.

### 3. Purification of ccfDNA

DNA from plasma was isolated with the QIAamp circulating nucleic acid kit (QIAGEN GmbH), using the protocol for "purification of circulating nucleic acids from 1ml, 2ml, or 3ml serum or plasma". If not stated otherwise, 2ml of plasma was mixed with proteinase K and lysis buffer ACL, incubated for 30min at 60°C, mixed with buffer ACB, bound on QIAamp Mini columns (which comprise a silica solid phase for binding the nucleic acids) with the use of a QlAvac 24 Plus vaccum manifold, washed and eluted with 60µl elution buffer AVE, according to the manufactures recommendations.

### 4. Quantitative, real time PCR assay for analyzing the isolated extracellular DNA

The isolated extracellular DNA was analysed in a real time PCR assay on Abi Prism HT7900 (Life technologies) using 3µl of eluate. In a 20µl assay volume using QuantiTect Multiplex PCR Kit reagents (QIAGEN GmbH) two fragments of the human 18S rDNA gene, 66bp and 500bp, were amplified in a multiplex PCR. Cycle threshholds (Ct values) of the individual samples were translated into amount of gDNA in the eluate according to a gDNA standard curve: total quantification was achieved by comparison with a standard curve generated with human genomic DNA diluted from 3000 to 0.3 genome equivalents (1 genome equivalent equates to around 6.6pg of human genomic DNA). The gDNA amount of the storage time point (in general 6 days after blood withdrawal) was compared to the time zero gDNA level from the same donor.

**Table 1: summarizes the information of the used DNA target sequences detected by quantitative real time PCR**

| Target description | position | position | Sequence 5' - 3' | dye |
|---|---|---|---|---|
| h 18S rDNA | p12 - region of chromosome 13, 14, 15, 21, | Forward | GCCGCTAGAGGTGAAATTCTTG | 5' Cy5 -BHQ 3' |
| 66bp | | reverse | CATTCTTGGCAAATGCTTTCG | |
| amplicon | 22 | probe | ACCGGCGCAAGACGGACCAGA | |
| h18S rDNA | p12 - region of chromosome 13, 14, 15, 21, 22 | forward | GTCGCTCGCTCCTCTCCTACTT | 5' FAM -BHQ 3' |
| 500bp amplicon | | reverse | GGCTGCTGGCACCAGACTT | |
| | | probe | CTAATACATGCCGACGGGCGCTG AC | |

Quantification of the 66bp fragment was used to deflect the total amount of 18S rDNA copies in the plasma. Quantification of the 500bp was used to determine the amount of 18S rDNA copies which derived from apoptotic or mechanically lysed leucocytes from whole blood. Cell free DNA has a typically lengths of 140 - 170bp. Therefore, 500bp fragments are believed to be derived from apoptotic, lysed or otherwise destructed blood cells. The increase of copy numbers from the 500bp fragment between T0 and 6 days storage, was used as a measurement of stability efficiency. Thus, the lower the amount of released 500bp DNA, the better the performance of the stabilization method. A higher amount of released 500bp DNA indicates that lysis of white blood cells occurs and hence, that the extracellular nucleic acid population was contaminated with intracellular genomic DNA.

For the subsequent experiments with different stabilization compositions blood samples from a plurality of different individual donors were used. The average fold change of copy numbers of 66bp and 500bp fragments of the 18S rDNA gene in stabilized or unstabilized blood stored for different time points (days) at room temperature to time point 0 (day 0) after blood draw was single calculated for each individual donor sample. The average of the corresponding single calculated mean values (fold changes) was used as a measure of stabilization efficacy of the different stabilization compositions used. As blood samples underlie natural individual variations in their composition and in the amount of contained extracellular nucleic acids depending on the donor, this may result in elevated standard deviations.

### 5. Measurement of haemoglobin

Absorbance at 414nm, found to be linearly correlated with hemolytical discoloration of plasma, was measured on a spectramax photometer.

### II. Examples

### 1. Example 1

In example 1, the stabilization effect of polyethylene glycol (PEG) with different molecular weights in combination with BA, EDTA and a caspase inhibitor (Q-VD-OPh) in the absence of water was tested and compared to a combined BA, EDTA and caspase inhibitor (Q-VD-OPh) approach. Moreover, the effect on hemolysis of such stabilization mixtures in plasma samples was measured in parallel. An EDTA blood sample served as unstabilized reference control.

### Blood collection and stabilization

Samples of 10ml whole blood from eight donors, collected in 10ml spray dry K2EDTA tubes, were stabilized with mixtures of butanamide (BA) and EDTA with or without PEG from different molecular weights without addition of water. Caspase inhibitor (Q-VD-OPh) dissolved in DMSO was added by pipetting. Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

All stabilized blood samples were set up in triplicates per condition and test time point. At time point 0 (reference time point), immediately after mixing the stabilization solution and blood, plasma was generated and the circulating extracellular DNA was extracted. As a reference control, the EDTA stabilized blood sample (collected in K2 EDTA tubes without further additives) was also stored for 0 or 6 days and analysed in triplicates.

Composition of stabilization reagent mixtures (for 10ml K2EDTA whole blood each):
- unstabilized: 1.8mg/ml K2EDTA
- BA, EDTA, Q-VD-OPh: 100mg BA, 132mg K2EDTA, 10µl Q-VD-OPh (1mg dissolved in 388 µl DMSO), water ad 2ml
- PEG (600, 1000 or 3000), BA, EDTA, Q-VD-OPh: 250mg PEG (600, 1000 or 3000), 100mg BA, 132mg K2EDTA, 10µl Q-VD-OPh (1mg dissolved in 388 µl DMSO) (no water)

Thereby, the following final concentrations of the different components in the mixture are obtained after contact with blood:
- unstabilized: 1.8mg/ml K2EDTA
- BA, EDTA, Q-VD-OPh: 1% (w/v) BA, 15mg/ml K2EDTA, 5µM Q-VD-OPh
- PEG(600, 1000 or 3000), BA, EDTA, Q-VD-OPh: 2.5% (w/v) PEG (600, 1000 or 3000), 1% (w/v) BA, 15mg/ml K2EDTA, 5µM Q-VD-OPh

### Results

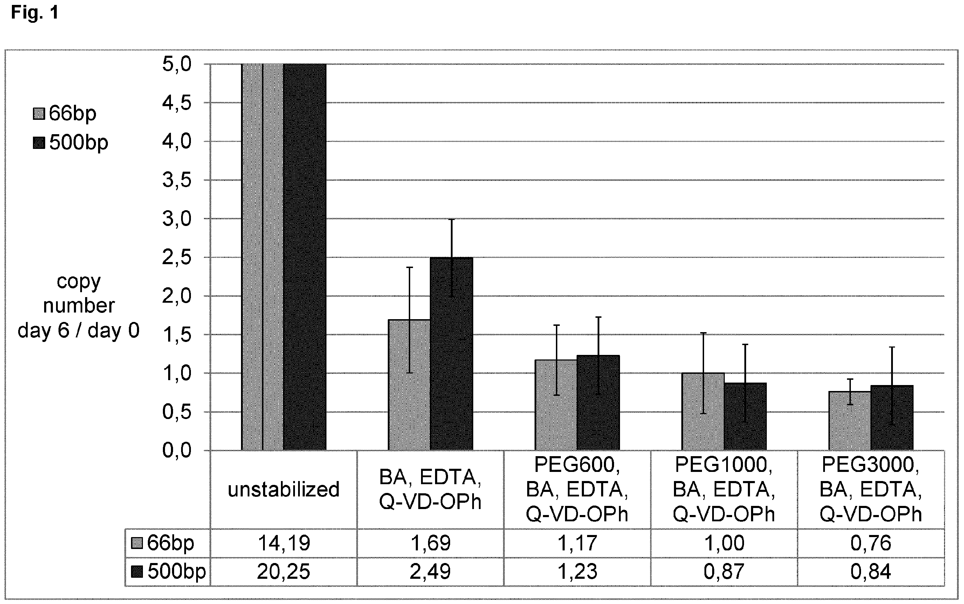

The average change of copy numbers (x fold change) of 66bp and 500bp fragments of the 18S rDNA gene in stabilized or unstabilized blood from 8 donors stored for 6 days at room temperature to time point 0 (day 0) after blood draw was single calculated for each of the eight blood donors. Fig. 1 shows the corresponding average fold change of copy numbers from 8 donors per condition. All stabilization compositions show significantly lower amounts of released genomic DNA after storage for 6 days at room temperature compared to the unstabilized control (EDTA blood) as the average fold change increases significantly less. Thus, a stabilization effect was achieved even throughout this long stabilization period of 6 days. Fig. 1 demonstrates that contacting the blood samples additionaly with polyethylene glycol significantly improved the stabilization effect achieved. Therefore, PEG of different molecular weights were highly effective in improving the stabilization effect as the average fold change increase was consistently reduced below 2-fold and in embodiments even below 1-fold. I.e. the DNA levels at day 6 are comparable to that of the basal time point (day 0).

To summarize, the stabilization effect of a stabilization composition comprising a caspase inhibitor and an amide, here a primary carboxylic acid amide, can be significantly increased when used in combination with a polyethylene glycol. Polyethylene glycol was effective in different molecular weights. Moreover, the results indicate that the stabilization properties of PEG increased with increasing molecular weight of PEG, indicating that there is a positive correlation between the molecular weight of used PEG and the resulting sample stabilization effect. Higher molecular weights improved the achieved stabilization effect.

### 2. Example 2

In example 2, the stabilization effect of a combination of EDTA, BA and a caspase inhibitor (Q-VD-OPh) in the absence of water was tested and compared to corresponding compositions additionally including different amounts (0.2g, 0.3g or 0.4g) of PEG with a molecular weight of 600 (PEG600). EDTA blood served as unstabilized reference.

### Blood collection and stabilization

Samples of 10ml whole blood from six donors, collected in 10ml spray dry K2EDTA tubes, were stabilized with mixtures of butanamide (BA) and EDTA with or without different amounts of PEG with a molecular weight of 600 (PEG600) without addition of water. In addition, a caspase inhibitor (Q-VD-OPh) dissolved in DMSO was added by pipetting. Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates per condition and test time point by real time PCR. As a reference control, the EDTA stabilized blood sample (collected in K2 EDTA tubes without further additives) was also stored for 6 days.

Composition of stabilization reagent mixtures (for 10ml K2EDTA whole blood each):
- BA, EDTA, Q-VD-OPh: 100mg BA, 182mg K2EDTA, 10µl Q-VD-OPh (1mg dissolved in 388 µl DMSO), ad 2ml water
- PEG600 (0.2 - 0.4g), BA, EDTA, Q-VD-OPh: 200, 300 and 400mg PEG600,100mg BA, 188mg K2EDTA, 10µl Q-VD-OPh (1mg dissolved in 388 µl DMSO)

Thereby, the following final concentrations of the different components in the mixture are obtained after contact with blood:
- unstabilized: 1.8mg/ml K2EDTA
- BA, EDTA, Q-VD-OPh: 1% (w/v) BA, 20mg/ml K2EDTA, 5µM Q-VD-OPh
- PEG600 (0.2 - 0.4g), BA, EDTA, Q-VD-OPh: 2, 3 and 4% (w/v) PEG600, 1% (w/v) BA, 20mg/ml K2EDTA, 5µM Q-VD-OPh

### Results

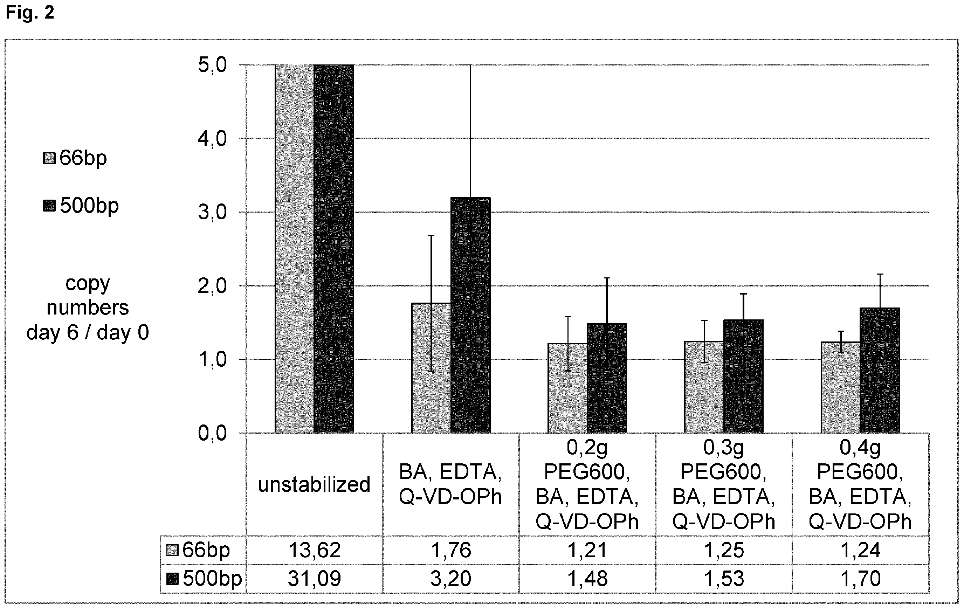

The results of the quantitative real time PCR analyses from six individual donor samples depicted as average fold change is shown in Fig. 2. The increase of DNA (66 bp and 500 bp fragment) relative to time zero with 0.2g, 0.3g or 0.4g PEG600 (average fold change) is shown. All tested stabilization compositions showed significant lower amounts of released DNA after storage for 6 days at room temperature compared to the reference EDTA blood. The stabilization effect was significantly improved if the cell-containing sample was additionally contacted with polyethylene glycol. The average fold change of both 18S rDNA amplicon copy numbers was clearly smaller in all three PEG based stabilization approaches compared the composition not comprising PEG (BA, EDTA, Q-VD-OPh). The x fold change was in all cases below 2-fold. This example demonstrates that additionally using a polyethylene glycol in different quantities for stabilizing the extracellular nucleic acid population significantly improves the stabilization results that are achieved with the caspase inhibitor and the primary carboxylic acid amide butanamide.

### 3. Example 3

In example 3, the stabilization effect of reagent mixtures, including a high molecular weight PEG (PEG3000), EDTA, BA and caspase inhibitor (Q-VD-OPh), directly lyophilized into blood collection tubes in the presence of water was tested and compared to a sample concomitantly treated with a solution comprising BA, EDTA and a caspase inhibitor (Q-VD-OPh).

### Blood collection and stabilization

Samples of 10ml whole blood from eight donors, collected in 10ml spray dry K2EDTA tubes, were stabilized with mixtures of butanamide (BA), EDTA, and caspase inhibitor (Q-VD-OPh) with or without PEG3000. For lyophilisation all components including caspase inhibitor, EDTA, BA and PEG were dissolved in water. Volumes of 1ml (final concentrations see below) were lyophilized on a dry freezer Epsilon 2-25D (Christ GmbH) in 5ml tubes. Blood was transferred from K2EDTA tubes into the 5ml tubes with the lyophilized stabilization reagent and stabilized by 10 times inverting the tubes. As a reference, reagents were freshly prepared and caspase inhibitor (Q-VD-OPh) dissolved in DMSO was added by pipetting.

Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

Composition of stabilization reagent mixtures (for 10ml K2EDTA whole blood each):
- Freshly prepared BA, EDTA, Q-VD-OPh: 100mg BA, 132mg K2EDTA, 10µl Q-VD-OPh (1mg dissolved in 388 µl DMSO), ad 2ml water
- Freshly prepared PEG3000, BA, EDTA, Q-VD-OPh: 250mg PEG3000, 100mg BA, 132mg K2EDTA, 10µl Q-VD-OPh (1mg dissolved in 388 µl DMSO) (no water)

Composition of stabilization reagent mixtures in 0.5ml for lyophilisation into 5ml tubes:
- Lyophilized: 0.5ml of stabilization reagent containing 125mg PEG3000, 50mg BA, 67.5mg K2EDTA, 5µl Q-VD-OPh (1mg dissolved in 388 µl DMSO)

Thereby, the following final concentrations of the different components in the mixture are obtained after contact with blood:
- unstabilized: 1.8mg/ml K2EDTA
- BA, EDTA, Q-VD-OPh: 1% (w/v) BA, 15mg/ml K2EDTA, 5µM Q-VD-OPh
- PEG3000, BA, EDTA, Q-VD-OPh: 1%(w/v) PEG 3000, 1% (w/v) BA, 15mg/ml K2EDTA, 5µM Q-VD-OPh

### Results

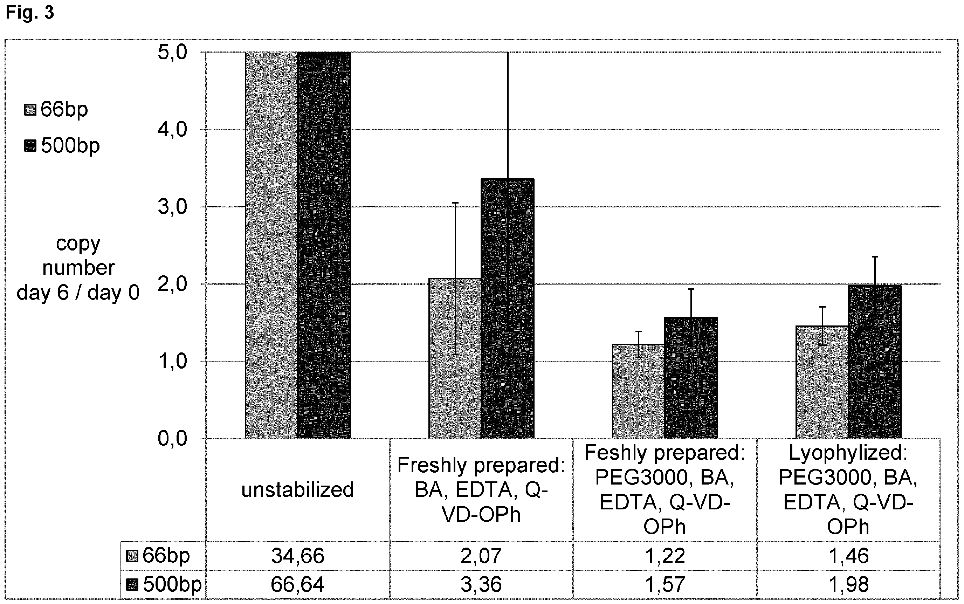

The results are shown in Fig. 3. Shown is the increase (average fold change) of DNA 6 days after blood withdrawal relative to time zero based on different amplicon length of the 18S rDNA gene. The results again demonstrate that the stabilization effect is significantly improved if polyethylene glycol is additionally used for stabilization and that it enhances the stabilization effect that is achieved with BA, EDTA and a caspase inhibitor (Q-VD-OPh). Also during the prolonged stabilization periods tested (6 days), the x fold change was below 2-fold. Furthermore, the example demonstrates that these stabilization compositions may be used either freshly prepared or in lyophilized form.

### 4. Example 4

In example 4, the stabilization effect of PEG6000 (high molecular weight PEG) either alone or in combination with a caspase inhibitor (Q-VD-OPh) on EDTA stabilized blood samples was tested in an aqueous stabilization solution and compared to EDTA stabilized blood alone or BA, EDTA stabilized blood.

### Blood collection and stabilization

Samples of 10ml whole blood from eight donors, collected in 10ml spray dry K2EDTA tubes, were stabilized with aqueous solutions containing either butanamide or PEG in combination with EDTA and optional caspase inhibitor (Q-VD-OPh). Plasma was directly generated from 5ml of stabilized or unstabilized blood samples. Residual blood was stored for additional 3 days at room temperature before plasma generation. ccfDNA was purified from 2ml plasma, copy numbers of 18S rDNA gene were determined in triplicates by real time PCR.

Composition of stabilization reagent mixtures (for 10ml K2EDTA whole blood each):
- BA, EDTA, Q-VD-OPh: 360mg BA, 68.4mg K2EDTA, with or without 2.4µl Q-VD-OPh (1mg dissolved in 388 µl DMSO), water ad 2ml
- PEG6000, EDTA, Q-VD-OPh: 137.5mg PEG6000, 132mg K2EDTA, with or without 2.2µl Q-VD-OPh (1mg dissolved in 388 µl DMSO), water ad 1ml

Thereby, the following final concentrations of the different components in the mixture are obtained after contact with blood:
- unstabilized: 1.8mg/ml K2EDTA
- BA, EDTA: 3% (w/v) BA, 7.2mg/ml K2EDTA
- BA, EDTA, Q-VD-OPh: 3% (w/v) BA, 7.2mg/ml K2EDTA, 1µM Q-VD-OPh
- PEG6000, EDTA: 1.25% (w/v) PEG6000, 7.2mg/ml K2EDTA
- PEG6000, EDTA, Q-VD-OPh: 1.25% (w/v) PEG6000, 7.2mg/ml K2EDTA, 1µM Q-VD-OPh

### Results

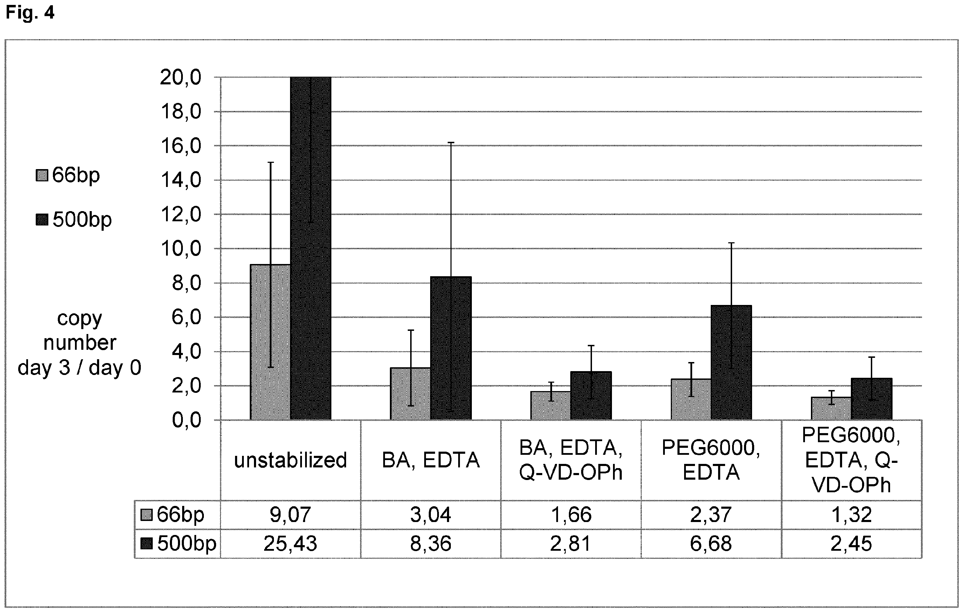

The results of qPCR analyses from eight different donors are shown in Fig. 4. Shown is the average change of copy numbers (fold change) of DNA copies of different 18S rDNA gene amplicons (66bp or 500bp) in stabilized or unstabilized blood from eight donors stored for 3 days at room temperature relative to time point 0 (day 0) after blood withdrawal. The stabilization compositions comprising the high molecular weight polyethylene glycol show significantly lower amounts of released DNA after storage for 3 days at room temperature compared to the unstabilized EDTA blood. The stabilization composition comprising only the high molecular weight PEG as stabilizer achieved over a three day storage period a stabilization effect that was better than the effect achieved with the stabilizing agent butanamide. This demonstrates that polyethylene glycol is also alone effective as stabilizing agent, if the stabilization is to be achieved over shorter stabilization periods. When using polyethylene glycol in combination with a caspase inhibitor (Q-VD-OPh), the stabilization effect was improved. The stabilization effect achieved with a combination of a high molecular weight PEG and the caspase inhibitor is even superior to a stabilization approach using a combination of butanamide and a caspase inhibitor. The results demonstrate that PEG dissolved in an aqueous solution in combination with only an anticoagulant (EDTA) stabilizes both ccfDNA and also white blood cells (thereby preventing the release of cellular DNA into the plasma). Moreover, it was found that this stabilization effect is even more pronounced compared to butanamide.

### 5. Example 5

In example 5, the stabilization effect of PEG with different molecular weights (PEG300, PEG600, PEG1000) in an aqueous stabilization solutions further comprising BA, EDTA and a caspase inhibitor (Q-VD-OPh) was tested and compared to a sample co-treated with BA, dimethylpropionamide (DMPA), EDTA and caspase inhibitor (Q-VD-OPh). Unstabilized EDTA blood served as reference control.

### Blood collection and stabilization

Samples of 10ml whole blood from eight donors, collected in 10ml spray dry K2EDTA tubes, were stabilized with mixtures of butanamide, EDTA and caspase inhibitor (Q-VD-OPh) in an aqueous solution with or without PEG of different molecular weights. Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

Composition of stabilization reagent mixtures (for 10ml K2EDTA whole blood each):
- BA, DMPA, EDTA, Q-VD-OPh: 180mg BA, 180µl DMPA, 68.4mg K2EDTA, 12µl Q-VD-OPh (1mg dissolved in 388 µl DMSO), water ad 2ml
- PEG (300, 600 or 1000), BA, EDTA, Q-VD-OPh: 287.5mg PEG (300, 600 or 1000), 115mg BA, 154.5mg K2EDTA, 11.5µl Q-VD-OPh (1mg dissolved in 388 µl DMSO), water ad 1.5ml

Thereby, the following final concentrations of the different components in the mixture are obtained after contact with blood:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG (300, 600 or 1000), BA, EDTA, Q-VD-OPh: 2.5% (w/v) PEG, 1% (w/v) BA, 15mg/ml K2EDTA, 5µM Q-VD-OPh

### Results

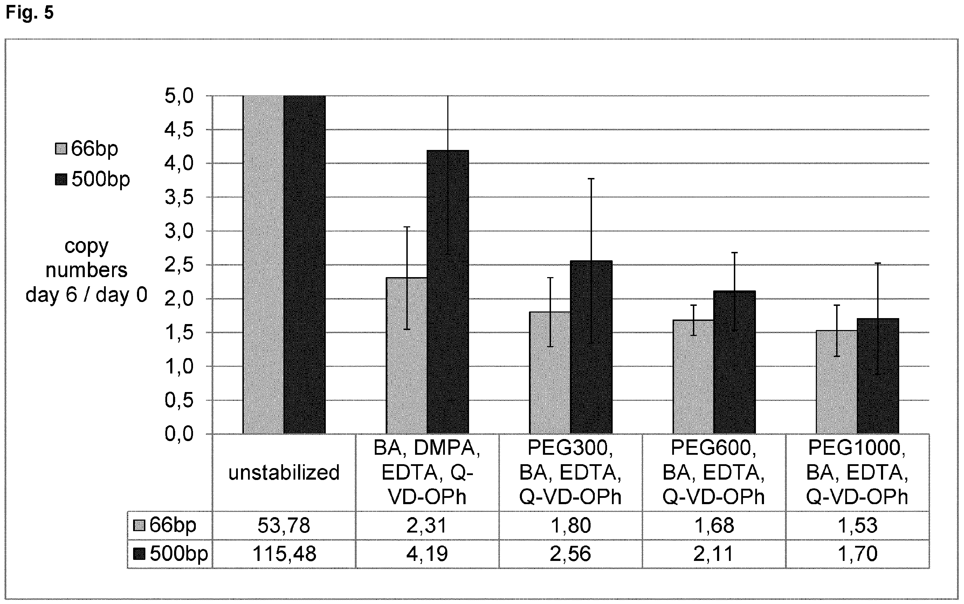

Fig. 5 shows the achieved stabilization results. As can be seen, the aqueous stabilization compositions comprising PEG of different molecular weight stabilized the blood samples and further increased the stabilization effect that was achieved with butanamide and the caspase inhibitor. The stabilization of white blood cells was significantly improved as can be seen from the reduced amount of contaminating genomic DNA. The results also demonstrate that the stabilization effect increases with increasing molecular weight of the used PEG. The increase of the 500bp fragment was reduced below 2-fold with when using polyethylene glycol having a molecular weight of 1000.

### 6. Example 6

Here, the stabilization effect of decreasing PEG concentrations (2%, 1.5%, 1% or 0.7%) in an aqueous stabilization solution were tested in combination with butanamide, EDTA and a caspase inhibitor (Q-VD-OPh). Unstabilized EDTA blood served as reference control. A composition comprising BA, EDTA and Q-VD-OPh was tested in parallel.

### Blood collection and stabilization

Samples of 10ml whole blood from eight donors, collected in 10ml spray dry K2EDTA tubes, were stabilized with mixtures of butanamide, EDTA and caspase inhibitor (Q-VD-OPh) in an aqueous solution with or without different concentrations of PEG6000. Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

Composition of stabilization reagent mixtures (for 10ml K2EDTA whole blood each):
- BA, EDTA, Q-VD-OPh: 110mg BA, 147mg K2EDTA, 11µl Q-VD-OPh (1mg dissolved in 388 µl DMSO), water ad 1ml
- PEG6000 (2 - 0.7%), BA, EDTA, Q-VD-OPh: 220, 165, 110, 77mg PEG6000, 110mg BA, 147mg K2EDTA, 11µl Q-VD-OPh (1mg dissolved in 388 µl DMSO), water ad 1ml

Thereby, the following final concentrations of the different components in the mixture are obtained after contact with blood:
- unstabilized: 1.8mg/ml K2EDTA
- BA, EDTA, Q-VD-OPh: 1% (w/v) BA, 15mg/ml K2EDTA, 5µM Q-VD-OPh
- PEG6000 (2 - 0.7%), BA, EDTA, Q-VD-OPh: 2, 1.5, 1, 0.7% (w/v) PEG6000, 1% (w/v) BA, 15mg/ml K2EDTA, 5µM Q-VD-OPh

### Results

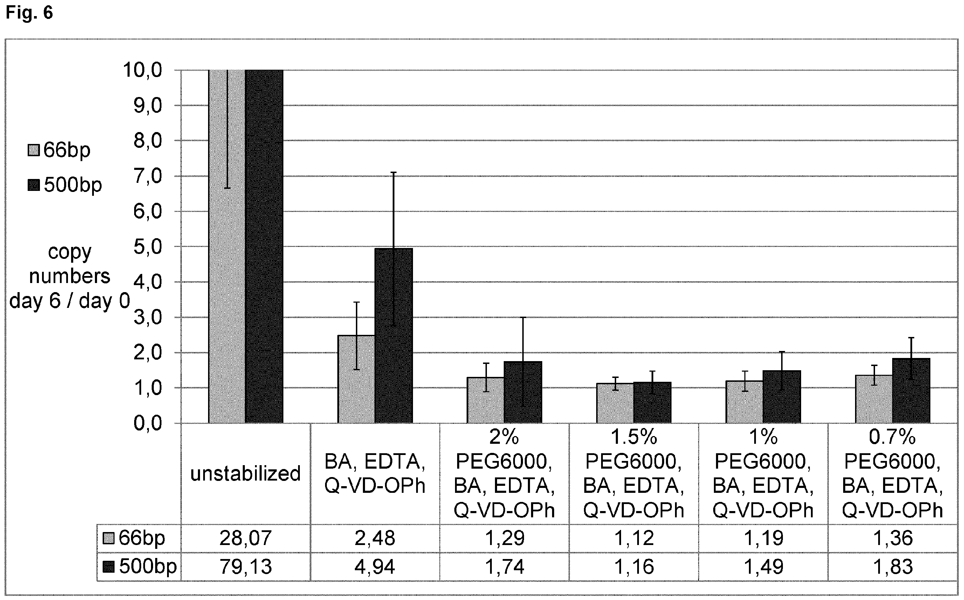

In example 6, decreasing concentrations of higher molecular PEG6000 were tested for their influence on the stabilization of white blood cells when applied in combination with butanamide, EDTA and a caspase inhibitor. Fig. 6 depicts the obtained stabilization result of the extracelluar nucleic acid population as determined by analyzing the increase of 18S rDNA via quantitative real time PCR. All stabilization compositions according to the present invention comprising PEG show significantly lower amounts of released DNA after storage for 6 days at room temperature compared to the stabilization approach involving butanamide, EDTA and a caspase inhibitor. Moreover, as can be seen, the high molecular weight polyethylene glycol can be used in different concentrations to stabilize white blood cells in aqueous solutions thereby reducing contaminations of the extracelluar nucleic acid population with genomic DNA. Furthermore, it is again shown that PEG increases the stabilization effect of butanamide and the caspase inhibitor thereby providing a very effective stabilization approach.

### 7. Example 7

In example 7, the stabilization effect of PEG in an aqueous stabilization solution with different volumes and in combination with EDTA, a caspase inhibitor (Q-VD-OPh) and different amides (BA or DMPA) were tested. Unstabilized EDTA blood served as reference control. A composition comprising BA, DMPA, EDTA and Q-VD-OPh was tested in parallel.

### Blood collection and stabilization

Samples of 10ml whole blood from eight donors, collected in 10ml spray dry K2EDTA tubes, were stabilized with mixtures of PEG6000, butanamide or DMPA, EDTA and caspase inhibitor (Q-VD-OPh) in an aqueous solution with different volumes 0.8 and 1.2 ml. Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

Composition of stabilization reagent mixtures (for 10ml K2EDTA whole blood each):
- BA, DMPA, EDTA, Q-VD-OPh: 180mg BA, 180µl DMPA, 68,4mg K2EDTA, 12µl Q-VD-OPh (1mg dissolved in 388 µl DMSO), water ad 2ml
- PEG6000, BA, EDTA, Q-VD-OPh: 112mg PEG6000, 56mg BA, 150mg K2EDTA, 2.23µl Q-VD-OPh (1mg dissolved in 388 µl DMSO), water ad 1.2 or 0.8ml
- PEG6000, DMPA, EDTA, Q-VD-OPh: 112mg PEG6000, 112µl DMPA, 150mg K2EDTA, 2.23µl Q-VD-OPh (1mg dissolved in 388 µl DMSO), water ad 1.2 or 0.8ml

Thereby, the following final concentrations of the different components in the mixture are obtained after contact with blood:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG6000, BA or DMPA, EDTA, Q-VD-OPh ad 1.2ml: 1% (w/v) PEG6000, 0.5% (w/v) BA or 1% (v/v) DMPA, 15mg/ml K2EDTA, 1µM Q-VD-OPh
- PEG6000, BA or DMPA, EDTA, Q-VD-OPh ad 0.8ml: 1.04% (w/v) PEG6000, 0.52% (w/v) BA or 1.04% (v/v) DMPA, 15.6mg/ml K2EDTA, 1.03µM Q-VD-OPh

### Results

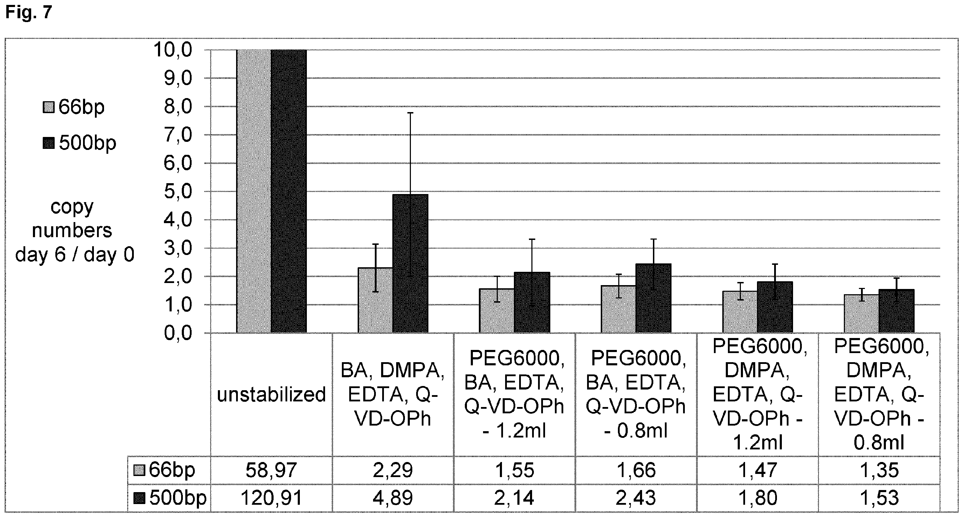

Fig. 7 shows the results of these stabilization assays. Shown is the average change of copy numbers (fold change) of DNA copies of different 18S rDNA gene amplicons (66bp or 500bp) in stabilized or unstabilized blood stored for 6 days at room temperature relative to time point 0 (day 0) after blood withdrawal. The results demonstrate that polyethylene glycol can be combined with different amides to stabilize white blood cells in different volumes of aqueous solutions, thereby providing stabilized blood samples wherein the extracellular nucleic acid population is preserved by preventing a dilution with intracellular nucleic acids.

### 8. Example 8

In example 8, the effect of stabilization reagents in aqueous stabilization solutions is tested by means of hemolysis assays.

### Blood collection and stabilization

Samples of 10ml whole blood from eight donors, collected in 10ml spray dry K2EDTA tubes, were stabilized with mixtures of butanamide or DMPA and EDTA with or without PEG with addition of water. Caspase inhibitor (Q-VD-OPh) dissolved in DMSO was added by pipetting. Plasma was directly generated from 5ml of stabilized or unstabilized blood samples. Residual blood was stored for additional 3, 6 and 10 days at room temperature before plasma generation. Hemoglobin content was determined by measuring absorbance at 414 nm on a spectrophotometer.

Composition of stabilization reagent mixtures (for 10ml K2EDTA whole blood each):
- BA, DMPA, EDTA, Q-VD-OPh: 180mg BA, 180µl DMPA, 68,4mg K2EDTA, 12µl Q-VD-OPh (1mg dissolved in 388 µl DMSO), water ad 2ml
- PEG6000, BA, EDTA, Q-VD-OPh: 137.5mg PEG6000, 55mg BA, 165mg K2EDTA, 11µl Q-VD-OPh (1mg dissolved in 388 µl DMSO), water ad 1.0ml
- PEG6000, DMPA, EDTA, Q-VD-OPh: 110mg PEG6000, 110µl DMPA, 147mg K2EDTA, 11µl Q-VD-OPh (1mg dissolved in 388 µl DMSO), water ad 1.0ml

Thereby, the following final concentrations of the different components in the mixture are obtained after contact with blood:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG6000, BA, EDTA, Q-VD-OPh: 1.25% (w/v) PEG6000, 0.5% (w/v) BA, 15mg/ml K2EDTA, 5µM Q-VD-OPh
- PEG6000, DMPA, EDTA, Q-VD-OPh: 1.0% (w/v) PEG6000, 1.0% (v/v) DMPA, 15mg/ml K2EDTA, 5µM Q-VD-OPh

### Results

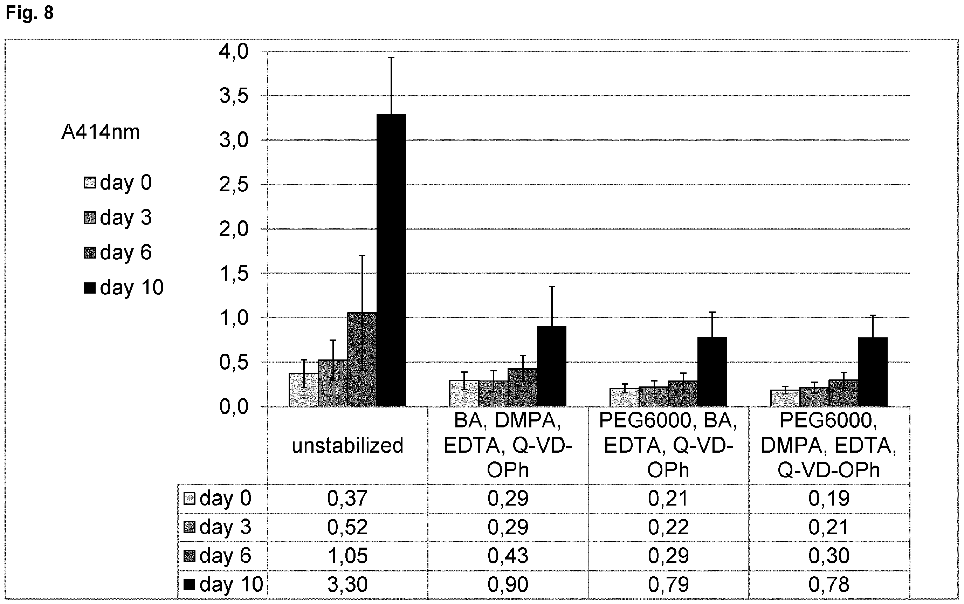

Fig. 8 shows the effect on hemolysis in plasma samples from 8 different donors after 0, 3, 6 and 10 days of storage. Figure 8 depicts the average increase of hemolysis from the eight donors as increase of absorbance at 414nm in the plasma fraction.

Whereas in EDTA control experiments hemolysis is elevated over storage time, hemolysis was reduced in the analyzed time points with the aqueous stabilization solutions according to the invention containing PEG6000 combined with EDTA and either BA or DMPA compared to the EDTA reference as can be seen in Fig. 8. Noteworthy, the increase of hemolysis from storage day 6 to storage day 10 seen in the EDTA control is essentially reduced in all tested solutions comprising the inventive stabilization reagent composition and water. The extent of reduced hemolysis in PEG containing aqueous solutions was comparable to the combined BA, DMPA, EDTA, caspase inhibitor (Q-VD-OPh) stabilized samples. Therefore, dissolving PEG in aqueous stabilization composition is advantageous as it efficiently reduces hemolysis.

### 9. Example 9

In example 9 the effect of using different molecular weight PEGs (PEG300, PEG600, PEG1000, PEG3000) on the ccfDNA copy numbers is tested in combination with BA, EDTA and a caspase inhibitor (Q-VD-OPh) and compared to unstabilized EDTA control blood. A BA, DMPA, EDTA and caspase inhibitor (Q-VD-OPh) containing composition was tested in parallel.

### Blood collection and stabilization

Samples of 10ml whole blood from eight donors, collected in 10ml spray dry K2EDTA tubes, were stabilized with combinations of butanamide, EDTA and caspase inhibitor (Q-VD-OPh) in an aqueous solution comprising PEG of increasing molecular weights. Plasma was directly generated from 5ml of stabilized or unstabilized blood samples, one hour after blood collection. ccfDNA was purified from 2ml plasma, copy numbers of 18S rDNA gene were determined in triplicates by real time PCR.

Composition of stabilization reagent mixtures (for 10ml K2EDTA whole blood each):
- BA, DMPA, EDTA, Q-VD-OPh: 180mg BA, 180µl DMPA, 68.4mg K2EDTA, 12µl Q-VD-OPh (1mg dissolved in 388 µl DMSO), water ad 2ml
- PEG (300, 600, 1000 or 3000), BA, EDTA, Q-VD-OPh: 287.5ml PEG 300 or 287.5mg PEG (600, 1000 or 3000), 115mg BA, 154.5mg K2EDTA, 11.5µl Q-VD-OPh (1mg dissolved in 388 µl DMSO), water ad 1.5ml

Thereby, the following final concentrations of the different components in the mixture are obtained after contact with blood:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG (300, 600, 1000 or 3000), BA, EDTA, Q-VD-OPh: 2.5% (v/v or w/v) PEG, 1% (w/v) BA, 15mg/ml K2EDTA, 5µM Q-VD-OPh

### Results

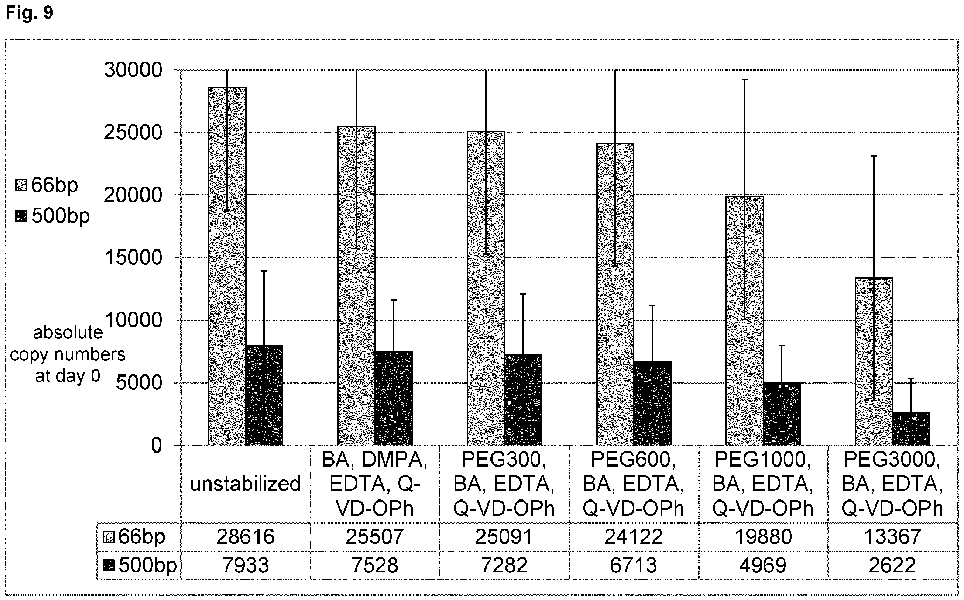

The results of absolute quantitative real time PCR analyses from eight different donors are shown as average in Fig. 9. Particularly shown is the average of absolute copy numbers of DNA of different 18S rDNA gene amplicons (66bp or 500bp) in stabilized or unstabilized blood samples of the donors at day 0 after blood withdrawal. The aim was to test the effect of the used stabilization approach on the subsequent nucleic acid yield when using a silica column based nucleic acid isolation procedure. Fig. 9 shows that the addition of a higher molecular weight PEG led to a reduction of detectable amplicon gene copy numbers in plasma compared to either the EDTA reference samples (unstabilized approach) or the stabilized blood solution containing BA, DMPA, EDTA and a caspase inhibitor (Q-VD-OPh). The results indicates that for the use of PEG with increasing molecular weight or chain lengths for stabilization may lead when used in higher concentrations to a reduction of detectable ccfDNA gene copy numbers in plasma when using a silica column based nucleic acid isolation approach for isolating the extracellular nucleic acids from the stabilized samples.

### 10. Example 10

In example 10 PEG6000 was tested in different concentrations (1.0%, 1.25% or 1.5%) in combination with BA, EDTA and a caspase inhibitor (Q-VD-OPh). An EDTA stabilized blood sample served as reference control. BA, DMPA, EDTA and a caspase inhibitor (Q-VD-OPh) containing blood mixture was analyzed in parallel.

### Blood collection and stabilization

Samples of 10ml whole blood from eight donors, collected in 10ml spray dry K2EDTA tubes, were stabilized with combinations of amides (DMPA and/or BA), EDTA, caspase inhibitor (Q-VD-OPh) with or without PEG6000 in a volume of 1.5ml to 10ml blood with increasing concentration of PEG. Plasma was generated from 5ml of stabilized or unstabilized blood samples, one hour after blood collection. ccfDNA was purified from 2ml plasma, copy numbers of 18S rDNA gene were determined in triplicates by real time PCR.

Composition of stabilization reagent mixtures for 10ml K2EDTA whole blood each:
- BA, DMPA, EDTA, Q-VD-OPh: 180mg BA, 180µl DMPA, 68,4mg K2EDTA, 12µl Q-VD-OPh (1mg dissolved in 388 µl DMSO), water ad 2ml
- PEG6000 (1 - 1.5%), BA, EDTA, Q-VD-OPh - ad 1.5ml: 115, 144 and 172mg PEG6000, 115mg BA, 155mg K2EDTA, 11.5µl Q-VD-OPh (1mg dissolved in 388 µl DMSO), water ad 1.5ml

Thereby, the following final concentrations of the different components in the mixture are obtained after contact with blood:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG6000, BA, EDTA, Q-VD-OPh: 1, 1.25 and 1.5% (w/v) PEG6000, 1% (w/v) BA, 15mg/ml K2EDTA, 5µM Q-VD-OPh

### Results

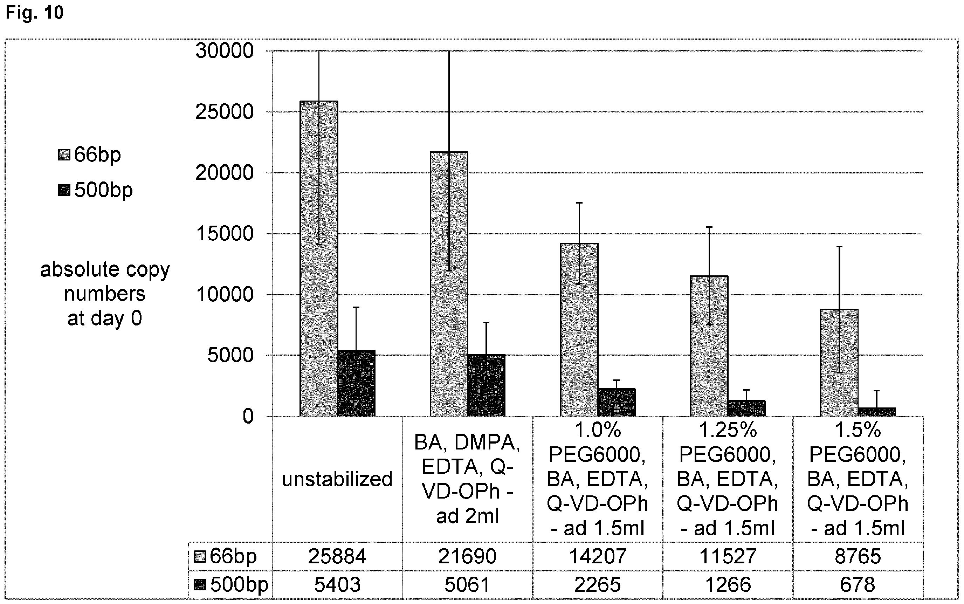

The results of example 10 are shown in Fig. 10. Shown is the average decrease of absolute copy numbers of ccfDNA 0 days after blood draw from 8 donors based on different amplicon length of the 18S rDNA gene with stabilization compositions according to the invention comprising PEG6000 in different concentrations (1.0%, 1.25% or 1.5%) and BA, EDTA and a caspase inhibitor (Q-VD-OPh). Fig. 10 shows that the reduction of absolute copy numbers of the 66bp and 500bp fragment of the 18S rDNA gene in stabilized blood-plasma containing PEG occurs in a PEG concentration dependent fashion. This demonstrates that increasing concentrations of higher molecular PEG (PEG6000) in the stabilization solution, may lead to a reduction of detectable ccfDNA gene copy numbers in plasma when using a silica column based nucleic acid isolation approach.

### 11. Example 11

In example 11, the stabilization effect of different volumes of an aqueous stabilization composition comprising a high molecular weight PEG (PEG6000) in combination with BA, EDTA and a caspase inhibitor (Q-VD-OPh) was analysed. Blood incubated with a stabilization solution comprising a combination of two amides (DMPA, BA), EDTA and a caspase inhibitor (Q-VD-OPh) was analyzed in parallel. EDTA blood served as unstabilized reference.

### Blood collection and stabilization

Samples of 10ml whole blood from eight donors, collected in 10ml spray dry K2EDTA tubes, were stabilized with combinations of amides (DMPA and/or BA), EDTA, caspase inhibitor (Q-VD-OPh) with or without PEG6000 in different volumes of an aqueous solution. Plasma was generated from 5ml of stabilized or unstabilized blood samples, one hour after blood collection. ccfDNA was purified from 2ml plasma, copy numbers of 18S rDNA gene were determined in triplicates by real time PCR.

Composition of stabilization reagent mixtures for 10ml K2EDTA whole blood each:
- BA, DMPA, EDTA, Q-VD-OPh: 180mg BA, 180µl DMPA, 68,4mg K2EDTA, 12µl Q-VD-OPh (1mg dissolved in 388 µl DMSO), water ad 2ml
- PEG6000, BA, EDTA, Q-VD-OPh - ad 1ml: 110mg PEG6000, 110mg BA, 147mg K2EDTA, 11µl Q-VD-OPh (1mg dissolved in 388 µl DMSO), water ad 1ml
- PEG6000, BA, EDTA, Q-VD-OPh - ad 1.5ml: 115mg PEG6000, 115mg BA, 155mg K2EDTA, 11.5µl Q-VD-OPh (1mg dissolved in 388 µl DMSO), water ad 1.5ml
- PEG6000, BA, EDTA, Q-VD-OPh - ad 2ml: 120mg PEG6000, 120mg BA, 162mg K2EDTA, 12µl Q-VD-OPh (1mg dissolved in 388 µl DMSO), water ad 2ml

Thereby, the following final concentrations of the different components in the mixture are obtained after contact with blood:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG6000, BA, EDTA, Q-VD-OPh ad 1, 1.5 or 2ml: 1% (w/v) PEG6000, 1% (w/v) BA, 15mg/ml K2EDTA, 5µM Q-VD-OPh

### Results

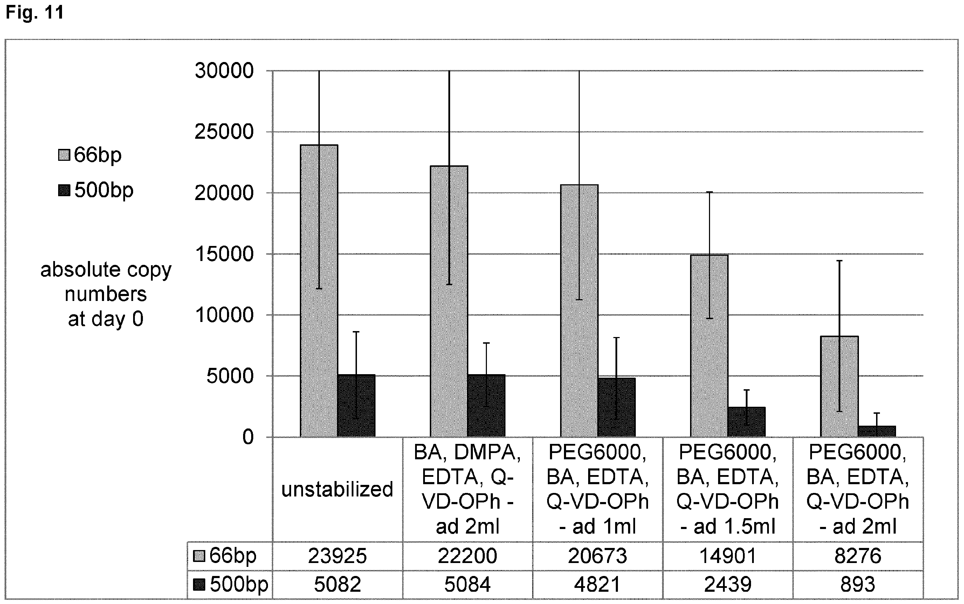

In example 11, different volumes of stabilization solutions (1ml, 1.5ml or 2ml) according to the invention comprising PEG6000 in combination with BA, EDTA and a caspase inhibitor (Q-VD-OPh) were tested. The results shown in Fig. 11 demonstrate that the reduction of absolute copy numbers of the two tested fragments of the 18S rDNA gene in the stabilized samples is dependent on the stabilization reagent volume. Thus, increasing the volume of the stabilization composition (and accordingly increasing the ratio of stabilization composition to blood) containing the high molecular weight PEG lead to a reduction of detectable ccfDNA gene copy numbers in plasma. The copy number was not significantly reduced when using a lower volume as in apparent from the results shown for 1ml stabilization solution. This result is surprising, because the overall concentration was the same in the mixture containing the sample.

### 12. Example 12

Example 12 shows the stabilization effect of stabilization compositions containing a combination of a high molecular weight polyethylene glycol (PEG6000) and a low molecular weight polyethylene glycol (PEG300) in addition to DMPA, EDTA and a caspase inhibitor (Q-VD-OPh) in an aqueous solution with two different volumes (1.5 ml and 2.0 ml). EDTA blood serves as unstabilized reference control.

### Blood collection and stabilization

Samples of 10ml whole blood from eight donors, collected in 10ml spray dry K2EDTA tubes, were stabilized with mixtures of PEG300 and PEG6000, DMPA, EDTA and caspase inhibitor (Q-VD-OPh) in an aqueous solution with different volumes 1.5 and 2.0 ml. Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

Composition of stabilization reagent mixtures (for 10ml K2EDTA whole blood each):
- 0.5% PEG6000, 2.5 or 5% PEG300, DMPA, EDTA, Q-VD-OPh - 2ml: 60mg PEG6000, 300 or 600µl PEG300, 120µl DMPA, 162mg K2EDTA, 12µl Q-VD-OPh (1mg dissolved in 388 µl DMSO), water ad 2ml
- 0.5% PEG6000, 2.5 or 5% PEG300, DMPA, EDTA, Q-VD-OPh - 1.5ml: 57.5mg PEG6000, 287.5µl or 575µl PEG300, 115µl DMPA, 155mg K2EDTA, 11.5µl Q-VD-OPh (1mg dissolved in 388 µl DMSO), water ad 1.5ml

Thereby, the following final concentrations of the different components in the mixture are obtained after contact with blood:
- unstabilized: 1.8mg/ml K2EDTA
- PEG6000, PEG300, DMPA, EDTA, Q-VD-OPh - 2ml: 0.5% (w/v) PEG6000, 2.5 or 5% (v/v) PEG300, 1% (v/v) DMPA, 15mg/ml K2EDTA, 5µM Q-VD-OPh

### Results

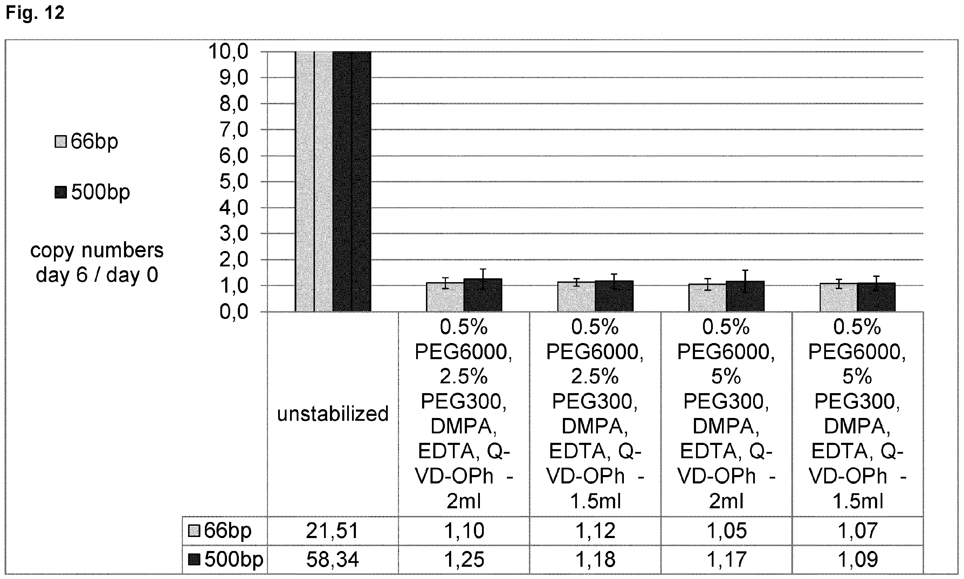

Fig. 12 depicts the results of quantitative real time PCR analyses from eight different donors as change of copy numbers (average fold change) of the two tested 18S rDNA gene amplicons (66bp or 500bp) in stabilized or unstabilized blood from the eight donors stored for 6 days at room temperature to time point 0 (day 0) after blood draw. All stabilized samples showed a comparable low increase of copy number fold change of 18S rDNA gene amplicons. The achieved stabilization effect was extraordinarily high as in all cases, the increase in the 500bp fragment was far below 2-fold and was even below 1.5-fold even though the samples were stored for a prolonged stabilization period of 6 days.

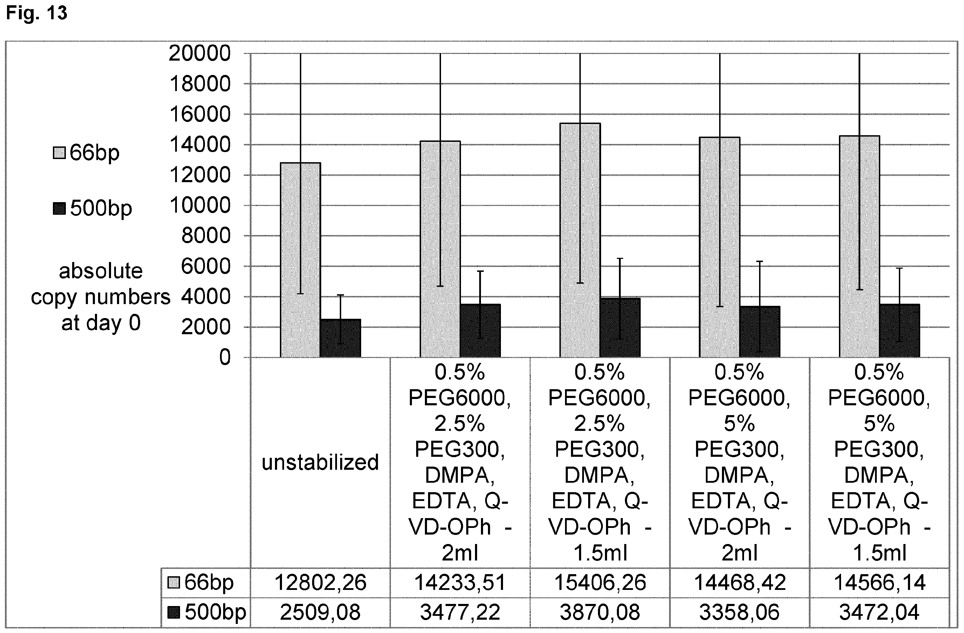

The respective stabilization compositions were also tested for change of absolute copy numbers of 18S rDNA amplicons at day 0 after blood draw in order to analyse whether the detectable ccf DNA gene copy numbers is reduced. Fig. 13 shows the results. As can be seen, all tested stabilization compositions show an absolute copy number that is comparable or even better compared to the unstabilized sample. Therefore, the nucleic acid yield was not reduced when using a silica column based nucleic acid isolation approach for isolating the nucleic acids from the stabilized samples. The reduction in nucleic acid yield observed when a high molecular weight polyethylene glycol was used in higher concentrations was not seen when using a combination of a high molecular weight polyethylene glycol with a low molecular weight polyethylene glycol. Using a respective combination allows to reduce the concentration of high molecular weight polyethylene glycol without compromising the stabilization effect which is supported by the low molecular weight polyethylene glycol. The low molecular weight polyethylene glycol can also be used in higher concentrations without impairing a subsequent nucleic acid isolation procedure that involves a silica column. The elevated standard deviations observed are attributable to the fact that there are variations in the amount of ccfDNA from donor to donor.

This demonstrates that a combination of a high molecular weight polyethylene glycol in combination with a low molecular weight polyethylene glycol is highly advantageous with respect to the achieved stabilization effect and the yield of nucleic acids that can be isolated from the stabilized samples. Therefore, mixtures of different polyethylene glycols can be used in combination to efficiently stabilize the blood samples without a reduction of absolute ccfDNA copy numbers.

### 13. Example 13

In example 13 the stabilization effect of a combination of a high molecular weight PEG (0.5% PEG6000) and a low molecular weight PEG (2.5% or 5% PEG300) combined in aqueous stabilization solutions with BA, EDTA and a caspase inhibitor (Q-VD-OPh) were analyzed. Blood incubated with a stabilization solution comprising a mixture of two amides (DMPA, BA), EDTA and a caspase inhibitor (Q-VD-OPh) was co-analyzed. EDTA blood served as unstabilized reference control.

### Blood collection and stabilization

Samples of 10ml whole blood from eight donors, collected in 10ml spray dry K2EDTA tubes, were stabilized with mixtures of PEG300 and PEG6000, BA, EDTA and caspase inhibitor (Q-VD-OPh) in an aqueous solution with a volume of 1.5 ml. Plasma was directly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

Composition of stabilization reagent mixtures (for 10ml K2EDTA whole blood each):
- BA, DMPA, EDTA, Q-VD-OPh: 180mg BA, 180µl DMPA, 68,4mg K2EDTA, 12µl Q-VD-OPh (1mg dissolved in 388 µl DMSO), water ad 2ml
- 0.5% PEG6000, 2.5 or 5% PEG300, BA, EDTA, Q-VD-OPh - 1.5ml: 57.5mg PEG6000, 287.5µl or 575µl PEG300, 115mg BA, 155mg K2EDTA, 11.5µl Q-VD-OPh (1mg dissolved in 388 µl DMSO), water ad 1.5ml

Thereby, the following final concentrations of the different components in whole blood/stabilization mixtures were obtained:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG6000, PEG300, BA, EDTA, Q-VD-OPh - 1.5ml: 0.5% (w/v) PEG6000, 2.5 or 5% (v/v) PEG300, 1% (w/v) BA, 15mg/ml K2EDTA, 5µM Q-VD-OPh

### Results

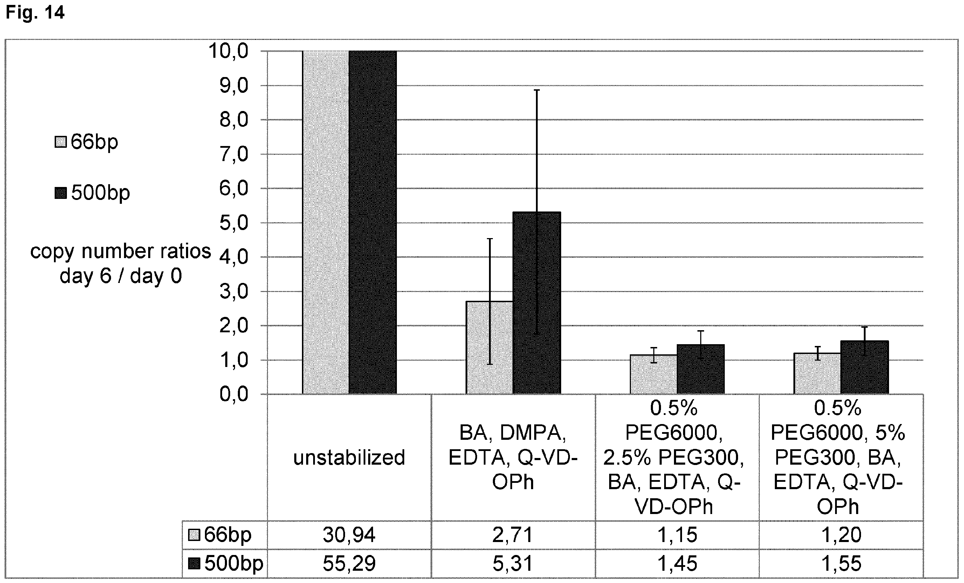

Fig. 14 shows the results of qPCR analyses from eight donors as average change of copy numbers (fold change) of the tested 66bp or 500bp long 18S rDNA gene amplicons in stabilized or unstabilized blood from the donors stored for 6 days at room temperature to time point 0 (day 0) after blood withdrawal. Whereas the unstabilized EDTA blood control manifested an elevation in average fold change in copy numbers, all stabilization compositions containing PEG showed only a low increase with respect to the average fold change of 18S rDNA gene amplicons copy numbers. The results indicate a similar stabilization capability of the tested PEG containing stabilization compositions. The achieved stabilization was superior to the stabilization compositions comprising BA, DMPA, EDTA and the caspase inhibitor thereby again demonstrating the important advantages that are achieved with the invention.

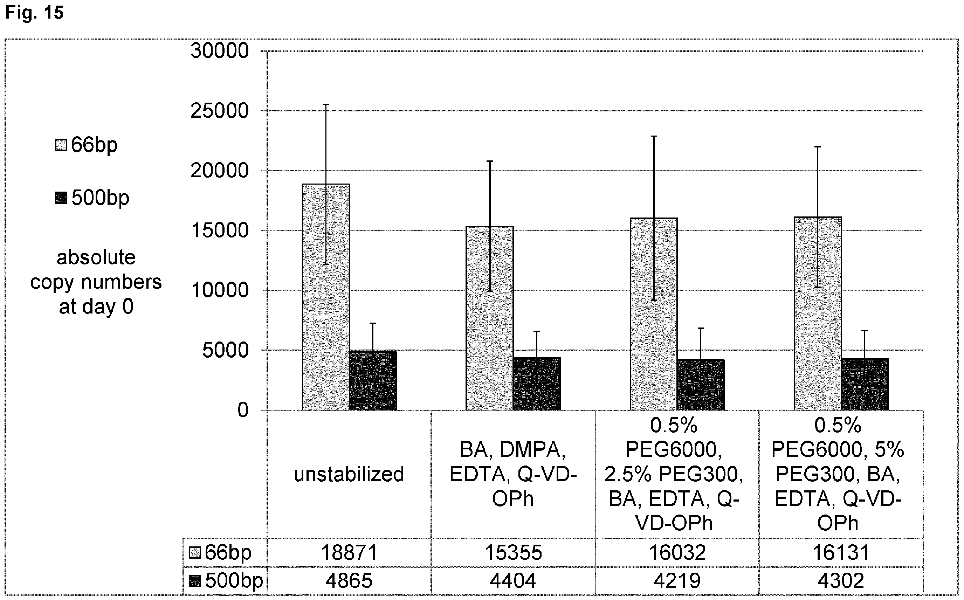

Additionally, it was confirmed that the described advantageous stabilization capability of the used stabilization solutions with combinations of high and low molecular weight PEG is not accompanied by a reduction of ccfDNA copy numbers. This was analyzed by testing the same solutions for change of absolute copy numbers of 18S rDNA amplicons at day 0 in plasma after blood draw. Fig. 15 shows the obtained results. The obtained absolute copy number was similar to the stabilization composition comprising BA, DMPA, EDTA and the caspase inhibitor. Therefore, no significant reduction in the absolute ccf DNA copy number was detected in this assay. Thus, combinations of different molecular weight PEGs, particularly of a high and low molecular weight PEG can be combined in different volumes of aqueous solutions containing BA to effectively stabilize the extracellular nucleic acid population of blood samples, in particular by stabilizing white blood cells without significant reduction of absolute ccfDNA copy numbers when using a standard nucleic acid isolation procedure involving a silica membrane.

### 14. Example 14

In example 14, the effect of aqueous stabilization solutions was tested by hemolysis assays. Here, a combination of a high molecular weight PEG (0.5% PEG6000) and a low molecular weight PEG (2.5% or 5% PEG300) in combination with BA or DMPA and EDTA and caspase inhibitor (Q-VD-OPh) was analyzed. Blood incubated with a stabilization solution comprising a mixture of two amides (DMPA, BA), EDTA and a caspase inhibitor (Q-VD-OPh) was co-analyzed. EDTA blood served as unstabilized reference control.

### Blood collection and stabilization

Samples of 10ml whole blood from eight donors, collected in 10ml spray dry K2EDTA tubes, were stabilized with mixtures of PEG300 and PEG6000, BA or DMPA, EDTA and caspase inhibitor (Q-VD-OPh) in an aqueous solution with a volumes of 1.5 ml. Plasma was directly generated from 5ml of stabilized or unstabilized blood samples. Residual blood was stored for additional 6 days at room temperature before plasma generation. Hemoglobin content was determined by measuring absorbance at 414 nm on a spectrophotometer.

Composition of stabilization reagent mixtures (for 10ml K2EDTA whole blood each):
- BA, DMPA, EDTA, Q-VD-OPh: 180mg BA, 180µl DMPA, 68,4mg K2EDTA, 12µl Q-VD-OPh (1mg dissolved in 388 µl DMSO), water ad 2ml
- 0.5% PEG6000, 2.5 or 5% PEG300, DMPA, EDTA, Q-VD-OPh: 57.5mg PEG6000, 287.5µl or 575µl PEG300, 115µl DMPA, 155mg K2EDTA, 11.5µl Q-VD-OPh (1mg dissolved in 388 µl DMSO), water ad 1.5ml
- 0.5% PEG6000, 2.5 or 5% PEG300, BA, EDTA, Q-VD-OPh: 57.5mg PEG6000, 287.5µl or 575µl PEG300, 115mg BA, 155mg K2EDTA, 11.5µl Q-VD-OPh (1mg dissolved in 388 µl DMSO), water ad 1.5ml

Thereby, the following final concentrations of the different components in whole blood/stabilization mixtures were obtained:
- unstabilized: 1.8mg/ml K2EDTA
- BA, DMPA, EDTA, Q-VD-OPh: 1.5% (w/v) BA, 1.5% (v/v) DMPA, 7.2mg/ml K2EDTA, 5µM Q-VD-OPh
- PEG6000, PEG300, DMPA, EDTA, Q-VD-OPh: 0.5% (w/v) PEG6000, 2.5 or 5% (v/v) PEG300, 1% (v/v) DMPA, 15mg/ml K2EDTA, 5µM Q-VD-OPh
- PEG6000, PEG300, BA, EDTA, Q-VD-OPh: 0.5% (w/v) PEG6000, 2.5 or 5% (v/v) PEG300, 1% (w/v) BA, 15mg/ml K2EDTA, 5µM Q-VD-OPh

### Results

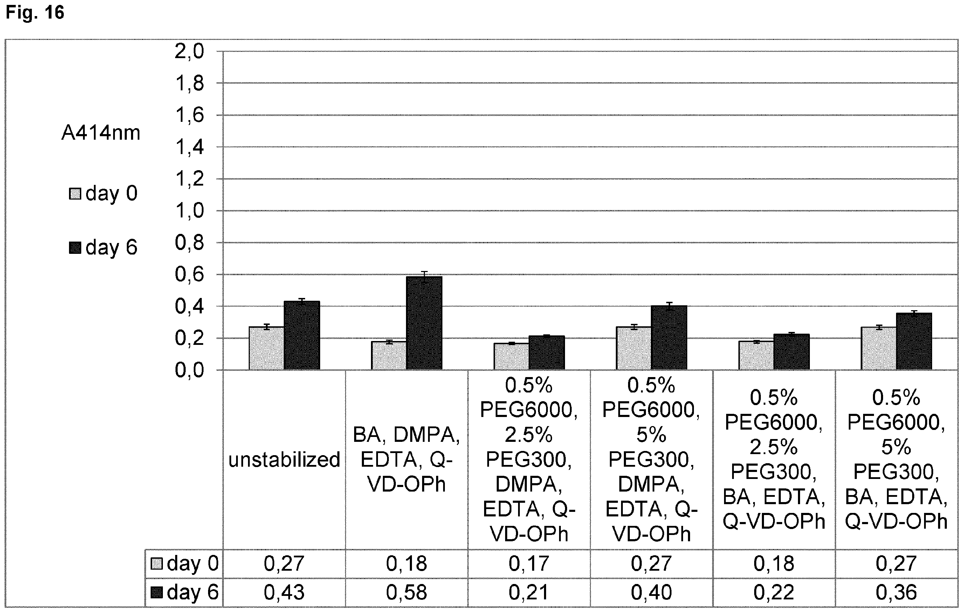

Fig. 16 shows the effect of the analyzed aqueous solutions comprising different molecular PEGs with either DMPA or BA on hemolysis in plasma samples from 8 different donors after 0 and 6 days of blood storage after draw. Fig. 16 shows the increase of hemolysis as increase of absorbance at 414nm in the plasma fraction after 0 and 6 days of blood storage.

As can be seen in Fig. 16, EDTA control experiments reveal an increase in hemolysis (increase of absorption at 414 nm) after 6 days of blood sample storage compared to the initial time point at 0 days after storage. With the stabilization compositions comprising mixtures of 0.5% PEG6000 (higher molecular PEG) and a PEG300 concentration of 5% (lower molecular PEG) in combination with EDTA, caspase inhibitor (Q-VD-OPh) and BA or DMPA, hemolysis was similar to the EDTA reference sample. In contrast, a stabilization composition according to the present invention using lower concentrations of of PEG300 (here: 2.5%) in combination with 0.5% PEG6000, EDTA, caspase inhibitor (Q-VD-OPh) and BA or DMPA reduced hemolysis following blood storage for 6 days. This demonstrates that hemolysis may be prevented in stabilization compositions containing a balanced composition of high and low molecular weight PEG when dissolved in an aqueous solution.

### 15. Example 15

A stabilization composition comprising PEG6000, BA, EDTA and a caspase inhibitor (Q-VD-OPh) pre-filled in vacuumized blood collection tubes (Alpha tubes) was compared to commercially available Streck Cell-Free DNA BCT tubes which comprise a stabilization composition that is based on the use of a formaldehyde releaser as stabilizing agent.

### Blood collection and stabilization

Samples of 10ml whole blood from eight donors were collected in 10ml spray dry K2EDTA, Alpha tubes pre-filled with amounts of stabilization composition of the invention comprising PEG6000, EDTA, a caspase inhibitor (Q-VD-OPh) and BA or DMPA and in Streck Cell-Free DNA BCT tubes. Plasma was directly generated from 5ml of stabilized or unstabilized blood samples. Residual blood was stored for additional 3, 6 and 10 days at room temperature before plasma generation. ccfDNA was purified from 2ml plasma, copy numbers of 18S rDNA gene were determined in triplicates by real time PCR.

Composition of stabilization reagent mixtures in different tubes (all with a draw volume of 10ml blood):
- EDTA - 10ml spray dried EDTA
- Alpha1-Tube (PEG6000, BA, EDTA, Q-VD-OPh): 137.5mg PEG6000, 55mg BA, 165mg K2EDTA, 11µl Q-VD-OPh (1mg dissolved in 388 µl DMSO), water ad 1.0ml
- Alpha2-Tube (PEG6000, DMPA, EDTA, Q-VD-OPh): 110mg PEG6000, 110µl DMPA, 147mg K2EDTA, 11µl Q-VD-OPh (1mg dissolved in 388 µl DMSO), water ad 1.0ml
- Streck Cell-Free DNA BCT tube: comprises a formaldehyde releaser as stabilizer

Thereby, the following final concentrations of the different components in whole blood/stabilization mixtures were obtained:
- EDTA tube: 1.8mg/ml K2EDTA
- Alpha1-tube (PEG6000, BA, EDTA, Q-VD-OPh): 1.25% (w/v) PEG6000, 0.5% (w/v) BA, 15mg/ml K2EDTA, 5µM Q-VD-OPh
- Alpha2-tube (PEG6000, DMPA, EDTA, Q-VD-OPh): 1.0% (w/v) PEG6000, 1.0% (v/v) DMPA, 15mg/ml K2EDTA, 5µM Q-VD-OPh
- Streck Cell-Free DNA BCT tube: concentrations not applicable

### Results

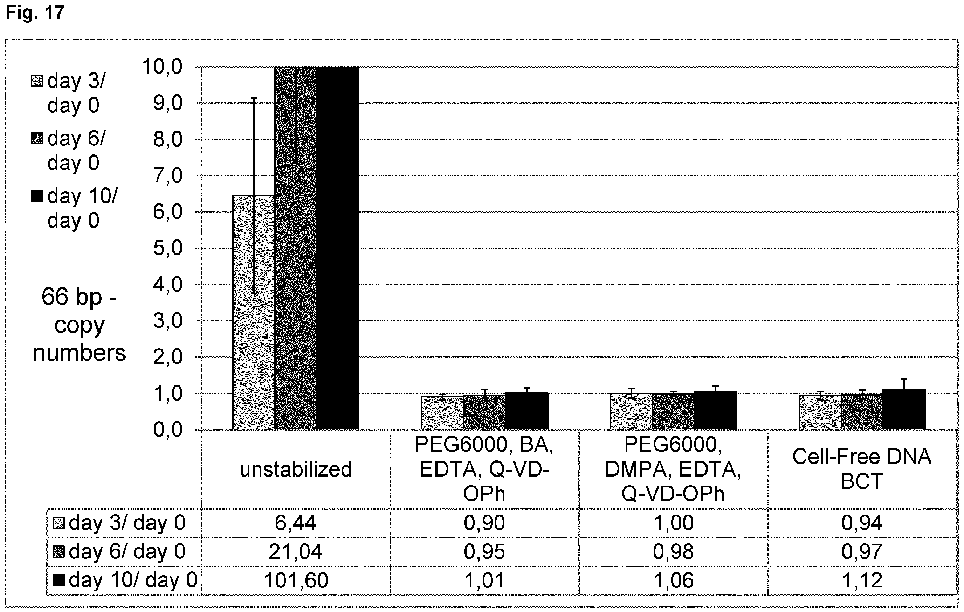

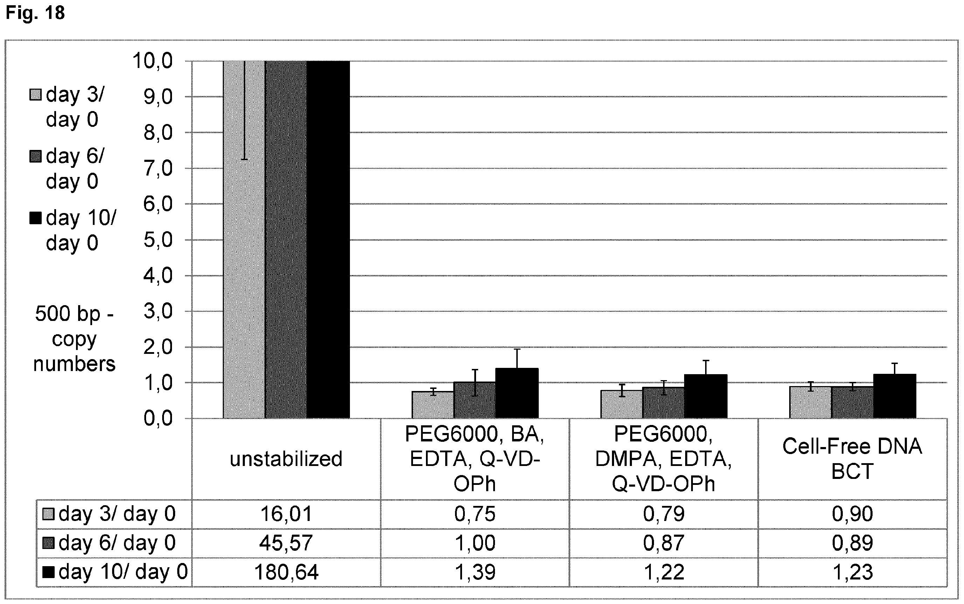

The results are shown in Figs. 17 and 18. The change in copy numbers (average fold change) of 66bp fragment (Fig. 17) and 500bp fragment (Fig. 18) of the 18S rDNA gene in stabilized or unstabilized blood from 8 donors stored for 3, 6 or 10 days at room temperature relative to time point 0 (day 0) after blood draw from the eight blood donors was analyzed. Bars indicate the corresponding standard deviation of the average fold change of the copy numbers from the eight donors per condition. As shown in Fig. 17 and 18, both tested stabilization compositions comprising PEG6000, EDTA, a caspase inhibitor (Q-VD-OPh) and either BA or DMPA are highly efficient in stabilizing the extracellular nucleic acid population in blood. The average fold change of copy numbers of the 66bp fragment of the 18S rDNA stayed on the basal level of time point zero for all tested time points (fold change at around 1.0). Fig. 18 shows comparable results for the 500bp fragment levels of the 18S rDNA gene i.e. the test fragment for cellular nucleic acids released during cell breakage substantially stayed at the status of time point zero over the tested time period. This stabilization effect was comparable to the results where Streck Cell-Free DNA BCT tubes were used. Thus, the stabilization composition according to the present invention efficiently stabilizes the extracellular nucleic acid population in a blood sample by reducing the release from intracellular nucleic acids such as in particular genomic DNA from white blood cells similar to Streck Cell-Free DNA BCT tubes which comprises formaldehyde releasers. However, as explained above, the use of formaldehyde-releasing substances has drawbacks, as they compromise the efficacy of extracellular nucleic acid isolation by induction of cross-links between nucleic acid molecules or between proteins and nucleic acids. Therefore, specific nucleic acid isolation methods must be used. The stabilization composition according to the invention which is based on the use of a poly(oxyethylene) polymer which does not involve the use of such cross-linking substances has important advantages over cross-linking based stabilization techniques.

### 16. Example 16

The stabilization effect of different high molecular weight PEGs (0.5% PEG6000, PEG10000 or PEG20000) in combination with a low molecular weight PEG (3.5% PEG300) combined in aqueous stabilization solutions with BA or DMPA, EDTA and a caspase inhibitor (Q-VD-OPh) were analyzed. The stabilization additives were prefilled in vacuumized blood collection tubes (Alpha tubes). EDTA blood served as unstabilized reference control.

### Blood collection and stabilization

Samples of 10ml whole blood from eight donors were collected in 10ml spray dry K2EDTA tubes, and in Alpha tubes pre-filled with amounts of stabilization composition of the invention comprising either PEG6000, PEG10000 or PEG20000 and additionally PEG300, EDTA, a caspase inhibitior (Q-VD-OPh) and BA or DMPA. Plasma was quickly generated from 5ml of stabilized or unstabilized blood samples. Residual blood was stored for additional 6 days at room temperature before plasma generation. ccfDNA was purified from 2ml plasma, copy numbers of 18S rDNA gene were determined in triplicates by real time PCR.

Composition of stabilization reagent mixtures in different tubes (all with a draw volume of 10ml blood).
- EDTA (unstabilized) - 18mg spray dried EDTA
- Alpha3-Tube (0.5% PEG6000, 3.5% PEG300, DMPA, EDTA, Q-VD-OPh): 57.5mg PEG6000, 402.5µl PEG300, 115µl DMPA, 152mg K2EDTA, 11.5µl Q-VD-OPh (1mg dissolved in 388 µl DMSO), water ad 1.5ml
- Alpha4-Tube (0.5% PEG6000, 3.5% PEG300, BA, EDTA, Q-VD-OPh): 57.5mg PEG6000, 402.5µl PEG300, 115µg BA, 152mg K2EDTA, 11.5µl Q-VD-OPh (1mg dissolved in 388 µl DMSO), water ad 1.5ml
- Alpha5-Tube (0.5% PEG10000, 3.5% PEG300, DMPA, EDTA, Q-VD-OPh): 57.5mg PEG10000, 402.5µl PEG300, 115µl DMPA, 152mg K2EDTA, 11.5µl Q-VD-OPh (1mg dissolved in 388 µl DMSO), water ad 1.5ml
- Alpha6-Tube (0.5% PEG10000, 3.5% PEG300, BA, EDTA, Q-VD-OPh): 57.5mg PEG10000, 402.5µl PEG300, 115µg BA, 152mg K2EDTA, 11.5µl Q-VD-OPh (1mg dissolved in 388 µl DMSO), water ad 1.5ml
- Alpha7-Tube (0.5% PEG20000, 3.5% PEG300, DMPA, EDTA, Q-VD-OPh): 57.5mg PEG20000, 402.5µl PEG300, 115µl DMPA, 152mg K2EDTA, 11.5µl Q-VD-OPh (1mg dissolved in 388 µl DMSO), water ad 1.5ml
- Alpha8-Tube (0.5% PEG20000, 3.5% PEG300, BA, EDTA, Q-VD-OPh): 57.5mg PEG20000, 402.5µl PEG300, 115µg BA, 152mg K2EDTA, 11.5µl Q-VD-OPh (1mg dissolved in 388 µl DMSO), water ad 1.5ml

Thereby, the following final concentrations of the different components in whole blood/stabilization mixtures were obtained:
- unstabilized: 1.8mg/ml K2EDTA
- PEG6000, PEG10000 or PEG20000, PEG300, DMPA or BA, EDTA, Q-VD-OPh - 1.5ml: 0.5% (w/v) PEG6000 (or PEG10000, or PEG20000), 5.5% (v/v) PEG300, 1% (v/v) DMPA or (w/v) BA, 13,2mg/ml K2EDTA, 5µM Q-VD-OPh

### Results

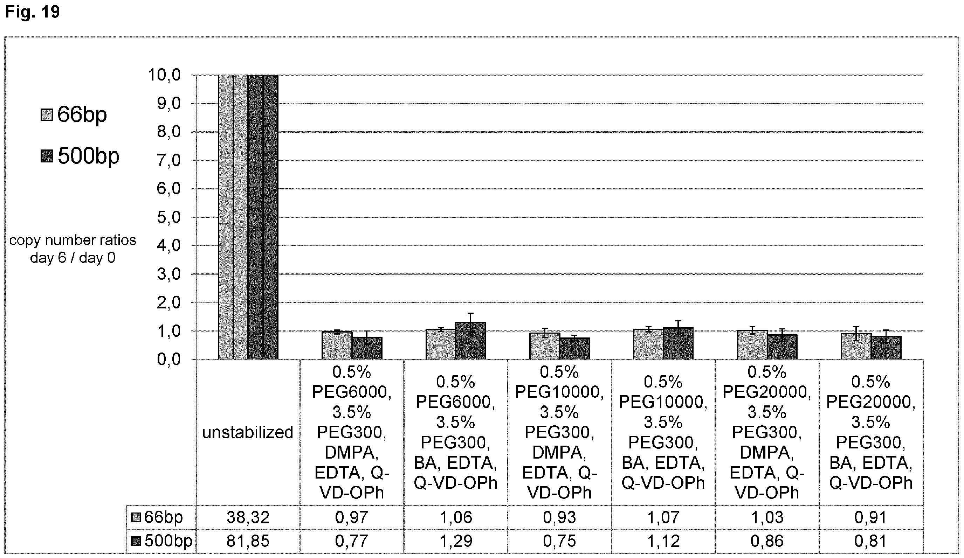

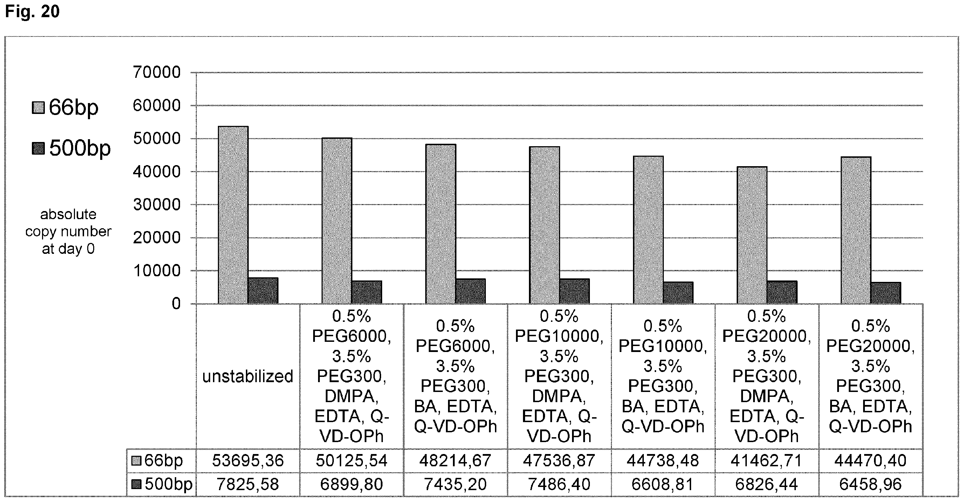

The results are shown in Figs. 19 and 20. Fig 19. demonstrates the change in copy numbers (average fold change) of 66bp or 500pb fragment of the 18S rDNA gene in stabilized or unstabilized blood from 8 donors stored for 6 days at room temperature relative to time point 0 (day 0). Fig. 20 shows the absolute copy numbers at day 0 in plasma, directly after the blood draw. As shown in Fig. 19, PEGs with a molecular weight of up to 20.000 in compositions comprising additionally PEG300, EDTA, a caspase inhibitor (Q-VD-OPh) and either BA or DMPA are highly efficient in stabilizing the extracellular nucleic acid population in blood. In contrast to unstabilized EDTA blood, for the stabilized blood from Alpha Tubes 3 - 8 the average fold change of copy numbers for both the 66 and 500bp fragment of the 18S rDNA stayed on the basal level of time point zero after 6 days storage (fold change at around 1.0). In addition, the balanced compositions of high and low moleculare weight PEGs did not reduce the absolute copy numbers of ccfDNA in comparison to plasma from unstabilized EDTA directly after blood draw (see Fig. 20), thereby demonstrating that extracellular nucleic acids could be effectively isolated from the stabilized samples. That the absolute copy numbers are lower in the stabilized samples compared to the unstabilized reference sample is attributable to the fact that the obtained plasma is diluted with the liquid stabilization composition. Due to this dilution, the initial amount of nucleic acids is lower in the stabilized samples.

### 17. Example 17

The stabilization technology of the present invention is also compatible with an anion exchange based nucleic acid isolation protocol. Extracellular nucleic acids were isolated from plasma that was obtained from a stabilized blood sample. Stabilization was performed using a stabilization composition comprising K₂EDTA, Q-VD-OPH, DMPA, PEG 10000, PEG300 in water (see above). Extracellular nucleic acids were isolated from the stabilized plasma samples using the QIAamp circulating nucleic acid kit (QIAGEN) according to the manufacturers instructions (2ml plasma volume, 60µl elution volume). Alternatively, extracelluar nucleic acids were isolated from stabilized plasma samples (2ml) using magnetic particles comprising anion exchange groups (tertiary amine groups) as solid phase for nucleic acid binding. The sample was disrupted and binding occurred at an acidic pH (4.5). The bound nucleic acids were washed three times and eluted using 75µl of an alkaline elution buffer (pH 12.5). The protocol was performed using an automated system (QIAsymphony).

The nucleic acid yield (18s rDNA (66bp) and 18s rDNA (500bp) obtained with the anion exchange based nucleic acid protocol was compared to the results obtained with the QIAamp circulating nucleic acid kit (set as 100%) by PCR analysis (compared to a genomic DNA dilution series to determine the copy number). The subsequent table shows the results:

| | Anion exchange based nucleic acid isolation protocol | QIAamp circulating nucleic acid kit |
|---|---|---|
| 18srDNA (66bp) | 98.64 | 100.00 |
| 18s rDNA (500bp) | 88.04 | 100.00 |

As can be seen, extracellular nucleic acids could be obtained with high yield from the stabilized samples using an anion exchange based nucleic acid isolation protocol.

## Claims

1. A collection device for collecting a cell-containing biological sample, wherein the collection device comprises:
i) a poly(oxyethylene) polymer as stabilizing agent or
ii) mono-ethylene glycol as stabilizing agent
and one or more further additives selected from the group consisting of
- one or more primary, secondary or tertiary amides;
- a caspase inhibitor;
- an anticoagulant and/or a chelating agent.

2. The collection device according to claim 1, comprising a composition suitable for stabilizing a cell-containing biological sample, the composition comprising:
i) a poly(oxyethylene) polymer as stabilizing agent or
ii) mono-ethylene glycol as stabilizing agent
and one or more further additives selected from the group consisting of
- one or more primary, secondary or tertiary amides;
- a caspase inhibitor;
- an anticoagulant and/or a chelating agent.

3. The collection device according to claim 1 or 2, comprising a poly(oxyethylene) polymer which is a high molecular weight poly(oxyethylene) polymer having a molecular weight of at least 1500 as stabilizing agent and additionally comprising one or more, preferably two or more further additives selected from the group consisting of
- at least one further poly(oxyethylene) polymer having a molecular weight that is at least 100, preferably at least 200, at least 300 or at least 400 below the molecular weight of the high molecular weight poly(oxyethylene) polymer, wherein said further poly(oxyethylene) polymer preferably is a low molecular weight poly(oxyethylene) polymer having a molecular weight of 1000 or less;
- one or more primary, secondary or tertiary amides;
- a caspase inhibitor;
- an anticoagulant and/or a chelating agent.

4. The collection device according to one or more of claims 1 to 3, wherein the poly(oxyethylene) polymer is a polyethylene glycol, preferably unsubstituted polyethylene glycol.

5. The collection device according to one or more of claims 1 to 4, comprising a caspase inhibitor and one or more primary, secondary or tertiary amides according to formula 1 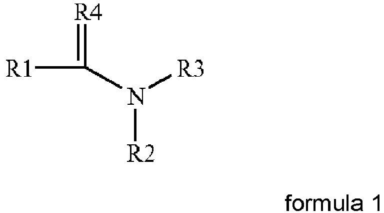 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue, preferably R4 is oxygen and wherein preferably, the collection device comprises butanamide and/or an N,N-dialkylpropanamide, wherein said N,N-dialkylpropanamide preferably is N,N-dimethylpropanamide.

6. The collection device according to one or more of claims 3 to 5, wherein the high molecular weight poly(oxyethylene) polymer has a molecular weight that lies in a range selected from 1500 to 50000, 1500 to 40000, 2000 to 30000, 2500 to 25000, 3000 to 20000, 3500 to 15000 and 4000 to 10000.

7. The collection device according to one or more of claims 3 to 6, wherein said further poly(oxyethylene) polymer is a low molecular weight poly(oxyethylene) polymer having a molecular weight of 1000 or less and wherein preferably, the molecular weight lies in a range selected from 100 to 1000, 150 to 800, 150 to 700, 200 to 600, 200 to 500 and 200 to 400.

8. The collection device according to one or more of claims 1 to 7, wherein the collection device comprises a poly(oxyethylene) polymer, which preferably is a high molecular weight poly(oxyethylene) polymer, at least one caspase inhibitor and an anticoagulant and optionally comprises at least one primary, secondary or tertiary amide.

9. The collection device according to claim 8, comprising at least one primary, secondary or tertiary amide, wherein preferably, said amide is a compound according to formula 1, more preferred butanamide and/or a N,N-dialkylpropanamide, preferably N,N-dimethlypropanamide.

10. The collection device according to one or more of claims 3 to 9, comprising:
a) at least one high molecular weight poly(oxyethylene) polymer having a molecular weight of at least 1500, preferably in a range of 2000 to 40000, more preferred 2500 to 30000, 2500 to 25000 or 3000 to 20000;
b) one or more primary, secondary or tertiary amides; preferably one or more compounds according to formula 1;
c) at least one caspase inhibitor, preferably a pancaspase inhibitor, more preferably the caspase inhibitor comprises a modified caspase-specific peptide, optionally wherein said caspase-specific peptide is modified by an aldehyde, nitrile or ketone compound, more preferred Q-VD-OPh;
d) optionally at least one further poly(oxyethylene) polymer having a molecular weight that is at least 100, preferably at least 200, at least 300 or at least 400 below the molecular weight of the high molecular weight poly(oxyethylene) polymer used and wherein said further poly(oxyethylene) polymer preferably is a low molecular weight poly(oxyethylene) having a molecular weight of 1000 or less, preferably having a molecular weight in a range of 200 to 800 or 200 to 600;
e) optionally an anticoagulant and/ or a chelating agent, more preferably EDTA.

11. The collection device according to claim 10, wherein
a) the high molecular weight poly(oxethylene) polymer is comprised in the collection device in a concentration so that when the cell-containing biological sample is collected into said device, the concentration of the high molecular weight poly(oxyethylene) polymer in the resulting mixture lies in a range selected from 0.05% to 4% (w/v), 0.1% to 3% (w/v), 0.2% to 2.5% (w/v), 0.25% to 2% (w/v), 0.3% to 1.75% (w/v) and 0.35% to 1.5% (w/v);
b) the one or more primary, secondary or tertiary amide is comprised in the collection device in a concentration so that when the cell-containing biological sample is collected into said device, the concentration of the amide in the resulting mixture lies in a range of 0.25% to 5%, 0.3% to 4%, 0.4% to 3%, 0.5% to 2% or 0.75% to 1.5%;
c) optionally the at least one caspase inhibitor is comprised in the collection device in a concentration so that when the cell-containing biological sample is collected into said device, the concentration of the caspase inhibitor in the resulting mixture lies in a range of 0.1µM to 20µM, more preferred 0.5µM to 10µM, more preferred 1µM to 10µM, more preferred 3µM to 7.5µM or 3µM to 5µM.

12. The collection device according to claim 1 or 2, comprising mono-ethylene glycol and optionally
- additionally comprising at least one caspase inhibitor and at least one primary, secondary or tertiary amide, preferably, as defined in claim 5; and/or
- comprising an anticoagulant, preferably a chelating agent, and/or a poly(oxyethylene) polymer as defined in one or more of the above claims.

13. The collection device according to one or more of claims 2 to 12, wherein the composition has one or more of the following characteristics:
a) it is capable of stabilizing cells and reducing the release of genomic DNA from cells contained in the cell-containing biological sample into the cell-free portion of the sample;
b) it is capable of reducing the degradation of nucleic acids, in particular genomic DNA, present in the stabilized sample;
c) it is capable of reducing or preventing the contamination of the extracellular DNA population comprised in the biological sample with genomic DNA originating from cells contained in the stabilized sample;
d) it is capable of reducing or preventing the contamination of the extracellular nucleic acid population comprised in the biological sample with intracellular nucleic acids originating from cells contained in the stabilized sample;
e) the stabilization composition does not comprise additives in a concentration wherein said additives would induce or promote cell lysis;
f) the stabilization composition does not comprise a cross-linking agent that induces protein-DNA and/or protein-protein crosslinks such as formaldehyde, formaline, paraformaldehyde or a formaldehyde releaser;
g) the stabilization composition does not comprise a toxic agent;
h) it comprises a compound according to formula 1 and has transcriptome stabilizing properties, and/or it is capable of stabilizing the extracellular nucleic acid population comprised in the cell-containing biological sample;
i) it is capable of stabilizing extracellular nucleic acid population comprised in the cell-containing biological sample without refrigeration, preferably at room temperature, for a time period selected from at least three days, at least four, at least five or at least six days; and/or
j) the composition additionally comprises the cell-containing sample to be stabilized which preferably is blood.

14. The collection device according to one or more of claims 2 to 13, wherein the stabilizing composition is liquid and is comprised in the collection device in an amount effective to provide the stabilization of an amount of sample to be collected in said device, wherein preferably, the stabilizing composition is contacted with the biological sample in a volumetric ratio selected from 10:1 to 1:20, 5:1 to 1:15, 1:1 to 1:10, 1:10 to 1:5 and 1:7 to 1:5, in particular about 1:6.

15. The collection device according to one or more of claims 1 to 14, wherein:
a) the collection device is a collection device for collecting a blood, plasma or serum sample, wherein preferably, the collection device comprises an anticoagulant, which preferably is a chelating agent, more preferably EDTA;
b) the collection device is for drawing blood from a patient; and/or
c) the cell-containing biological sample is a blood sample.

16. The collection device according to one or more of claims 1 to 15, wherein the collection device is evacuated.

17. The collection device according to one or more of claims 2 to 16, wherein the collection device is pre-filled with a defined amount of the stabilizing composition either in solid or liquid form and is provided with a defined vacuum and sealed with a septum.

18. The collection device according to one or more of claims 1 to 16, wherein the collection device has an open top, a bottom, and a sidewall extending therebetween defining a chamber, wherein the poly(oxyethylene) polymer, which preferably is a high molecular weight poly(oxyethylene) polymer, and the one or more further additives are comprised in the chamber.

19. The collection device according to claim 18, wherein the collection device is a tube, the bottom is a closed bottom, the collection device further comprises a closure in the open top, and the chamber is at a reduced pressure, preferably wherein the closure is capable of being pierced with a needle or cannula, and the reduced pressure is selected to draw a specified volume of a liquid sample into the chamber.

20. The collection device according to claim 19, wherein the chamber is at a reduced pressure selected to draw a specified volume of a liquid sample into the chamber, and the collection device comprises a stabilizing composition according to one or more of claims 2 to 13 which is a liquid and is disposed in the chamber such that the volumetric ratio of the stabilizing composition to the specified volume of the cell-containing sample is selected from 10:1 to 1:20, 5:1 to 1:15 and 1:1 to 1:10 and preferably is 1:10 to 1:5, more preferably 1:7 to 1:5.

21. The collection device according to one or more of claims 2 to 20, comprising the stabilizing composition mixed with a cell-containing biological sample, optionally wherein the cell-containing biological sample is whole blood and/or comprises extracellular nucleic acids, wherein preferably the volumetric ratio of the stabilizing composition to the cell-containing biological sample is selected from 10:1 to 1:20, 5:1 to 1:15, 1:1 to 1:10, 1:10 to 1:5 and 1:7 to 1:5, in particular about 1:6.

22. A method comprising the step of collecting a cell-containing biological sample from a patient directly into a chamber of the collection device according to one or more of claims 1 to 20.
